(19) Europäisches Patentamt / European Patent Office / Office européen des brevets

(11) **EP 4 478 367 A1**

(12) **EUROPEAN PATENT APPLICATION**

(43) Date of publication:
**18.12.2024 Bulletin 2024/51**

(21) Application number: **23386046.9**

(22) Date of filing: **13.06.2023**

(51) International Patent Classification (IPC):
***G16B 20/30*** *(2019.01)* ***G16B 40/20*** *(2019.01)*

(52) Cooperative Patent Classification (CPC):
**G16B 20/30; G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **NEC OncoImmunity AS**
**0379 Oslo (NO)**

(72) Inventors:
- **Simovski, Boris**
  **0379 Oslo (NO)**
- **Vardaxis, Alexandros Ioannis**
  **0379 Oslo (NO)**
- **Clancy, Trevor**
  **0379 Oslo (NO)**
- **Stratford, Richard**
  **0379 Oslo (NO)**

(74) Representative: **Gill Jennings & Every LLP**
**The Broadgate Tower**
**20 Primrose Street**
**London EC2A 2ES (GB)**

Remarks:
Claims 16-24 are deemed to be abandoned due to non-payment of the claims fees (Rule 45(3) EPC).

# (54) B-CELL EPITOPE PREDICTION

(57) A computer-implemented method of predicting whether a protein comprises a B-cell epitope that is likely to instigate a binding event with an antibody is disclosed. The method comprises: (a) accessing the amino acid sequence of the protein; (b) accessing one or more structure and/or surface characteristics of the protein; (c) generating one or more candidate encodings of respective one or more candidate B-cell epitopes on the protein, wherein each candidate encoding represents the respective candidate B-cell epitope as a plurality of data elements corresponding to the amino acids of the amino acid sequence; and (d) using a trained first machine learning model to predict whether any of the candidate B-cell epitope(s) is likely to be a true B-cell epitope on the protein; wherein the input to the trained first machine learning model comprises the amino acid sequence of the protein, the one or more structure and/or surface characteristics of the protein, and the one or more candidate encodings. A method of training a machine learning model is also disclosed.

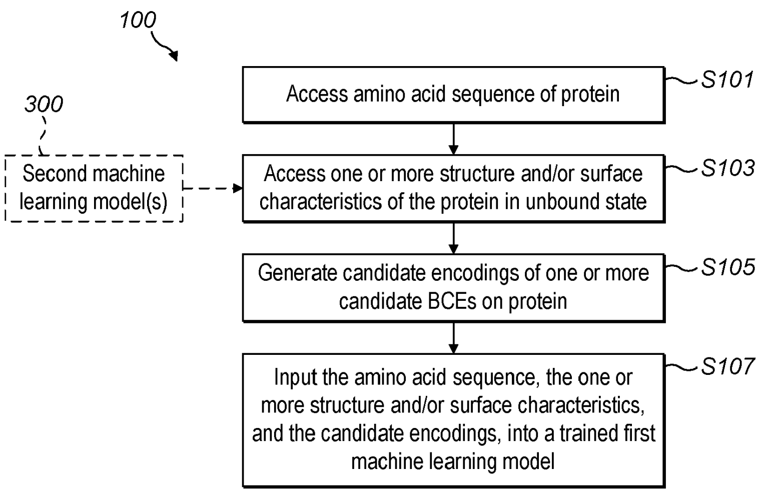


FIG. 1

EP 4 478 367 A1

## Description

## BACKGROUND

Lymphocytes are a type of white blood and are central components of the immune system of most vertebrates. They include B cells, T cells and natural killer (NK) cells. Their function is to identify and protect against pathogens, such as bacteria, viruses, or malignant cancer cells that harbor mutated protein antigens. Both Band T-Cells express surface receptors which identify specific molecular components on pathogens called antigens (Ags). The successful recognition of an Ag triggers a wider adaptive immune response which ultimately may eliminate the potentially pathogenic threat.

Antibodies (Abs), also known as immunoglobulins (Ig), are glycosylated protein molecules present on the surface of B cells (surface immunoglobulins) or B cell eluted Ig molecules, that act as antigen specific B cell receptors (BCR). Upon successful Ag recognition, the Ig or membrane-bound BCRs will bind on specific Ag amino acid contact points, collectively named here as a B-cell epitope (BCE). This binding event may trigger the activation of an immune response that includes a clonal B cell expansion which will produce a vast amount of the Ag specific Ig serum eluted molecules or effector B cells harbouring Ag specific BCRs. The secreted soluble Igs and effector BCRs, also called antibodies (Abs), will have the same binding specificity as the original Ig-Ag binding interaction event. Abs will then circulate into the serum and bind to the same BCEs to signal the elimination of the pathogenic or cell-stressed cells that correspond to the identified Ags.

Ab based vaccines have the potential to prepare or educate the immune system of each individual against potentially harmful Ags sourced from infectious pathogenic threats or stressed malignant cancer cells. Vaccine design aims for the modification of the Ag in such a way that it bypasses harmful malignant, pathogenic or infectious properties while maintaining potential protective immunes responses driven by immunogenic epitopes. The accurate identification or prediction of B-Cell epitopes on antigens is therefore of great importance not only for vaccine design, but also in the domains of molecular diagnostics, immune-monitoring and immunotherapy.

BCEs can be divided into two main categories: linear B cell epitopes (LBCEs) and conformational B cell epitopes (CBCEs). Both LBCEs and CBCEs can be bound by Abs and, thus, trigger an immune response. It is estimated that > 90% of the BCEs are conformational, while the rest are linear.

LBCEs are made up of continuous (sequential) sequences of amino-acids. These BCEs can be discovered by identifying positions on the unfolded Ag sequence which can be bound by Abs. LBCEs are often catalogued at the immune epitope database (IEDB) sourced from a large variety of experimental assays. Each assay provides information about the protein sequence tested (that is, the Ag), the LBCE sequence as well as its coordinates on the Ag, and the Ab which the current LBCE was tested on. Moreover, they provide an indicator as to whether the LBCE was bound by the given Ab. The binding information is sometimes described in the IEDB in the form of human readable labels, that is; positive, positive-high, positive-intermediate, positive-low and negative. The simplicity of LCBE as linear molecular entities, and the well-established experimental methods that are used characterized them has led to a relatively large amount of available LBCE data compared to CBCE data. Nevertheless, annotation errors are frequent in this type of data, since many amino acids of the identified BCEs are not actually interacting with the Abs under the tertiary conformation of the Ag.

CBCEs are made up of short segments of amino-acids with potential diverse cis, trans, short or distal coordinate distances between each of the amino acid contact points that constitute the BCE. These segments are come into proximity during the folding of the Ag in the act of binding to the Ab. CBCEs can be experimentally characterized using X-ray crystallography on Ag-Ab structure complexes. This data is usually catalogued in the Protein Data Bank (PDB) in the form of 3D structures, and typically consist of one or more Ag structures bound by at least one Ab structure. PDB structure data provides information about the 3D coordinates of each molecule in the structure. However, the complexity of this method makes the CBCE data somewhat more limited compared to that of LBCEs. Moreover, the 3D structures may not accurately reflect true CBCEs. For example, Abs on the complexes may be missing their heavy or light chains, or the 3D structure may only provide Ag-Ab contacts outside the CDR regions. Further, undiscovered CBCEs on any Ag, and the immensely sparse information available on ground truth negative epitopes on an Ag, makes the prediction of true CBCEs even more challenging.

There exist various algorithms for predicting either LBCEs and/or CBCEs. However, although many of those algorithms perform well on their test data, they do not produce the same satisfactory results on new independent test data when they are compared against other algorithms of a similar kind.

In conventional CBCE prediction algorithms, the expected input is either a linear protein sequence of the Ag, or a 3D protein structure file of the Ag. Algorithms that require the 3D protein structure as input generally generate better results than the ones which only need the sequence. However, due to the labour intensive and expensive scale of these laboratory experiments, the 3D protein structure of the Ag will almost certainly not be available at prediction time in most use cases; which limits the use of the 3D structure input algorithms. In the case of algorithms which require a sequence as input, it is difficult to capture relationships between spatially distant amino acids, and to distinguish between potentially different CBCEs present on the same antigen.

There is therefore a desire to improve the prediction of B-Cell epitopes, in particular CBCEs.

## SUMMARY OF INVENTION

In accordance with a first aspect of the present invention, there is provided a computer-implemented method of predicting whether a protein comprises a B-cell epitope that is likely to instigate a binding event with an antibody, the method comprising:

(a) accessing the amino acid sequence of the protein;
(b) accessing one or more structure and/or surface characteristics of the protein;
(c) generating one or more candidate encodings of respective one or more candidate B-cell epitopes on the protein, wherein each candidate encoding represents the respective candidate B-cell epitope as a plurality of data elements corresponding to the amino acids of the amino acid sequence; and
(d) using a trained first machine learning model to predict whether any of the candidate B-cell epitope(s) is likely to be a true B-cell epitope on the protein; wherein the input to the trained first machine learning model comprises the amino acid sequence of the protein, the one or more structure and/or surface characteristics of the protein, and the one or more candidate encodings.

The inventors have realised that a common problem with many conventional sequence-based techniques used to predict the presence of a B-Cell epitope on a protein is that the output prediction is typically in the form of a binary value per amino acid indicating whether each particular amino acid in the sequence of the protein is part of a B-Cell epitope. This conventional approach does not allow the user to distinguish between potentially different and distinct BCEs present on the protein that harbor some overlapping amino acid residues, and moreover does not necessarily consider the relationships between spaced-apart amino acids of the entire protein sequence being analysed in 3D conformational BCEs.

To improve upon these conventional techniques for BCE prediction, the present invention applies a trained machine learning model to a combination of the input amino acid sequence of the protein under investigation, and one or more candidate encodings of respective one or more candidate B-cell epitopes, where each candidate encoding represents the respective candidate B-cell epitope.

In this way, the predictions provided by the present invention are made on an "epitope by epitope" basis on the query protein (by considering each candidate encoding corresponding to a candidate epitope), rather than on an "amino acid by amino acid" approach. Thus, the present invention considers whether a candidate B-cell epitope, encoded on the amino acid sequence under test, is a true BCE (or not) on the protein. This approach used by the present invention is particularly advantageous as it provides improved confidence that an epitope as a whole is indeed present (or not) on the protein, rather than having to infer the presence of epitopes (or multiple overlapping epitopes) based on individual amino acid scores. Providing predictions on an "epitope by epitope" basis also provides increased accuracy and confidence with regard to predicting multiple (possibly overlapping) epitopes on the same protein.

Typically, the output of the trained first machine learning model is a probability that each of the candidate encoding(s) represents a true B-cell epitope on the protein.

Herein, the term "true" B-cell epitope is used to mean a B-cell epitope that instigates a binding event with an antibody (e.g., instigating an immunogenic response and/or instigated by an immunogenic response).

The B-cell epitope may be a conformational B-cell epitope. The B-cell epitope may be a linear B-cell epitope. The trained first machine learning model of the present invention is typically able to predict the presence of both linear B-cell epitopes and conformational B-cell epitopes, although in some embodiments different models may be employed for predicting linear and conformational B-cell epitopes respectively, based on the reference data used for training.

In general, the protein may be a protein, protein-domain, or a protein sub-unit. The term "protein" may include any protein subsequence that may have a viable 3D or functional structure. Typically, the protein comprises an antigen. The protein may comprise a neoantigen. The amino acid sequence of the protein may be obtained using techniques known to the skilled person in the art, for example by one of: oligonucleotide hybridisation methods, nucleic acid amplification based methods (including but not limited to polymerase chain reaction based methods), automated prediction based on DNA or RNA sequencing, de novo peptide sequencing, Edman sequencing or any peptide related sequencing including and not limited to mass spectrometry. The amino acid sequence may be downloaded from a bioinformatic depository such as UniProt (www.uniprot.org).

In some embodiments, the one or more candidate encodings represent all possible combinations of the amino acids within the amino acid sequence. This may include both linear B-cell epitopes (where each of the encoded amino acids will be sequentially positioned in the amino acid sequence), and conformational B-cell epitopes (where at least some of the encoded amino acids are spaced apart on the amino acid sequence). However, in some embodiments, prior knowledge of the protein and/or B cell epitope makeup may be used to reduce the search space of the encodings. For example, if the protein comprises a neoantigen, the encodings may be fixed in the position of the mutation point (e.g., each encoding will have the same data element at the position of the mutation point on the amino acid sequence).

The one or more candidate encodings representing combinations of amino acids within the amino acid sequence may be queried through deep reinforcement learning models. The one or more candidate encodings representing combinations of the amino acids within the amino acid sequence may be sourced from generative models. In some embodiments, deep reinforcement learning (DRL) techniques may be used to reduce or query efficiently the large search space.

As outlined above, the candidate encodings of the B-cell epitopes represent the respective candidate B-cell epitope as a plurality of data elements corresponding to the amino acids of the amino acid sequence. Typically, each data element of a candidate encoding (or at least a subset of the plurality of data elements) corresponds to an amino acid of the amino acid sequence of the protein. Preferably, each of the one or more encodings is in the form of a binary vector. In such embodiments, the binary vector will typically be in the form of a sequence of "1"s and "0"s, with the "1"s each corresponding to an amino acid of the protein being a part of the candidate epitope (e.g. a probable Ag-Ab contact point) , and the "0"s each corresponding to an amino acid of the protein not forming part of the candidate epitope (e.g. not a probable Ag-Ab contact point). In some embodiments, different encoding protocols may be used (e.g. one-hot encoding) to represent the candidate epitopes.

The first machine learning model may be (or comprise) any neural network such as convolutional neural networks and feedforward neural networks. Preferably, the first machine learning model is (or comprises) a recurrent neural network. Typically, the first machine learning model is (or comprises) a long short-term memory network, LSTM, preferably a bi-directional long short-term memory network, BLSTM Typically, each amino acid of the query protein sequence is treated as a time-step in the network, whose characteristics can be influenced by both previous and following amino acids. Thus, the entire protein amino acid sequence (e.g. paired with the other input features) may be processed as a "single data point" in contrast to conventional approaches which require segmentation of the protein amino acid sequence in to smaller sub-sequences of amino acids, for example into "k-mers". In this way, the present invention advantageously enables the discovery of relationships between spatially distant amino acids of the protein sequence which could, for example, be part of the same (e.g. conformational) BCE, and which relationships may not be discovered through the "k-mer" approach. The use of a BLSTM is particularly advantageous as this allows the protein amino acid sequence to be processed from both directions.

As described above, in step (b) of the method, one or more (e.g. three-dimensional) structure and/or surface (e.g. surface-exposed) characteristics of the protein are accessed. The structural and/or surface characteristics of the protein being investigated are important for predicting B-cell epitopes (in particular CBCEs), as the Abs are very specific to their binding BCEs. The one or more structure and/or surface characteristics of the protein may be characteristics at the whole protein level (e.g. secondary structure) or at the amino acid level (e.g. features associated with individual amino acids of the protein). The one or more structure and/or surface characteristics may be in the form of continuous features (such as relative solvent accessibility, RSA, or half-sphere exposure, HSA), and/or categorical features such as secondary structure.

In embodiments, the one or more structure and/or surface characteristics include one or more of: the secondary structure of the protein; the relative solvent accessibility, RSA; the half-sphere exposure, HSA. The HSA values may be divided into values for upper half-sphere exposure and lower half-sphere exposure. The SS may be regarded as a structure characteristic, and the RSA and HSE regarded as surface characteristics. A particular advantage of the present invention is that the three-dimensional full folding structure of the input protein (which may be difficult to measure, or may contain a large number of errors if predicted) is not required. Instead, the method accesses one or more structure and/or surface characteristics of the protein which are advantageously more straightforward to obtain than a full folding structure, and typically contain fewer errors. The inventors have found that the characteristics of RSA, HSE and secondary structure, in particular provide, good results for the predictions, as they provide reliable indicators of the three-dimensional structure of the protein. It is envisaged that further structure and/or surface characteristics may be also be used an input to the model. In this way, the inventive method can provide good BCE prediction results by using the amino acid sequence of the input protein, and a limited number of structure and/or surface characteristics thereof, rather than requiring obtaining the full three-dimensional structure of the protein under investigation. In some embodiments, as will be described further herein, the protein sequence may be used in combination with one or more further machine learning model(s) to obtain the structure and/or surface characteristics.

Typically, the accessed structure and/or surface characteristics are in the form of a value and/or a class assigned to each amino acid of the protein amino acid sequence that represents a structure and/or surface characteristic of the protein. In this way, the input to the first machine learning model may be a set of features per amino acid of the protein (e.g., a data element from the binary encoding together with one or more structure and/or surface characteristics for the amino acid).

The method may further comprise accessing one or more physiochemical characteristics of the protein, and/or the amino acids that constitute the protein, and inputting the one or more physiochemical characteristics into the first machine learning model (e.g. in addition to the one or more structure and/or surface characteristics). Examples of such physiochemical characteristics include bulkiness, molar fraction of buried residues, average flexibility index, normalised frequency for alpha helix, known number of codons coding for each amino acid in universal genetic code, polarity, conformational parameter for beta-turn, normalized frequency for beta-sheet, side chain classes, side chain polarity, retention coefficient, hydrophobic constants, normalized frequency of beta-turn, PK1 a-COOH, membrane buried helix parameter, antigenicity. Different sets of physiochemical characteristics may be used for predicting CBCEs and LBCEs. The one or more physiochemical characteristics may be computed (e.g. from the amino acid sequence of the protein) or measured using techniques known in the art. The physiochemical characteristics may be determined for each amino acid of the protein.

Typically, each amino acid (or side chains) that constitutes a protein sequence has physiochemical properties that contribute in some manner to its SS and 3D properties. In this way, embodiments that include the use of physiochemical characteristics in addition to the one or more structure and/or surface characteristics may further enhance the performance of the first machine learning model.

In general, each of the structure and/or surface characteristic(s) and physiochemical characteristic(s) (where used) may contribute separately or in combination to the training and subsequent use of the first machine learning model to predict whether a protein contains a B-cell epitope.

Typically, the first machine learning model is trained using a first reference dataset that comprises:

(i) a plurality of amino acid sequences of respective first reference proteins, each first reference protein comprising at least one true B-cell epitope,
(ii) a plurality of reference encodings of each true B-cell epitope, wherein each reference encoding represents the respective true B-cell epitope as a plurality of data elements corresponding to the amino acids of the amino acid sequence; and
(iii) one or more structure and/or surface characteristics of each first reference protein.

In this way, the first machine learning model may be trained to learn a relationship between the reference encodings, the structure and/or surface characteristic(s) of the first reference proteins, and the corresponding true B-cell epitopes (e.g. classified as true B-cell epitopes on the first reference proteins). In some embodiments, the first reference dataset may further comprise one or more physiochemical characteristics of the first reference proteins, and/or the amino acids that constitute them.

The reference encodings of each true B-cell epitope used for the first reference dataset are typically generated in the same manner as described above.

In particularly preferred embodiments, the one or more structure and/or surface characteristics of each first reference protein are one or more structure and/or surface characteristics of each first reference protein in an unbound state. During binding between a B-cell epitope and an antibody, the three-dimensional structure of the BCE may be altered. Therefore, a key goal of the present invention is to predict true B-cell epitopes in their unbound state (e.g. the unbound state of protein antigens): in other words, the state of the epitope(s) without influence from other proteins, structures or interacting molecules (e.g., before a binding event has taken place). Importantly, this unbound state is what the Ab "sees" and initiates the binding event, and not what an Ab has already "seen" and bound on. This aspect of the invention provides a key advantage over state-of-the-art models that are trained on Ag-Ab complexes (i.e., proteins in a bound state), and which may lead to errors as the predictions are made based on structural data obtained after the binding event has occurred. For example, the 3D structure of the epitope in the bound Ag-Ab complex may differ from the 3D structure of the epitope in its unbound ("native") state.

In this way, the first reference dataset used for training the first machine learning model is preferably generated to predict true B-cell epitopes before the binding event has taken place. As will be discussed in further detail herein, in some preferred embodiments, the structure and/or surface characteristics of each first reference protein may be accessed by predicting the structure and/or surface characteristics using one or more second trained machine learning model(s) trained on reference proteins in an unbound state.

Referring now back to step (b) of the inventive method, the structure and/or surface characteristics of the protein (e.g., in its unbound state) may be obtained using known techniques. For example, known algorithms (including computational approaches to predicting full 3D protein structures) or X-ray crystallography techniques, may be used to predict or measure the features of the protein. For example, RSA, HSE and secondary structure (and other) parameters could be accessed from a predicted or experimental full 3D structure of the protein, without the full structure itself being required as input to the model.

However, in particularly preferred embodiments, the one or more structure and/or surface characteristics accessed in step (b) are predicted by applying one or more trained second machine learning model(s) to the amino acid sequence of the protein accessed in step (a), wherein the one or more trained second machine learning model(s) are trained on a second reference dataset that comprises a plurality of amino acid sequences of respective proteins and their corresponding structure and/or surface characteristics. Preferably, the one or more structure and/or surface characteristics of the protein accessed (e.g. predicted) in step (b) are one or more structure and/or surface characteristics of the protein in an unbound state.

In this way, structure and/or surface characteristics of the protein under investigation may advantageously be computed directly from the amino acid sequence, with no requirement for extra information to be sourced or input by the user. Different learning models may be used to predict continuous features and categorical features. Typically, the output of the trained second machine learning model(s) is a value and/or a class assigned to each amino acid of the protein amino acid sequence that represents a structure and/or surface characteristic of the protein.

In preferred embodiments, the one or more structure and/or surface characteristics of each first reference protein are predicted by applying one or more trained second machine learning model(s) to the corresponding amino acid sequence, wherein the one or more trained second machine learning models are trained on a second reference dataset that comprises a plurality of amino acid sequences of respective proteins and their corresponding structure and/or surface characteristics.

Thus, the same second machine learning model(s) may be used to predict the structure and/or surface characteristics of the query protein, and the structure and/or surface characteristics of the reference proteins used to train the first machine learning algorithm. In some embodiments, different second machine learning models may be used to predict different characteristics. For example, in one embodiment a trained second machine learning model may be used to predict the secondary structure of a protein, and a different trained second machine learning model used to predict RSA and HSE characteristics.

In particularly preferred embodiments, the one or more second machine learning model(s) are trained on a second reference dataset that comprises a plurality of amino acid sequences of respective second reference proteins and their corresponding (e.g., three-dimensional) structure and/or surface characteristics in an unbound state. This enables the predictions of the structure and/or surface characteristics made by the second machine learning model(s) to be predictions of these features in the protein's unbound state (for example, before having been bound by an Ab). This provides particular advantages to the invention as has been outlined above, particularly when generating the first reference dataset used to train the first machine learning model.

Typically, in addition to the amino acid sequence, the input to the one or more second machine learning model(s) comprises a plurality of characteristics (e.g. features) of the first reference protein. The input characteristics are typically physiochemical characteristics but may in some embodiments include structure and/or surface characteristics. These characteristics are typically computed or measured per amino acid of the first reference protein using techniques known in the art. Examples of such (e.g. physiochemical) characteristics include one or more of hydrophobicity, side chain class, side chain polarity (e.g. for predicting HSE and RSA), and one or more of conformational parameter for coil, conformational parameter for alpha helix (e.g. for predicting secondary structure). Further characteristics may be used. In this way, the one or more second machine learning model(s) may be trained on the second reference dataset to learn a relationship between one or more (e.g. physiochemical) characteristics of the amino acid sequences of the respective second reference proteins, and the corresponding structure and/or surface characteristics (e.g. one or more of RSA, HSE, Secondary Structure) in an unbound state. Typically, the second machine learning algorithm(s) utilise a different set of protein characteristics from the first machine learning algorithm.

The second machine learning model(s) may be (or comprise) any neural network such as convolutional neural networks and feedforward neural networks. Typically, the one or more trained second machine learning model(s) is (or comprises) a recurrent neural network, preferably a long short-term memory network, LSTM, more preferably a bi-directional long short-term memory network, BLSTM.

One problem with conventional approaches to predicting B-cell epitopes, especially CBCEs, is a limited amount of data defining true CBCEs. Embodiments of the present invention therefore provide a method of obtaining first reference proteins each comprising at least one true B-cell epitope.

Thus, in some embodiments, at least some of the first reference proteins may be obtained by:

(i) accessing a plurality of (preferably non-obligate) protein complexes comprising at least three different protein chains;
(ii) filtering the plurality of protein complexes to leave only those having a protein chain mapped as a valid heavy ($V_H$) chain of an antibody, a protein chain mapped as a valid light ($V_L$) chain of an antibody, and a protein chain mapped as an antigen;
(iii) pairing the $V_H$ and $V_L$ chains to form $V_H$ and $V_L$ chain pairs; and
(iv) for each $V_H$ and $V_L$ chain pair, defining a true B-cell epitope on the corresponding antigen; wherein

at least some of the first reference proteins of the first reference dataset correspond to an antigen mapped in step (ii) that comprises at least one true B-cell epitope defined in step (iv).

The first reference proteins obtained or identified in the manner above are typically mapped onto its corresponding amino acid sequence. In this way, the structural and/or surface characteristics of the reference protein in an unbound state may be obtained, for example using the one or more trained second machine learning model(s) discussed above.

Typically, the protein complexes accessed in step (i) may be of any organism or entity assigned a taxonomy ID in established authorities such as the NCBI. This advantageously allows the predictions made by the trained first machine learning model to be non-Ab specific.

In embodiments, each true B-cell epitope is defined as the amino acids of the corresponding antigen in contact with the corresponding $V_H$ and $V_L$ chain pair, preferably wherein the amino acids are defined as being in contact with the corresponding $V_H$ and $V_L$ chain pair if they comprise any atoms within a predefined distance of the complementarity-determining region(s), CDR(s) of the $V_H$ and $V_L$ chain pair, preferably wherein the predetermined distance is equal to or less than 4 Angstroms.

As discussed above, the present invention advantageously generates encodings of one or more candidate B-cell epitopes to be tested in order to model and "test" each candidate epitope as a whole. Due to this approach, the first reference dataset used to train the first machine learning model may only contain observed true positive BCEs (i.e. only "positive" training data). Therefore, in some preferred embodiments, the method may further comprise generating pseudo-random data based on the plurality of reference encodings of each true B-cell epitope of the first reference dataset, and wherein the first reference dataset comprises said pseudo-random data as negative data. In this way, the performance of the first machine learning model may be improved as the model can be trained even in the absence of verified negative data.

The pseudo-random data is based on the encoded true B-cell epitope(s). Preferably, the pseudo-random data comprise a plurality of permutations of the plurality of data elements representing the true B-cell epitopes. In some embodiments, the pseudo-random data may be random permutations of the data elements. In some embodiments, the pseudo-random data may share at least one characteristic with the encodings of the true B-cell epitope(s). The characteristic may be physical property of the true B-cell epitope, for example a position on the amino acid sequence of one or more amino acids (e.g. the first and last amino acids of an epitope). In another example, the characteristic may be a linear distance between particular amino acids.

In a further aspect of the invention there is provided a method of training a machine learning model, comprising:

generating a reference dataset, the reference dataset comprising:

a plurality of amino acid sequences of respective reference proteins, each reference protein comprising at least one true B-cell epitope;
a plurality of reference encodings of the true B-cell epitope(s), wherein each reference encoding represents the respective true B-cell epitope as a plurality of data elements corresponding to the amino acids of the amino acid sequence; and
one or more structure and/or surface characteristics of each reference protein; and

training the machine learning model using the reference dataset to learn a relationship between the candidate encodings, the structure and/or surface characteristic(s), and the corresponding true B-cell epitopes (e.g. classified as true B-cell epitopes on the protein).

Thus, the method of training a machine learning model provides the advantages described above, and may be used in combination with any of the described examples of the invention. Advantageously, the machine learning model, once trained, may be used to predict whether a query protein comprises a B-cell epitope that is likely to instigate a binding event with an antibody.

Typically, the one or more structure and/or surface characteristics of each reference protein are one or more structure and/or surface characteristics of each first reference protein in an unbound state.

The reference dataset may further comprise one or more physiochemical characteristics of each protein, and/or the amino acids that constitute the reference protein, as described above.

The machine learning model may be (or comprise) any neural network such as convolutional neural networks and feedforward neural networks. Preferably, the machine learning model is (or comprises) a recurrent neural network. Typically, the first machine learning model is (or comprises) a long short-term memory network, LSTM, preferably a bi-directional long short-term memory network, BLSTM.

The invention also provides use of a machine learning model trained using the method described above (e.g. to predict the presence of BCEs on a query protein). The invention also provides a machine learning model trained using the method described above.

The invention may further comprise synthesising one or more proteins that are predicted to contain a B-cell epitope, and/or a predicted B-cell epitope.

Protein(s) that are predicted by the present invention to comprise a B-cell epitope may be used in the treatment or prevention of infectious diseases or other diseases such as autoimmune or immune-related diseases and cancer. The present invention finds use in one of more of the following use cases:

- Therapeutic antibody mapping
- Vaccine development and creation (both prophylactic and therapeutic)
- Immuno-diagnostics and immune monitoring.

In some embodiments, the method may further comprise encoding one or more proteins that are predicted to contain a B-cell epitope, and/or a predicted B-cell epitope, and/or B-cell epitope variant, and/or B-cell epitope predicted or simulated variant into a corresponding protein, peptide, DNA or RNA sequence. DNA or RNA sequences may be incorporated into a delivery system for use in a vaccine (e.g. using naked or encapsulated DNA, or encapsulated RNA). The method may comprise incorporating the protein, peptide, DNA or RNA sequence into a genome of a bacterial or viral delivery system to create a vaccine.

Thus, in accordance with a further aspect of the present invention, there is provided a method of creating a vaccine, comprising:

predicting that a protein comprises a B-cell epitope by a method according to any of the examples discussed above; and

synthesising the protein and/or the predicted B-cell epitope, or encoding at least one of the protein, the predicted B-cell epitope, B-cell epitope variant, B-cell epitope predicted or simulated variant, into a corresponding protein, peptide, DNA or RNA sequence.

Such a protein, peptide, DNA or RNA sequence may be delivered in a naked or encapsulated form or incorporated into a genome or cell of a bacterial or viral delivery system to create a vaccine. In addition, bacterial vectors can be used to deliver the DNA in to vaccinated host cells. For peptide vaccines, the identified protein(s) may typically be synthesised as an amino acid sequence or "string".

Such a vaccine may be a prophylactic or therapeutic vaccine. For example, the method may be used to create a personalised vaccine for an individual, for example a cancer therapeutic vaccine if the protein comprises a neoantigen.

In accordance with a further aspect of the present invention, there is provided a method of creating (e.g. and/or designing) a diagnostic assay to determine whether a patient has or has had a cancer or prior infection with a pathogen, wherein the diagnostic assay is carried out on a biological sample obtained from a subject, comprising identifying at least one protein of the pathogen or tumor that is predicted to comprise a B-cell epitope, using a method according to any of the examples discussed above; wherein the diagnostic assay comprises the utilisation or identification within the biological sample of the identified at least one protein and/or B-cell epitope.

In this way, the present invention may advantageously be used to create a diagnostic test or assay, by means of a rapid and/or automated molecular target discovery. Query proteins identified as being likely to comprise a true B-cell epitope may be further analysed in laboratory testing in order to create such a diagnostic test or assay, thereby significantly reducing the time taken to develop the test compared to traditional laboratory methods.

The term utilisation as used herein is intended to mean that the at least one protein and/or B-cell epitope thereon are used in an assay to identify an (e.g. protective) immune response in a patient. In this context, the identified protein(s) and/or epitope(s) within are not the target of the assay, but a component of said assay.

The *in vitro* diagnostic assay may comprise identification of an immune system component within the biological sample that recognises at least one epitope within the identified protein. In this way, the diagnostic assay may utilise the at least one identified protein and/or at least one predicted epitope. Typically, the diagnostic assay will contain the (e.g. synthesised) at least one identified protein, protein subunit, and/or predicted epitope. In a preferred embodiment, the immune system component may be a B-cell. For example, the assay may comprise identification of antibodies or B-cells that recognise predicted B-cell epitopes within the identified protein.

As an example of such a diagnostic use, a sample, preferably a blood sample, isolated from a patient may be analysed for the presence of B-cells or antibodies within the biological sample that recognise and bind to epitope(s) within the identified protein(s), identified as part of the present invention and that are contained within the assay.

Suitable diagnostic assays would be appreciated by the skilled person, but may include enzyme-linked immune absorbent spot (ELISPOT) assays, enzyme-linked immunosorbent assays (ELISA), cytokine capture assays, intracellular staining assays, tetramer staining assays, microfluidic devices, lab-on-a-chip, microarrays, flow cytometry, CyTOF, proteomics, molecular sequencing, or limiting dilution culture assays.

In a method of creating a diagnostic test, the amino acid sequence of the one or more proteins to be tested for the presence of BCEs may be chosen based on the desired response to be tested. For example, the one or more source proteins may be one or more source proteins of any pathogen and/or virus (or fragments thereof), such as the SARS-CoV-2 virus. In such a case, the present invention may be used to create a diagnostic test for determining whether a patient has or has had prior infection with the SARS-CoV-2 virus, and/or its variants and/or related viral species. However, as will be appreciated by the skilled person, the one or more source proteins may be from any pathogen (e.g. any virus, parasite, bacterium, or cancer indication).

Further disclosed herein is a diagnostic assay to determine whether a patient has or has had prior infection with a pathogen and/or cancer, wherein the diagnostic assay is carried out on a biological sample obtained from a subject, and wherein the diagnostic assay comprises the utilisation or identification within the biological sample of at least one protein of the pathogen or tumour that is predicted to comprise a B-cell epitope, identified using any of the methods discussed above. The diagnostic assay may comprise identification of an immune system component (e.g. a B-cell) within the biological sample that recognises said at least one identified protein and/or at least one predicted epitope.

In accordance with a further aspect of the invention there is provided a computer program product comprising instructions which, when executed by a computer, cause the computer to perform the method of any of the examples described above. The invention also provides a non-transitory computer readable medium comprising executable instructions that, when executed by a computer, cause the computer to perform the method of any of the examples described above.

In accordance with a further aspect of the invention, there is provided a system for predicting whether a protein comprises a B-cell epitope that is likely to instigate a binding event with an antibody, comprising at least one processor in communication with at least one memory device, the at least one memory device having stored thereon instructions for causing the at least one processor to perform a method in accordance with any of the examples of the invention discussed above.

In accordance with a further aspect of the invention, there is provided a method of synthesising a protein, comprising: predicting that a protein comprises a B-cell epitope that is likely to instigate a binding event with an antibody according to any of the examples discussed above; and synthesising the predicted protein. The synthesis of the predicted protein can be performed using techniques known in the art. In accordance with a further aspect of the invention, there is provided a protein synthesised using the said method.

## BRIEF DESCRIPTION OF THE DRAWINGS

Embodiments of the invention will now be described with reference to the accompanying figures, in which:-

Figure 1 is a flow diagram illustrating the steps of a method according to an embodiment of the invention;
Figure 2 is a flow diagram illustrating the steps of a method of training a first machine learning algorithm according to an embodiment of the invention;
Figure 3 is a flow diagram illustrating the steps of a method of training a second machine learning algorithm according to an embodiment of the invention;
Figure 4 is an example of a system suitable for implementing embodiments of the method is shown;
Figure 5 is an example of a suitable server;
Figures 6(a) to 6(f) show the correlation results of the feature-feature pairs;
Figure 7 shows the Spearman's correlation coefficients for continuous features against (a) RSA, (b) UHSE and (c) LHSE;
Figure 8 shows the Kendall's correlation coefficients for categorical features against (a) RSA, (b) UHSE and (c) LHSE;
Figure 9 shows the Kendall's correlation coefficients for continuous features against (a) SS, (b) CBCE and (c) LBCE;
Figure 10 shows the Mutual Information criterion for categorical features against (a) SS, (b) CBCE and (c) LBCE;
Figure 11 schematically shows the structure of a Prediction model for RSA and HSE according to an embodiment of the invention;
Figures 12(a) to (f) show cross-validation results for a RSA/HSE model according to an embodiment of the invention;
Figures 13(a) to (f) show test results for a RSA/HSE model according to an embodiment of the invention;
Figures 14(a) to (f) show final training results for a RSA/HSE model according to an embodiment of the invention;
Figures 15(a) to (f) illustrate CV results for a Secondary Structure (SS) prediction model according to the invention;
Figures 16(a) and (b) show the test results for SS model;
Figures 17(a) to (d) show final training results for the SS model according to an embodiment of the invention;
Figures 18(a) and 18(b) show Paratope Analysis for CBCE;
Figure 19(a) schematically illustrates the architecture of a first machine learning algorithm for predicting CBCEs, according to an embodiment of the invention;
Figure 19(b) schematically illustrates the architecture of a first machine learning algorithm for predicting CBCEs, according to a further embodiment of the invention;
Figures 20(a) to (d) illustrate outlier analysis for CBCEs;
Figures 21(a) to (f) shows results for the precision-recall metric for a first machine learning algorithm for predicting CBCEs, according to a further embodiment of the invention;
Figures 22(a) to (f) shows results for the precision-recall metric for a first machine learning algorithm for predicting CBCEs, according to an embodiment of the invention;
Figures 23(a) and (b) shows the results for the precision-recall metric for a first machine learning algorithm for predicting CBCEs, according to an embodiment of the invention;
Figures 24(a) and (b) shows the results for the precision-recall metric for a first machine learning algorithm for predicting CBCEs, according to an embodiment of the invention;
Figures 25(a) to (c) shows the results of final training for a first machine learning algorithm for predicting CBCEs, according to an embodiment of the invention;
Figures 26(a) and (b) show the results of final training for a first machine learning algorithm for predicting CBCEs, according to an embodiment of the invention;
Figures 27(a) to (d) illustrates outlier analysis for LBCEs; and
Figure 28 schematically illustrates a pipeline according to an embodiment of the invention.

## DETAILED DESCRIPTION

### 1. Overview

Embodiments of the invention provide a B-cell epitope (BCE) prediction algorithm ("first machine learning algorithm" or "first machine learning model"), trained using BLSTMs. The model accurately predicts BCEs on a given Ag from its primary sequence in combination with one or more 3D structure and/or surface characteristics of the Ag, with no requirement for the full 3D structure of the Ag as an input into the predictor. The model is trained using BLSTMs, which allows for the processing of the whole Ag sequence as one data-point, processed from both directions without the need for *k-mer* segmentation. Each amino-acid of the Ag is treated as a time-step in the network, whose characteristics can be affected by both preceding and succeeding amino-acids that flank the amino-acid in question, thus, allowing for the discovery of the contextual relationships between spatially distant amino-acids which could be important parts of conformational BCEs (CBCEs).

The first machine learning model is capable of discovering separate or independent CBCEs that may exist on a single Ag protein sequence. A particularly advantageous feature of the model is that it takes as input an Ag protein sequence and a binary "permutation vector" or "encoding" representing each single candidate CBCE (or LCBE), as well as the one or more 3D structure and/or surface characteristics of the Ag. The output of the model is a single probability indicating the likelihood that the input permutation vector is a *bone fide* CBCE. In other words, the user asks the question to the model if the specific query CBCE, encoded as a binary permutation vector on the given Ag sequence, is a true CBCE or not. However, the model may be used to provide a second output in the form of a probability vector with a probability for each amino-acid in the Ag sequence. The second output can be seen as the contribution of each amino acid to the CBCE in question, and may be used to compare the current approach with conventional models that predict on an "amino acid by amino acid" basis. The first machine learning algorithm is preferably trained on unbound 3D Ag protein structures prior to the Ag-Ab binding event, to learn the properties of BCEs on the Ag prior to the Ab binding event, in order to capture the *bone fide* ("true") BCEs on the query Ag protein sequence.

Structural characteristics of the Ag sequence are important for predicting CBCEs. However, embodiments of the present invention negate the need for the complete 3D structure of the protein sequence to be experimentally measured or predicted, and then used as input into the algorithm. The first machine learning algorithm does not require the coordinates of each atom in each amino acid in the 3D protein sequence (i.e. it does not require the full 3D structure), and instead may predict BCEs using one or more structure and/or surface characteristics of the query protein, such as RSA, HSE and SS.

The "top-level" most important features such as RSA, HSE and SS, may be predicted with one or more trained machine learning algorithms ("second machine learning algorithm" or "second machine learning model"), typically receiving as input the amino acid sequence of the protein. In this way, structural and/or surface characteristics of the protein under investigation may advantageously be computed directly from the amino acid sequence, with no requirement for extra information to be sourced or input by the user. Those second algorithms use BLSTM networks also and are shown to have a high performance.

In this way, the first and second machine learning models may together form a "pipeline" which requires as input only the amino acid sequence of a query protein in order to predict whether that query protein comprises a true B-cell epitope. A schematic diagram of such a pipeline constituting the first and second machine learning models according to an embodiment of the invention is shown in Figure 28.

Figure 1 is a flowchart 100 summarising the steps of a preferred embodiment of the present invention. At step S101, the amino acid sequence of a protein to be analysed for the presence of B-cell epitopes is accessed. Typically the protein under investigation will comprise an antigen. Various techniques known to the skilled person may be used to access the amino acid sequence, for example the sequence may be downloaded from a bioinformatics repository such as UniProt.

At step S103, one or more (e.g. three-dimensional) structure and/or surface characteristics of the protein in an unbound state are accessed. Typically, these characteristics of the protein are predicted using one or more trained second machine learning model(s) (schematically illustrated at 300, and described below) that receives the amino acid sequence accessed in step S101 as input. The structure and/or surface characteristics typically include the secondary structure of the query protein and relative solvent accessibility. The half sphere exposure (both upper and lower half sphere exposure) may also be used. Values or categories for each of these characteristics are typically assigned to each amino acid of the sequence. However, other techniques for accessing the three-dimensional structure and surface characteristics of the query protein may be employed, such as database entries, X-ray crystallography or computational approaches to predicting full 3D protein folding structures (from which the structure and/or surface characteristics can be obtained).

At step S105, candidate encodings of one or more candidate BCEs on the query protein are generated. The candidate encodings are each typically in the form of a binary vector, with each data element in the binary vector corresponding to an amino acid of the amino acid sequence forming the respective candidate BCE. In such a binary "permutation vector" encoding, each amino acid of the sequence forming part of the candidate BCE is assigned a "1" and each amino acid of the sequence not forming part of the candidate BCE is assigned a "0". For example, for the exemplary amino acid sequence "ABCDEFG", a candidate conformational B-cell epitope constituted by the amino acids B, D, F and G may be encoded as the binary vector (0101011). In some embodiments, a candidate encoding for each possible candidate BCE on the protein may be generated. However, in order to reduce computational load, the search space may be limited based on physical priors (e.g. knowledge of a mutation point on a neoantigen of the input protein or receptor binding domain on the spike protein of SARS-CoV-2, etc) or on techniques such as deep reinforcement learning or related generative models.

In step S107, the amino acid sequence accessed in step 101, the one or more structure and/or surface characteristics accessed in step 103, and the candidate encodings generated in step S105, are input into a trained first machine learning model, which has been trained using BLSTMs. One or more physiochemical characteristics assigned to each amino acid of the amino acid sequence may also be used as input to the trained first machine learning model. The output of the trained first machine learning model is a probability that each of the candidate encoding(s) represents a true B-cell epitope on the query protein.

Figure 2 is a flow diagram 200 summarising the steps of a preferred embodiment of training the first machine learning model. At step S201, a plurality of protein complexes are accessed. These may be accessed from publicly available sources such as the Protein Data Bank (PDB) for CBCEs or Immune Epitope Database (IEDB) for LBCEs.

At step S203, the protein complexes accessed in step S201 are analysed to define true epitopes and antigens. Linear BCEs may be defined in the complexes of the IEDB. Further analysis may be required to define true conformational epitopes from protein complexes accessed from the PDB, and further details on these techniques are described herein.

At step S205, each antigen defined in step S203 as having a true B-cell epitope is mapped onto its amino acid sequence. At step S207, each true epitope is encoded onto the amino acid sequence of the corresponding antigen. In this way, a plurality of reference epitope encodings is generated, corresponding to the true epitopes. The reference encodings are typically encoded as binary vectors in the same manner as described above in step S105.

At step S209 of the method, three-dimensional structure and/or surface characteristics of the antigens in an unbound state are accessed, for example by prediction from the amino acid sequence of the antigen obtained in step S205. Typically, the structure and/or surface characteristics of the antigens are predicted using the trained second machine learning model(s) (schematically illustrated at 300). However, other techniques of accessing the structure and/or surface characteristics of the antigens in an unbound state may be used, for example using known computational approaches for predicting 3D protein structures. Typically, the characteristics include the secondary structure, half sphere exposure (both UHSE and LHSE), and relative solvent accessibility.

At step S211, a first reference dataset is generated. The first reference dataset comprises the amino acid sequences of the reference antigens (step S205), the encodings of the true epitopes (step S207) on the corresponding reference antigens, and the accessed (e.g. predicted) structure and/or surface characteristics of the reference antigens in an unbound state. In some embodiments the first reference dataset may further comprise one or more physiochemical characteristics assigned to each amino acid of the reference antigens.

At step S213, the first machine learning model is trained using the first reference dataset generated in step S211. Here, the first machine learning model is or comprises a BLSTM, and may be trained using techniques known in the art. By training the model using the first reference dataset generated through performing the steps S201 to S211, the first machine learning model is trained to learn a relationship between the reference encodings of the true epitopes, the structure and/or surface characteristic(s), and the corresponding true B-cell epitopes.

As discussed above with reference to Figures 1 and 2, in preferred embodiments the second machine learning model(s) (schematically shown at 300) is used to predict structure and/or surface characteristics both when training the first machine learning model (Figure 2), and when using the trained first machine learning model to predict the presence of B-cell epitopes on a protein of interest (Figure 1). A particularly advantageous feature of the use of the second machine learning model is that it is trained to predict structure and/or surface characteristics of the protein in an unbound state. In this way, the first machine learning model is configured to predict BCEs in their unbound state, i.e., having the three dimensional form and characteristics that the Ab "sees" and initiates binding upon.

Figure 3 is a flow diagram summarising the steps of a preferred embodiment of training the second machine learning model.

At step S301, a plurality of second reference proteins in an unbound state (i.e., single protein structures) are accessed. These second reference proteins may be accessed from publicly available databases such as the PDB.

At step S303, the amino acid sequences of the second reference proteins are accessed.

At step S305, the three-dimensional structure and/or surface characteristics of the second reference proteins are accessed. Typically, these include the secondary structure, the relative solvent accessibility, and the upper and lower half sphere exposures. The DSSP algorithm *(*Wolfgang Kabsch and Christian Sander. Dictionary of protein secondary structure: Pattern recognition of hydrogen-bonded and geometrical features. Biopolymers, 22(12):2577-2637, 2018/11/30 December 1983) was used to access the secondary structure and RSA chracteristics, and the BioPython package *(*Peter JA Cock, Tiago Antao, Jeffrey T Chang, Brad A Chapman, Cymon J Cox, Andrew Dalke, Iddo Friedberg, Thomas Hamelryck, Frank Kauff, Bartek Wilczynski, et al. Biopython: freely available python tools for computational molecular biology and bioinformatics. Bioinformatics, 25(11):1422-1423, 2009*)* utilised to compute the UHSE and LHSE. The DSSP and BioPython algorithms may be applied to the structure files of the second reference proteins (e.g. obtained from the Protein Data Bank, PDB)

At step S307, a second reference dataset is generated using the amino acid sequences accessed in step S303, and the structure and/or surface characteristics of the second reference proteins accessed in step S305.. In some embodiments the second reference dataset may further comprise one or more physiochemical characteristics assigned to each amino acid of the second reference proteins.

At step S309, the second machine learning model is trained using the second reference dataset (e.g. to learn a relationship between one or more features of the amino acid sequences of the second reference proteins and the corresponding structure and/or surface characteristics). Here, the second machine learning model is or comprises a BLSTM, and may be trained using techniques known in the art. It will be appreciated that different second machine learning models may be trained to predict different respective structure and/or surface characteristics. For example, one second machine learning model may be trained to predict RSA and HSE metrics, and further second machine learning model trained to predict the secondary structure.

The various steps outlined in Figures 1-3 will be described in further detail in the following sections. Section 2 explains pre-training techniques used for all the models. Section 3 presents some preliminary analyses we performed to choose input features for the models. Section 4 presents the structural algorithms for RSA, HSE and SS used in the current BCE algorithms. Section 5 presents the structural algorithms for RSA, HSE and SS that are used as features in the BCE model. Section 6 presents an example of a first machine learning algorithm according to the invention, used to predict CBCEs, while Section 7 presents an outline of a first model used to predict linear BCEs (LBCEs). In section 8 we present applications of the present invention.

## 2 Pre-training Techniques

To ensure reproducible results and avoid the need of random seed in the networks used in our models, we used auto-encoders (aEs). aEs are types of NNs trained on compressed data which mirrors the inputs of the main model. More specifically, an AE takes an input at layer number $i = 0$ and processes it through an arbitrary number of layers, say $i = 1, ..., N$, which constitute the encoder part. It then processes it back through a mirrored structure of the encoder part, called the decoder part. Finally, it returns the output which is the same as the input. The intermediate layers may reduce or expand the dimensions of the input.

Each layer in the main network is converted into an AE, and trained on some compressed amount data. The pre-trained weights of the outer layers are used as constants to the subsequent aEs. This is repeated until all the main network layers are pre-trained. The pre-trained weights can then be used as starting points for the main training procedure.

The following procedure were performed once and the pre-trained weights were used on all the cross-validation folds, test of unseen data, and final model.

First, we randomly generated 10000 protein sequences of length between 50 to 1000 amino-acids. The actual amino-acids used was random as well. We then pre-trained each layer of the neural network as an individual AE on the whole generated data. The pre-training was done for maximum 1000 epochs for each AE, with the possibility of early stopping if the loss did not decrease after 10 epochs. The weights of the outer layers were copied to deeper aEs after pre-training, and were also kept constant during the pre-training of those. The last layer of the model (output) was not pre-trained at all.

This procedure was done once and the same pre-trained weights were used on all the validation procedures. This procedure was faster, it used much more data to pre-train and the same initial weights were used for all downstream analyses.

3 Preliminary Analysis and Feature Selection Techniques

We ran various preliminary analyses in order to see how different features correlate with the targets we wanted to predict, as well as with each other. The goal of those analyses was two-fold. First, we tried to find highly feature-targets correlations which would help us decide which features to use in order to predict the targets. Secondly, feature-feature correlations would help us subset the feature-target correlation space by keeping features which were not correlated with each other, thus, reducing the input space of the models. Note, however, that all the analysed using this selection technique mostly capture linear-relationships between feature-targets and feature-features.

3.1 First Method

For deciding which features will be used as input to predict different targets of the models we performed a correlation analysis using the features in Table 0. The analysis was done using the whole data for those models. Namely, the data-set used for those analyses was the ones described in section 4.1. This first method of analysis was run for the 3D structure prediction models and not for the CBCE or LBCE models.

3.1.1 Pre-processing continuous features

Continuous features provide a single value per amino-acid in the alphabet. To model the effect of neighbour amino-acids in a protein sequence, we averaged the values of each feature by scanning the protein sequence using a sliding window of a peptide length of 9 amino-acids. The average feature value was assigned to the middle amino-acid in the peptide. This was done for every feature on each protein sequence in the entire training and test datasets.

3.1.2 Correlation between continuous features and continuous targets

These analyses were done for RSA (relative solvent accessibility), UHSE (upper half-sphere exposure) and LHSE (lower half-sphere exposure). First, we pre-processed the continuous features as described in section 3.1.1. The values of the target variables were used as they were, that is not averaged, since their value already represents neighbours. The Spearman's correlation coefficient was computed for every window-averaged feature in each protein sequence and every target variable. Correlations from all the proteins were then averaged per feature-target pair. For all the three targets we then chose the 30 most correlated features (positively or negatively), and we chose features that were highly correlated with all the three targets.

3.1.3 Correlation between categorical features and continuous targets

These analyses were done for RSA, UHSE and LHSE. Categorical features provide a single value per amino-acid. Those features were used as they are, per-amino-acid in any given sequence. The point-biserial correlation coefficient between feature and target was computed per class. The targets were also used as they are, that is, no windows, for the same reason as in section 3.1.2. Correlations from all the proteins were then averaged per feature-target pair. The top highest (positively or negatively) correlated features with all the three targets were chosen as inputs in the model.

3.1.4 Correlation between continuous features and categorical targets

This analysis was done for SS. The same procedure was used as in Section 3.1.2 for the windows of the features. The only difference is that the target is categorical. For assessing the correlation with continuous features, we used the point-biserial correlation coefficient. Correlations from all the proteins were then averaged per feature-target pair. Top correlated features (positively or negatively) were used in the models.

3.1.5 Correlation between categorical features and categorical targets

This analysis was done for SS. For assessing the dependency between categorical features-targets we used the Chi-Square on each protein. We then did multiple hypothesis correction of the resulting p-values using the Benjamini-Hochberg procedure. Finally, we averaged their corrected p-values from all the sequences, for each feature-response pair, using the harmonic mean. The top most significant feature-target correlations were used in the models.

3.2 Second Method

For deciding which features will be used as input to predict different targets of the models we did a correlation analysis using the features in Table 0. Further information on each feature (e.g. characteristic) is given in the Appendix. The analysis was done using the whole data for those models. Namely, the data-set used for those analyses was the ones described in section 4.1 (for RSA, UHSE, LHSE, SS) and section 5.1 and 6.1 (for CBCE and LBCE). Which data we used depends on the model tested, accordingly. Moreover, for the CBCE and LBCE analyses, those two data-sets were merged.

Table 0: Table of Global Features

| Feature | Abbreviation | Feature | Abbreviation |
|---|---|---|---|
| Mass [25] | MassKnown | Refractivity [30] | REFRJones |
| Bulkiness [29] | BulkZimmerman | Normalized consensus hydrophobicity [31] | NCHPBCEisenberg |
| Hydrophilicity [32] | HPLCParker | Hydrophilicity [1] | HPLCHopp |
| Polarity [2] | POLGrantham | Average surrounding hydrophobicity [15] | ASHPLCManavaian |
| Composition [2] | COMPGrantham | Hydrophobicity of physiological L-alpha amino-acids [33] | HPBCOPLAAABlack |
| Molecular volume [2] | MOLVGrantham | Hydrophobicity [34] | HPBCFauchere |
| Recognition factors [17] | RECFFraga | Free energy of transfer from inside to<br>outside of a globular protein [3] | FEOTITOJanin |
| Hydrophobicity [35] | OMHSweet | Membrane buried helix parameter [5] | MBHPRao |
| Hydropathicity [36] | HPTCKyte | Hydrophobicity [37] | HPBCTanford |
| Hydrophobicity [38] | HPBCAbraham | Antigenicity value X 10 [18] | AVX10Weilling |
| Hydrophobicity [19] | HPBCBull | Hydrophobicity indices at ph 7.5<br>determined by HPLC [39] | HPBCPH75Cowan |
| Hydrophobicity [4] | HPBCGuy | Retention coefficient in HFBA [20] | RCHFBABrowne |
| Hydrophobicity [6] | HPBCMiyazawa | Transmembrane tendency [7] | TransTrendZhao |
| Hydrophobicity [40] | HPBCRoseman | Retention coefficient in HPLC, pH 7.4 [22] | RCHPLCPH74Meek |
| Hydration potential [21] | HPWolfenden | Molar fraction of accessible residues [3] | MOLFAR3220Janin |
| Hydrophobic constants [26] | HPLCWilson | Mean fractional area loss [9] | MFALRose |
| Hydrophobicity indices at ph 3.4 [39] | HPLCph34Cowan | Average area buried on transfer from standard state to folded protein [9] | AABOTRose |
| Mobilities on chromatography paper [41] | MOAAOCPAboderin | Conformational parameter for alpha helix [10] | CPFAH29Chou |
| Retention coefficient in TFA [20] | RCTFABrowne | Conformational parameter for beta-turn [10] | CPFBT29Chou |
| Retention coefficient in HPLC, pH 2.1 [22] | RCHPLCph21 Meek | Conformational parameter for beta-sheet [14] | CPFBSDeleage |

(continued)

| Feature | Abbreviation | Feature | Abbreviation |
|---|---|---|---|
| Molar fraction of buried residues [3] | MOLF2001OBRJanin | Conformational parameter for coil [14] | CPFCDeleage |
| Proportion of residues buried [8] | PORB12Clothia | Normalized frequency for beta-sheet | NFFBSLevitt |
| Average flexibility index [23] | AFIBhaskaran | Conformational preference for total beta strand (antiparallel+parallel) [11] | CPFTBSAPLifson |
| Conformational parameter for beta-sheet [10] | CPFBS29Chou | Conformational preference for total beta strand [11] | CPFTBSLifson |
| Conformational parameter for alpha helix [14] | CPFAHDeleage | Surface accessibility [13] | SASEmini |
| Conformational parameter for beta-turn [14] | CPFBTDeleage | Antigenicity [27] | AGCKolaskar |
| Normalized frequency for alpha helix [16] | NFFAHLevitt | Side chain classes [12, p.51] | SCClassKnown |
| Normalized frequency for beta-turn [16] | NFFBTLevitt | Side chain polarity [12, p51] | SCPolKnown |
| Conformational preference for antiparallel beta strand [11] | CPFABSLifson | Side chain charge [12, p51] | SCPolarityKnown |
| Overall amino-acid composition [28] | OAACMccaldon | Isoelectric points [25] | PLKnown |
| Relative mutability of amino-acids [24] | RMOAADayhoff | Van der Waals volume [25] | VanDerWaalsKnow n |
| Known Number of codon(s) coding for each amino-acid in universal genetic code [12] | NCFAAKnown | PK1 a-COOH [25] | PK1Known |
| Polarity [81] | POLZimmerman | PK2 a-+NH3 [25] | PK2Known |

3.2.1 Pre-processing continuous features

The same procedure as in section 3.1.1.

3.2.2 Feature-Feature correlations

First, we ran a feature-feature correlation analysis of all the features in Table 0. We did this on two different datasets, one for the one in section 5.1 and one for the merged one from sections 6.1 and 7.1. The purpose of those analysis was to identify high correlated feature pairs, such that if both features from a correlated pair were correlated with a response in a later analysis, only one of them will be used as input feature for the corresponding model. Thus, reducing the input features space.

We first pre-processed the continuous features as described in section 3.2.1. Categorical features were used as they are, that is, a single value per-amino-acid in every sequence. We then computed correlation metrics for every feature pair on every sequence. For continuous-continuous feature pairs we used Spearman's Correlation coefficient. For continuous-categorical features we used Kendall rank correlation coefficient, and for categorical-categorical we used Mutual information criterion. For every pair-wise feature coefficient population we removed outliers using the IQR (box-plot) method. The median of the whole population is then taken as the final coefficient for every pair-wise feature-feature correlation. We defined as correlated continuous-continuous feature pairs with median Spearman's correlation coefficient > 0.7, continuous-categorical feature pairs with Kendall rank correlation coefficient > 0.7, and categorical-categorical feature pairs with Mutual Information > 2.

Figure 6 shows the correlation results of the feature-feature pairs for the RSA/HSE model. Figures 6(a) to (c) show feature-feature correlations using the data from 6.1 and 7.1. Figures 6(d) to (f) show feature-feature correlations using the data from 5.1. Black dots correspond to correlated pairs. The heat-maps are symmetric. Note that not all feature names are written in the x- and y-axis. For the 6.1 and 7.1 datasets, the features for RSA, UHSE, LHSE and SS were predicted using the current models (see 4). For the 5.1 dataset, those figures were the observed ones, from PDB. As we can see, for all datasets, continuous-continuous feature-pairs resulted in many correlated features. For continuous-categorical feature-pairs only a few were correlated, while no correlation was observed between categorical-categorical feature pairs.

3.2.3 Feature-response correlations

For each of the following feature-response correlation analyses, we followed the same procedure. For continuous features we did the same pre-processing as described in section 3.1.1, but we used windows of 0, 3, 7, 9 and 11. We computed the correlations based on every window for each feature-response pair in every sequence of the corresponding data. Moreover, we removed outliers using the IQR (box-plot) method, and then computed the median of each correlation population.

We merged all the correlations from every window for each feature into one population, we also removed outliers with the same method, and finally we recomputed the median of that global population. The final "pooled" median was used to rank the features from highest to lowest correlated with the corresponding response. Categorical responses as well as predicted RSA, UHSE and LHSE were used as they are, that is, no windows.

Each of the following analyses gave us an indication of highly correlated feature-response pairs. We chose the top correlated features with the responses in such a way that if two features were correlated with the response and also with each other (using the results from section 3.2.3), only the one feature with the highest correlation with the response was used.

3.2.3.1 Correlation between continuous features and continuous targets

These analyses were done for RSA, UHSE and LHSE. We used data from 5.1. The metric used here was Spearman's Correlation coefficient. See Figure 7 for the results, and section 5 for the models and the features they used.

Figure 7 shows the Spearman's correlation coefficients for continuous features against (a) RSA, (b) UHSE and (c) LHSE. On each figure, the x-axis is the correlation, the y-axis are the feature names, sorted from highest to lowest absolute correlation of the median of the population. Each plot is from all windows.

3.2.3.2 Correlation between categorical features and continuous targets

These analyses were done for RSA, UHSE and LHSE. We used data from 5.1. The metric used here was Kendall rank correlation coefficient. See Figure 8 for the results, and section 5 for the models and the features they used.

Figure 8 shows the Kendall's correlation coefficients for categorical features against (a) RSA, (b) UHSE and (c) LHSE. On each figure, the x-axis is the correlation, the y-axis are the feature names, sorted from highest to lowest absolute correlation of the median of the population. Each plot is from all windows.

3.2.3.3 Correlation between continuous features and categorical targets

These analyses were done for SS, CBCE and LBCE. We used data from 5.1 (for SS) and merged 6.1 and 7.1 (for CBCE and LBCE). The metric used here was Kendall rank correlation coefficient. See Figure 9 for the results, and section 5, 6 and 7 for the models and the features they used.

Figure 9 shows the Kendall's correlation coefficients for continuous features against (a) SS, (b) CBCE and (c) LBCE. On each figure, the x-axis is the correlation, the y-axis are the feature names, sorted from highest to lowest absolute correlation of the median of the population. Each plot is from all windows.

3.2.3.4 Correlation between categorical features and categorical targets

These analyses were done for SS, CBCE and LBCE. We used data from 5.1 (for SS) and merged 6.1 and 7.1 (for CBCE and LBCE). The metric used here was Mutual information criterion. See Figure 10 for the results, and section 5, 6 and 7 for the models and the features they used.

Figure 10 shows the Mutual Information criterion for categorical features against (a) SS, (b) CBCE and (c) LBCE. On each figure, the x-axis is the correlation, the y-axis are the feature names, sorted from highest to lowest absolute correlation of the median of the population. Each plot is from all windows.

4 Predicting 3D features ("Second machine learning algorithm")

We created two BLSTM models to predict structure and surface characteristics of proteins from their native sequences. Those models are later used in the first machine learning model, to predict structural and surface features of the Ag before the binding event with the Ab takes place. For structural characteristics we chose to create a model for the SS, and for surface characteristics we chose RSA, UHSE and LHSE.

4.1 Data preparation for the 3D features

To accurately predict the SS, RSA, UHSE and LHSE from a native protein sequence, we used all available high quality 3D protein structures from the PDB from all available organisms. The goal of these models was to predict the most pertinent surface and structural characteristics of single proteins, that are not affected by binding to any other protein interaction, including Ab binding. Therefore, we only kept structures that are not bound by any other structure or molecule. Structures containing more than one copy of the same molecule, but slightly different conformation, are kept in the data. We filtered the structures and kept only those with $\leq$ 3 Angstrom resolution, ensuring that every atom of each amino-acid is mapped with coordinates, and with protein chains longer than 200 amino-acids.

After filtering, the data-base consisted of 41592 total structures. Those structures contained 70489 protein sequences which resulted in 53524 unique sequences.

Subsequences of longer sequences were kept as different data point entries. The reason being that their 3D conformational folding properties may be different because of their shorter length.

The DSSP algorithm was used to compute the SS and the RSA for each molecule in each structure file. DSSP computes the following secondary structure classes for a protein sequence; a-helix, 310-helix, $\pi$-helix, isolated $\beta$-bridge, $\beta$-strand, turn, bend and coil. We merged those classes into three super-classes; Helices (a-helix, 310-helix and rr-helix), Strands (isolated $\beta$-bridge and $\beta$-strand) and Coils (turn, bend and coil). Finally, the BioPython package was used in to compute the UHSE and LHSE.

At the end of the filtering, each amino-acid in the data base was assigned a value for the RSA, UHSE, LHSE and a class for the SS. To create a unique database, the mean per amino-acid was taken for RSA, UHSE, LHSE among identical sequences. Finally, amino-acids of identical sequences but with different SS classes, were assigned to the coil class.

4.2 Prediction model for RSA and HSE (struct 3d rsa hse m2 model)

We chose to create a single BLSTM model for predicting all surface features. More specifically, the model predicts RSA, UHSE and LHSE from a primary protein sequence. Although LHSE may not give useful information about the surface position of an amino-acid, it may help predicting UHSE more accurately, since both together are forming a probability metric around each amino-acid.

This is a BLSTM model which takes as inputs a batch of features, each computed per amino-acid from each input sequence and predicts a three-way output. For each protein sequence given as input, a value for each RSA, UHSE and LHSE is predicted per amino-acid.

For all the three outputs the individual losses were calculated on the basis of the mean square error (MSE), but in principle the loss is not limited to this function. The global model loss was the weighted sum of the individual losses with weights: 50, 100 and 125 for RSA, UHSE and LHSE, respectively. The weights were decided by first training the model without them and then see the differences in the magnitude of the three losses. The weights contributed in such a way that the three losses gave the same contribution to the global model loss.

Figure 11 shows a schematic simplified architecture of the model. As shown, the first layer is the input layer. The input layer may comprise one or more input nodes, for example to receive the input features discussed below. Each input node is coupled to a respective masking node in a masking layer. Similarly, each masking node is coupled to a respective BLSTM node in a BLSTM layer. Each BLSTM node is coupled to a concatenation node in a concatenation layer. Thus, a plurality of outputs from the nodes of the preceding BLSTM layer are concentrated together in the concatenation layer. The concatenation layer can be coupled to one or more further BLSTM layers, each comprising a corresponding BLSTM node. The final of these BLSTM nodes is coupled to an output layer, which in this case comprises a plurality of output nodes corresponding, respectively, to the RSA, UHSE and LHSE output predictions.

Note: the input features were computed per amino-acid as they are, not averaged by windows.

4.2.1 Preliminary Analysis and Features Used

See procedure in section 3.1 for the procedure. The 14 features that we chose to use in the model (including the protein-sequence in binary representation) can be seen in Table 1.

Table 1: Features used in the struct 3d rsa hse m2 model.

| Feature \| | Abbreviation | Feature | Abbreviation |
|---|---|---|---|
| Polarity [2] | POLGrantham | Free energy of transfer from inside to outside of a globular protein [3] | FEOTITOJanin |
| Hydrophobicity [4] | HPBCGuy | Membrane buried helix parameter [5] | MBHPRao |
| Hydrophobicity [6] | HPBCMiyazawa | Transmembrane tendency [7] | TransTrendZhao |
| Proportion of residues buried [8] | PORB12Clothia | Mean fractional area loss [9] | MFALRose |
| Conformational parameter for beta-sheet [10] | CPFBS29Chou | Conformational preference for total beta strand [11] | CPFTBSLifson |
| Side chain classes [12, p51] | SCClassKnown | Surface accessibility [13] | SASEmini |
| Side chain polarity [12, p51] | SCPolKnown | Binary representation of protein sequence | BPS |

4.2.2 Pre-training

See section 2.1.

4.2.3 Cross Validation (CV) results

We used- 80%of the data on 5-fold cross-validation to assess the performance of the model. See Figure 12 for the Spearman's correlation coefficient metric. The models performed well, without overfitting and had high performance for all the evaluation metrics. Moreover, the model demonstrated high stability, with only a small variation observed between different CV runs.

Figure 12 shows the results for RSA/HSE model. Figures 12(a)-(c) show the Spearman's correlation coefficients, and 12(d)-(f) the Mean Square Error (MSE) losses. The x-axis is the epochs, the y-axis is the metric values. The plots are the average of 5-fold CVs including Gaussian Cl. The blue line is for training and the red (dashed) for validation sets.

4.2.4 Test results

We used the remaining ~ 20% of the data as test data to assess the performance of the model on completely unseen data. See Figure 13 for the Spearman's correlation coefficient metric. The models performed well, without overfitting and had high performance for all evaluation metrics.

Figure 13 shows the results for RSA/HSE model. (a)-(c): Spearman's correlation coefficients. (d)-(f): MSE losses. The x-axis is the epochs, the y-axis is the metric values. The solid line is for the training and the dotted for the validation set.

4.2.5 Final Training

The final training was done on the entire dataset. Using the optimal number of epochs determined by the cross-validation procedure. See Figure 14 for the Spearman's correlation coefficient metric. The models seem to perform well for all metrics. The model for trained for 70 epochs, chosen based on the CV and test-set analysis.

Figure 14 shows the results for RSA/HSE model. (a)-(c): Spearman's correlation coefficients (d)-(f): MSE losses. The x-axis is the epochs, the y-axis is the metric values.

4.3 Prediction model for SS (struct 3d ss m3 model)

We chose to create a BLSTM model for predicting a three-tier class output for SS. The model uses the categorical cross-entropy loss to assign one of the following classes to each amino-acid in its input sequence; Helices, Strands and Coils. The architecture of the model is substantially the same as that illustrated in igure 11, with a single output node in the output layer. Note: the input features were computed per amino-acid as they are not averaged over sliding windows.

4.3.1 Preliminary Analysis and Features Used

See procedure in section 3.1. The 8 features that we chose to use in the model (including the protein-sequence in binary representation) can be seen in Table 2.

Table 2: Features used in the struct 3d ss m3 model.

| Feature | Abbreviation | Feature | Abbreviation |
|---|---|---|---|
| Conformational parameter for coil [14] | CPFCDeleage | Average surrounding hydrophobicity [15] | ASHPLCManavaian |
| Conformational preference for total beta strand (antiparallel+paral lel) [11] | CPFTBSAPLifson | Normalized frequency for beta-sheet [16] | NFFBSLevitt |
| Conformational parameter for alpha helix [14] | CPFAHDeleage | Side chain classes [12, p.51] | SCClassKnown |
| Normalized frequency for beta-turn [16] | NFFBTLevitt | Binary representation of protein sequence | BPS |

4.3.2 CV results

We used ~ 80%of the data on 5-fold cross-validation to assess the performance of the model. See Figure 15 for the precision recall metric. The model seems to perform well, without significant overfit and gives hight performance for all metrics. Moreover, small variation is observed between different CV runs for the loss. Finally, for demonstration purposes, the models were trained for 70 epochs with slight overfit after 60.

Figure 15 illustrates the results for SS model. Figures 15(a)-(e) show PR curves. The x-axis is the TPR, the y-axis is the PPV. Figure 15(f) shows categorical cross-entropy loss. The x-axis is the epochs, the y-axis is the loss values. The plot is the average of 5-fold CVs including Gaussian CI. The blue line is for training and the red (dashed) for validation sets.

4.3.3 Test results

We used the rest ~ 20% of the data as test data to assess the performance on completely unseen data. See Figure 16 for the precision-recall metric. The model performed well, without significant overfitting and had high performance for all evaluation metrics. The model was trained for 100 epochs, chosen based on the CV analysis, so slightly more epochs were required than the CV analysis.

Figure 16 shows the results for SS model. Figure 16(a): PR curves. The x-axis is the TPR, the y-axis is the PPV. Figure 16(b): Categorical cross-entropy loss. The x-axis is the epochs, the y-axis is the loss values. The solid line is for the training and the dotted for the validation set.

4.3.4 Final Training

The final training was done on the whole data. Using the optimal number of epochs determined by the cross-validation procedure. See Figure 17 for the F1 metric. The final trained model performed well, without significant overfitting and gave high performance for all metrics. The model for trained for 100 epochs, chosen based on the CV analysis, so slightly more epochs than the CV and test-set analysis.

Figure 17 shows results for SS model. Figure 17(a)-(c): F1 metrics. The x-axis is the epochs, the y-axis is the F1 value. Figure 17(d): Categorical cross-entropy loss. The x-axis is the epochs, the y-axis is the loss values.

5 Predicting 3D features ("Second Machine Learning Algorithm") (Version 2)

5.1 Data preparation for the 3D features (Version 2)

Same as Section 4.1. The only difference is that for the SS class there was a misplacement of one of the classes. Namely, the isolated β-bridge class was given to the Coil super-class, while here it is moved correctly to the Strand super-class. This small detail only affects some parts of the SS models data.

5.2 Prediction model for RSA and HSE (Version 2) (rsa hse m4 v1 model)

Same as section 4.2, only the preliminary analysis has changed and the input features. The model is deeper as well. The architecture of the model is substantially the same as that illustrated in Figure 11. Note: the input features were computed per amino-acid as they are, not averaged over sliding windows.

The global model loss was the weighted sum of the individual ones as in the previous model. However, the weights this time were: 1, 10 and 125 for RSA, UHSE and LHSE, respectively. The weights were decided by first training the model without them, and see the differences in the magnitude of the three losses.

5.2.1 Preliminary Analysis and Features Used

See section 3.2 for the procedure. The 19 features that we chose to use in the model (including the protein-sequence in binary representation) can be seen in Table 3.

Table 3: Features used in the rsa hse m4 v1 model.

| Feature | Abbreviation | Feature | Abbreviation |
|---|---|---|---|
| Recognition factors [17] | RECFFraga | Antigenicity value X 10 [18] | AVX10Weilling |
| Hydrophobicity [19] | HPBCBull | Retention coefficient in HFBA [20] | RCHFBABrown e |
| Hydration potential [21] | HPWolfenden | Retention coefficient in HPLC, pH 7.4 [22] | RCHPLCPH74 Meek |
| Average area buried on transfer from standard state to folded protein [9] | AABOTRose | Mean fractional area loss [9] | MFALRose |
| Conformational parameter for beta-turn [10] | CPFBT29Cho u | Conformational parameter for alpha helix [10] | CPFAH29Chou |
| Average flexibility index [23] | AFIBhaskara n | Side chain polarity [12, p51] | SCPolKnown |
| Normalized frequency for alpha helix [16] | NFFAHLevi*tt* | Side chain classes [12, p51] | SCClassKnown |
| Relative mutability of amino-acids [24] | RMOAADayh *off* | Isoelectric points [25] | PLKnown |
| Known Number of codon(s) coding for each amino-acid in universal genetic code [12] | NCFAAKnown | PK2 a-+NH3 [25] | PK2Known |
| Binary representation of protein sequence | BPS | | |

5.2.2 Pre-traininq

See section 2.2.

5.2.3 CV results

We used ~ 80% of the data on 5-fold cross-validation to assess the performance of the model.

5.2.5 Test results

We used the rest ~ 20% of the data as test data to see the performance on completely unseen data.

5.2.6 Final Training

The final training was done on the whole data. Using the optimal number of epochs determined by the cross-validation procedure.

5.3 Prediction model for SS (Version 2) (ss3 m6 v1 model)

Similar to the results described section 4.3. The architecture of the model is substantially the same as that illustrated in Figure 11. Note: the input features were computed per amino-acid as they are, not averaged by windows.

5.3.1 Outliers

Same as section 4.3.

5.3.2 Preliminary Analysis and Features Used

See section 3.2 for the procedure. The 19 features that we chose to use in the model (including the protein-sequence in binary representation) can be seen in table 4.

Table 4: Features used in the ss3 m6 v1 model.

| **Feature** \| | **Abbreviation** | **Feature** \| | **Abbreviation** |
|---|---|---|---|
| Molecular volume [2] | MOLVGrantham | Recognition factors [17] | RECFFraga |
| Hydrophobicity [19] | HPBCBull | Antigenicity value X 10 [18] | AVX10Weilling |
| Hydrophobic constants [26] | HPLCWilson | Molar fraction of accessible residues [3] | MOLFAR3220Janin |
| Retention coefficient in TFA [20] | RCTFABrowne | Conformational parameter for coil [14] | CPFCDeleage |
| Proportion of residues buried [8] | PORB12Clothia | Side chain classes [12, p51] | SCClassKnown |
| Conformational parameter for beta-turn [14] | CPFBTDeleage | Antigenicity [27] | AGCKolaskar |
| Relative mutability of amino-acids [24] | RMOAADayhoff | Side chain polarity [12, p51] | SCPolKnown |
| Overall amino-acid composition [28] | OAACMccaldon | Isoelectric points [25] | PLKnown |
| PK2 a-+NH3 [25] | PK2Known | PK1 a-COOH [25] | PK1Known |
| Binary representation of protein sequence | BPS | | |

5.3.3 Pre-training

See section 2.2.

5.3.4 CV results

We used ~ 80% of the data on 5-fold cross-validation to assess the performance of the model.

5.3.5 Test results

We used the rest ~ 20% of the data as test data to see the performance on completely unseen data.

5.3.6 Final Training

The final training was done on the whole data. Using the optimal number of epochs determined by the cross-validation procedure.

6 First Machine Learning Model (CBCEs)

For predicting CBCEs we used BLSTMs based models. This allowed us to model the whole protein sequences bidirectionally as one observation, without the need of segmenting it. Therefore, distant, and contextual amino-acid relationships are captured by the model. Moreover, the use of BLSTMs allowed us to train different protein lengths simultaneously. The main goal of our models was to create non-Ab specific CBCE predictors. This was achieved by the manner we prepared the training data described in section 6.1.

6.1 Data preparation

For modelling CBCEs we downloaded non-obsolete protein complexes from the PDB. We allowed structures of any resolution and organisms, as long as they had at least three different protein chains. The reason for this is that two of the chains may be the $V_H$ and $V_L$ chains of an Ab, and the third chain may be an Ag. Of course, there may be multiple Abs orAgs in one PDB structure.

We created a local database using all immunoglobulin V-, D- and J-region genes from the IMGT (The international ImMunoGeneTics information system) database. Those genes were downloaded for both $V_H$ and $V_L$ chains, from all the available organisms, that is; human, mouse, rhesus monkey, rabbit and rat. The IMGT provides information about the $V_H$ and $V_L$ chains of an Ab, and its paratope regions (the CDRs [Complementary determining region of the antibody] and FRs [framework region of antibody] of each chain).

To identify the Abs in this local database we used IgBlast *(*Jian Ye, Ning Ma, Thomas L Madden, and James M Ostell. Igblast: an immunoglobulin variable domain sequence analysis tool. Nucleic Acids Res, 41(Web Server issue):W34-40, Jul 2013*)* for protein sequences on the IMGT database. For identifying the paratope in a chain we used the Kabat system that the IgBlast provides (G Johnson and T T Wu. Kabat database and its applications: 30 years after the first variability plot. Nucleic acids research, 28(1):214-218, 01 2000*)*. We considered $V_H$ and $V_L$ chains as valid, only if at least their CDR1 and CDR2 were found by IgBlast. Since CDR3 is more difficult to map, we allowed it to be missing in the search. We then aligned all the protein chains of each structure to that database. Structures were filtered out if they did not have at least one chain mapped as a valid $V_H$ and one as a valid $V_L$. Any other chain that was not mapped as $V_H$ or $V_L$ was assumed to be an Ag chain. We considered as valid Ag chains, those chains that were longer than 100 amino-acids and were not bound by any other chain other than a $V_H$ or $V_L$ chain. Finally, only structures including at least one $V_H$, $V_L$ and Ag chains were taken to further analyses.

We then paired $V_H$ and $V_L$ chains in each structure to recreate valid Abs. In case there were multiple $V_H$ and $V_L$ chains in one single structure, we measured the mean distance from every atom on each $V_H$ to every atom on each $V_L$ chain. $V_H$ and $V_L$ chains with the minimum mean atom distance were assigned as pairs and assumed to belong to one single Ab. Stand-alone $V_H$ or $V_L$ chains were not considered on the downstream analysis as they could not define a complete Ab. A paratope analysis provided information about the contacts formed with each Ag. Two amino-acids were assumed to be in contact if any of their atoms were located within a certain probe distance from each other. We computed the total number of Ag amino-acids that form contacts with each paratope's parts, using probe distances of 4, 6 and 8 Angstroms. Figure 18(a) shows the normalised mean of those contacts (x-axis shows the amino acids, y-axis shows the paratope parts). Most of the contacts are made with CDR1 and CDR2 of the $V_H$s and CDR1 and CDR3 of the $V_L$s. The absence of CDR3 on $V_H$s may be due to the difficulty of identifying it using IgBlast. Moreover, the FR regions do not seem to form as many contacts as the CDR regions, as expected. Additionally, the standard deviation of the total contacts per amino-acid and paratope part is relatively low in Figure 18(b), indicating that similar number of contacts are made between different Ags and Abs. This could be an indication of the possibility of predicting CBCEs without any further information about the actual Abs. Figure 18(b) shows the STD for number of contacts. X-axis shows the amino-acids, y-axis shows the paratope parts.

For each $V_H$ and $V_L$ chain pair we defined a single CBCE. This was done by first identifying atoms on any Ag whose distance from the CDRs regions of any $V_H$ and $V_L$ chain pair was $\leq 4$ Angstroms. The amino-acids that those atoms belonged to were defined as contacts between the Ag and the Ab, that is, they defined the CBCE. Multiple CBCEs from different Abs could be defined on the same Ag.

Finally, structures that did not define any CBCE within the 4 Angstrom distance were discarded.

Observed CBCEs were also mapped to similar Ag. Undiscovered CBCEs could increase the false negative rate of the prediction models. To decrease the possibility of assigning undiscovered CBCEs as negative data we copied observed CBCEs to similar Ags. First we identified clusters of similar Ags using BlastPlus with > 90% similarity and at most 2 gaps. CBCEs were copied from an Ag in a cluster to all the other Ags in the same cluster, as long as their corresponding position and distancing was exactly the same based on the mapping from BlastPlus.

Lastly, we created a unique Ag local database. Duplicated Ags were completely removed from the data. On the other hand, Ag sequences that were sub-sequences of longer Ags were kept in the data and treated as different observations. The resulted database consisted of 1003 Ag sequences in FASTA format and 12968 CBCEs, of which 6986 were unique. Each Ag sequence was associated with at least one CBCE.

## 6.2 CBCE models summary

We have created two main CBCE predictors, namely model 13 conf v1 and model 17 conf v1. There are two differences between the models. Firstly, the negative data generator is different, see section 6.3 for details. Secondly, the model 13 conf v1 model gives two outputs, while model 17 conf v1 gives one. Both models take as input features, among others, a permutation vector. Every sequence in the data is associated with at least one true CBCE, those CBCEs are turned into binary 2D vectors and are given as input to the models. The common output of the two models is a probability, so both models are basically asking the question: "Is this permutation vector, on this specific sequence, a true CBCE or not?". The second output of the model 13 conf v1 model is the permutation vector itself. This part of the model works as an AE, where it returns a probability per amino-acid. This output can be seen as a contribution of each amino-acid in the sequence to the specific CBCE in question.

The goal of those models was to predict CBCEs on the protein Ag sequence, before the Ab binding event took place. The dataset used, described in section 6.1, contains the linear protein sequence and the true CBCEs. Therefore, there is no information about any structural or surface characteristic of those protein sequences. However, we need information about how each protein sequence's secondary structure and surface would look like before the binding event. Therefore, we used our prediction models for RSA, UHSE and LHSE (model struct 3d rsa hse m2 res) and SS (model struct 3d ss m3 res1) described in section 4 to predict those characteristics in every protein in the dataset.

There are three main differences of our models compared to the currently existing publicly available CBCE algorithms. Firstly, we use BLSTMs which allows us to capture relationships between distant and contextual amino-acids which may constitute a CBCE on a native folded 3D protein structure. On the other hand, the other algorithms are segmenting the protein sequences in k-mers and thus, not capturing these important relationships. Secondly, the training dataset is created in such a way to predict true CBCEs before the binding event takes place. This difference brings the model conceptually closer to modelling the reality of Ab binding. As the 3D folded protein structure is exactly what an Ab sees and triggers the binding, and not what an Ab has already seen and bound on.

Thirdly, the currently existing publicly available CBCE algorithms require an experimentally measured, or potentially predicted, 3D protein Ag structure in order to predict epitopes. Our first machine learning model does not require the 3D coordinates of every atom in each amino acid on the Ag or Ab. Finally, our model addresses the epitope question in a conceptually very different manner, but in a manner that is more fined tuned to capture bone fide ("true") BCEs. We predict probabilities of stand-alone CBCEs on each Ag as a pair or independent class, and therefore multiple CBCEs on the same protein sequence can be discovered and assessed independently. On the other hand, other algorithms provide probabilities per amino-acid, without the ability of separation between different CBCEs.

Figure 19(a) schematically shows the architecture of the model 13 conf v1 model. The architecture of the model is substantially the same as that shown in Figure 11. Here, the output layer comprises two output nodes. The BCE amino acid output (BCE_aa) is the actual permutation output, where binary cross-entropy loss was used. The BCE perm output is a probability vector indicating if the input permutation is a true CBCE (second position on vector) or not (first position on vector). Figure 19(b) shows the architecture of the model 17 conf v1 model. The BCE perm represents the same output as the previous model. Note: the input features were computed per amino-acid as they are, not averaged by windows.

For both models, the BCE perm output is computed from amino-acid values. The last layer of the output is a Dense layer with sigmoid activation. Such that, for each protein sequence, a value in $\in$ [0, 1] is returned per amino-acid. Then a 2-class probability vector is computed for that sequence as:

$$[1 - \sum_{i=0}^{N} x_i/N, \sum_{i=0}^{N} x_i/N],$$

where N is the length of the sequence and xi is the dense layer output on amino-acid at position i on the sequence. This 2-class probability vector is then used in the binary cross-entropy loss.

The model 13 conf v1 model had weighted global loss. The global model loss was the weighted sum of the individual ones, that is BCE perm and BCE aa, with weights: 1, and 1, respectively. Different weights could be tested as well.

### 6.3 Negative Data

If we were to model the CBCEs as per amino-acid, then the positive data would be every amino-acid in a sequence which is a part of any true CBCE, and the negative data would be any other amino-acid on the same sequence. However, since we model the whole CBCE as a single permutation vector of a given protein sequence, the response to this vector is a single probability (that is output BCE perm from section 6.2). Since we only have observed true positive CBCEs from the data in section 6.1, we do not have any permutation corresponding to negative data.

We developed a solution to improve the performance of the models, whereby negative permutation data was generated in a manner which resample the ground truth as close as possible. To cover a large spectrum of negative data and make the models more robust, we generated negative permutations for every sequence in the data, on every epoch during training. The two different generation schemes are described in the following sections.

#### 6.3.1 CBCE model (model 13 conf v1)

For this model we generated completely random permutations. For each sequence in the training and validation data we generated 10 completely random permutations and assumed that they are not true CBCEs. Both the total number of amino-acids and the placement of those in the given sequence were random. New permutations were generated on each epoch.

The advantage of this method is that because of the complete randomness, it is quite unlikely that any generated CBCE permutation will be false negative. However, the disadvantage is that the randomly generated CBCE permutations may be extremely different than the true CBCE permutations.

6.3.2 CBCE model (model 17 conf v1)

For this model we applied three different types of negative data generating methods. For each sequence in the training and validation data we generated 30 completely random permutations, 10 from each method, and assumed that they are not true CBCEs. The first method is like the one described in 6.3.1. The second and third methods were applied on every true CBCE of the given protein sequence. The second method kept the first and last amino-acid of a given true CBCE at the correct position, while it randomly shuffled the internal CBCE amino-acids indicators inside the region. The third method kept the total amount of amino-acids of a true CBCE, as well their linear distance, constant. It then randomly shifted the whole true CBCE on other parts of the protein. New permutations were generated on each epoch from every method.

The advantage of this method is that the randomly generated CBCE permutations cover both extremely similar and extremely different true CBCE permutations. This will probably lead to that the algorithm is more robust to both positive and negative data. On the other side, the disadvantage of the method is that the false negatives may increase . Leading to lower performance on new data.

6.4 Outliers

We removed outliers before any training of either model. We did this by applying Isolation forests (IF) on the total amino-acids of each observed CBCE in our data from section 5.1. We run the algorithm for every observed amount of total amino-acids as the max samples argument in the IF algorithm from the sklearn Python package *(*F. Pedregosa, G. Varoquaux, A. Gramfort, V. Michel, B. Thirion, O. Grisel, M. Blondel, P. Prettenhofer, R. Weiss, V. Dubourg, J. Vanderplas, A. Passos, D. Cournapeau, M. Brucher, M. Perrot, and E. Duchesnay. Scikit-learn: Machine learning in Python. Journal of Machine Learning Research, 12:2825-2830, 2011*.)* The most common minimum and maximum borders from all the runs were taken as global borders for inliers.

Figure 20 shows the total amount of amino-acids from all the observed CBCEs. Figures 20(a) and 20(b) show, respectively, density and box-plot of observed amount of total amino-acids in the true CBCEs. Figures 20(c) and 20(d) shown log-scales of Figures 20(a) and 20(b) respectively. Where we see that the median amount is at 10 amino-acids. After the outlier analysis, we found that valid CBCEs should contain a total amount of amino-acids in range, which is lower than what discussed in section 1.3. However, those studies did not perform any outlier detection or removal of their observed CBCEs. As we can see from Figure 20, the maximum amount of total amino-acids before outlier removal is around 30 to 35, which corresponds with that reported in the previous studies. The outliers were finally removed from the data, and the models were trained without them.

6.5 Preliminary Analysis and Features Used

See section 3.2 for the procedure. The 14 features that we chose to use in the models (including the protein-sequence in binary representation and the permutation vectors) is shown in in Table 5. Note that the RSA and SS were predicted by models struct 3d rsa hse m2 res and struct 3d ss m3 res1, respectively, described in section 4. The same features were used for both model model 13 conf v1 and model 17 conf v1.

Table 5: Features used in the model 13 conf v1 and model 17 conf v1 models.

| **Feature** | **Abbreviation** | **Feature** | **Abbreviation** |
|---|---|---|---|
| Bulkiness [29] | BulkZimmerman | Polarity [2] | POLGrantham |
| Molar fraction of buried residues [3] | MOLF2001OBRJanin | Conformational parameter for beta-turn [10] | CPFBT29Chou |
| Average flexibility index [23] | AFIBhaskaran | Normalized frequency for beta-sheet [16] | NFFBSLev*itt* |
| Normalized frequency for alpha helix [16] | NFFAHLev*itt* | Side chain classes [12, p51] | SCClassKnown |
| Known Number of codon(s) coding for each amino-acid in universal genetic code [12] | NCFAAKnown | Side chain polarity [12, p51] | SCPolKnown |

(continued)

| Feature | Abbreviation | Feature | Abbreviation |
|---|---|---|---|
| Binary representation of protein sequence | BPS | 3-Class Secondary Structure | SS3 |
| Relative Solvent Accessibility | RSA | Permutation Vectors | PERM |

6.6 Pre-training

See section 2.2.

6.7 CV results

We defined as "age" of the protein Ag in our data the date at which the most recent CBCE was registered to the protein sequence. We used ~ 94% of the "oldest" proteins in the data on a 5-fold cross-validation in order to assess the performance of the model. We used custom callbacks in Tensorflow to allow for early stopping in case the loss did not decrease anymore.

6.7.1 Model model 13 conf v1

Figure 21 shows results for the precision-recall metric. The model performed well, without significant overfitting and had high performance for all evaluation metrics. Moreover, a small variation is observed between different CV runs for the loss. The models were trained for around 466 epochs and apparent bumps in the loss may indicate that the random generator create negative CBCE permutations which were similar to the positives. However, their negative effect became smaller as the epochs proceeded further.

Figure 21 illustrates results for CBCE model 13 conf v1. Figure 21 (a)-(e): PR curves. The x-axis is the TPR, the y-axis is the PPV. Figure 21(f): Weighted cross entropy loss. The x-axis is the epochs, the y-axis is the loss values. The plot is the average of 5-fold CVs including Gaussian Cl. The blue line is for training and the red (dashed) for validation sets.

6.7.2 Model model 17 conf v1

Figure 22 shows results for the precision-recall metric. The model seems to perform well, without significant overfit before 250 epochs and gives high performance for all metrics. Moreover, small variation is observed between different CV runs for the loss. The models were trained for 455 epochs.

Figure 22 illustrates results for CBCE model 17 conf v1. Figure 22 (a)-(e): PR curves. The x-axis is the TPR, the y-axis is the PPV. Figure 22(f): Weighted cross entropy loss. The x-axis is the epochs, the y-axis is the loss values. The plot is the average of 5-fold CVs including Gaussian Cl. The blue line is for training and the red (dashed) for validation sets.

6.8 Test results

We used ~ 6% of the "newest" proteins in the data in order to see the performance on completely unseen data. The reason for keeping the newest data as test set was because the same data was used for comparison with other algorithms. This way we ensure that those algorithms were not trained on that data. The training on the following models was done on the "oldest" ~ 94% of the data.

6.8.1 Model model 13 conf v1

Figure 23 shows the results for the precision-recall metric. The model performed well, without significant overfitting and gave high performance for all evaluation metrics. The model for trained for 466 epochs, chosen based on the CV analysis, so slightly more epochs than the CV analysis.

Figure 23 illustrates the results for CBCE model 13 conf v1. Figure 23(a): PR curves. The x-axis is the TPR, the y-axis is the PPV. Figure 23(b): Overall model loss. The x-axis is the epochs, the y-axis is the loss values. The solid line is for the training and the dotted for the validation set.

6.8.2 Model model 17 conf v1

Figure 24 shows the results for the precision-recall metric. The model seems to perform well, without significant overfit and gives high performance for all metrics. The model for trained for 455 epochs, chosen based on the CV analysis, so slightly more epochs than the CV analysis.

Figure 24 illustrates the results for CBCE model 17 conf v1. Figure 24(a): PR curve. The x-axis is the TPR, the y-axis is the PPV. Figure 24(b): Overall model loss. The x-axis is the epochs, the y-axis is the loss values. The solid line is for the training and the dotted for the validation set.

6.9 Final Training

The final training was done on the whole data. Using the optimal number of epochs determined by the cross-validation procedure.

6.9.1 Model model 13 conf v1

Figure 25 shows the results for the F1 metric. The model seems to perform well, without significant overfit and gives hight performance for all metrics. The model for trained for 500 epochs, chosen based on the CV analysis.

Figure 25 shows the results for CBCE model 13 conf v1. Figure 25(a)-(b): F1 metrics. The x-axis is the epochs, the y-axis is the F1 value. Figure 25(c): Overall model loss. The x-axis is the epochs, the y-axis is the loss values.

6.9.2 Model model 17 conf v1

Figure 26 shows the results for the F1 metric. The model seems to perform well, without significant overfit and gives high performance for all metrics. The model for trained for 700 epochs, chosen based on the CV analysis.

Figure 26 shows the results for CBCE model 17 conf v1. Figure 26(a): F1 metric. The x-axis is the epochs, the y-axis is the F1 value. Figure 26(b): Overall model loss. The x-axis is the epochs, the y-axis is the loss values.

7 First Machine Learning Model (LBCEs)

For predicting LBCEs we use BLSTMs models as well. This allows us to model the whole protein sequences as one observation, without the need of segmenting it. Therefore, distant amino-acid relationships should be able to be captured by the model. Moreover, the use of BLSTMs allows us to train different protein lengths simultaneously. Therefore, the model type here is the same as the one for the CBCEs in section 6. The main goal of our models was to create non-Ab specific LBCE predictors. This is achieved by the way we prepare the training data as described in section 7.1.

7.1 Data preparation

For modelling LBCEs we downloaded LBCE data from IEDB We downloaded all non-obsolete assays from the IEDB, from all organisms.

Since the main goal of our models was to create non-Ab specific LBCE predictors, we considered all positive LBCEs for our database. That is, if a LBCE was tested positive against at least one Ab, we included it in the database. Each Ag ID was then mapped to the universal protein resource (UniProt), in order to retrieve the protein sequence in fasta format. Assays were discarded if their IDs were non-mappable, or if their IDs were mappable but the coordinates of the corresponding LBCE did not give the same sequence on the retrieved Ag sequence.

Observed LBCEs were also mapped to similar Ag, as we did in section 6.1 for the CBCEs. Undiscovered LBCEs could increase the false negative rate of the prediction models. In an attempt to decrease the possibility of assigning undiscovered LBCEs as negative data we copied observed LBCEs to similar Ags. First we identified clusters of similar Ags using BlastPlus with > 90% similarity and at most 2 gaps. LBCEs were copied from an Ag in a cluster to all the other Ags in the same cluster, as long as their corresponding linear coordinates were exactly the same based on the mapping from BlastPlus.

Lastly, we created a unique Ags database. Duplicated Ags were completely removed the data. On the other hand, Ags sequences that were sub-sequences of longer Ags were kept in the data and treated as different observations. The resulted database constituted of 4695 Ag sequences in fasta format and 177782 CBCEs from which 63363 were unique. Each Ag sequence was associated with at least one LBCE.

7.2 LBCE model summary

Every sequence in the data is associated with at least one true LBCE. The model takes as input features, among others, a permutation vector. This vector is the LBCEs which are turned into binary 2D vectors and are given as input to the model. The output of the model is a probability, so the model is basically asking the question; "Is this permutation vector, on this specific sequence, a true LBCE or not?". Basically, the permutation vectors are the same as described in section 6.2, where the only difference is that they represent LBCEs and not CBCEs.

The goal of those models was to predict LBCEs before the binding event took place. The dataset used, described in section 7.1, contains the linear protein sequence and the true LBCEs. Therefore, there is no information about any structural or surface characteristic of those protein sequences. However, we need information about how each protein sequence's surface would look like before the binding event. Therefore, we used our prediction model for RSA (model struct 3d rsa hse m2 res), described in section 4, in order to predict those characteristics in every protein in the dataset.

There are three main differences of our models with the publicly available LBCE algorithms. Firstly, we use BLSTMs which allows us to capture relationships between distant amino-acids which may constitute a LBCE. On the other hand, the other algorithms are segmenting the protein sequences and thus, breaking such bonds. Secondly, the training dataset is created in such a way in order to predict true LBCEs before the binding event takes place. Which is exactly what an Ab sees and triggers the binding, and not what an Ab has already seen and bound on. Finally, we predict probabilities of stand-alone LBCEs, and therefore multiple LBCEs on the same protein sequence can be discovered and separated. On the other hand, other algorithms provide probabilities per amino-acid, without the ability of separation between different LBCEs.

The architecture of the model 12 linear v1 model has substantially the same structure as the model depicted in Figure 19(b). The bce output is a probability vector indicating if the input permutation is a true LBCE (second position on vector) or not (first position on vector). The bce output is computed from amino-acid values as described in section 6.2. Note: the input features were computed per amino-acid as they are, not averaged by windows.

7.3 Negative Data

We followed a similar procedure as in section 6.3.1. The only difference here is that we ensured that the randomly generated LBCEs were unbroken. Let N be the length of a given protein sequence. We first generate the total amount X of amino-acids that a random LBCE will have from the uniform distribution in [0,N]. Then we generated the starting position i of this LBCE from the uniform distribution in [0,N - X]. Finally, we assigned all amino-acids in [i, i + X] as the ones corresponding this random LBCE. A total amount of 10 randomly generated LBCEs was generated pre sequence, for every epoch.

7.4 Outliers

We removed outliers before any training of the model 12 linear v1 model. We followed exactly the same procedure as in section 6.4, and the data we used was the amount of total amino-acids of each observed LBCE in our data from section 7.1.

Figure 27 shows the total amount of amino-acids from all the observed LBCEs. Where we see that the median amount is at 17 amino-acids. After the outlier analysis, we found that valid LBCEs should contain a total amount of amino-acids in range [5, 30]. The outliers were finally removed from the data, and the models were trained without them.

Figure 27 shows outlier analysis for LBCEs. Figures 27(a) and (b) show, respectively, density and box-plot of observed amount of total amino-acids in the true LBCEs. Figures 27(c) and (d) show, log-scales of Figures 27(a) and 27(b) respectively.

7.5 Preliminary Analysis and Features Used

See section 3.2 for the procedure. The 13 features that we chose to use in the model (including the protein-sequence in binary representation and the permutation vectors) can be seen in Table 6. Note that the RSA was predicted by the model struct 3d rsa hse m2 res described in Section 4.

Table 6: Features used in the model 12 linear v1 model.

| Feature \| | Abbreviation | Feature | Abbreviation |
|---|---|---|---|
| Retention coefficient in HPLC, pH 7.4 [22] | RCHPLCPH74Me ek | Membrane buried helix parameter [5] | MBHPRao |
| Hydrophobic constants [26] | HPLCWilson | Normalized frequency for beta-sheet [16] | NFFBSLevitt |
| Side chain classes [12, p51] | SCClassKnown | Antigenicity [27] | AGCKolaskar |
| Normalized frequency for beta-turn [16] | NFFBTLevitt | Side chain polarity [12, p51] | SCPolKnown |

(continued)

| Feature \| | Abbreviation | Feature | Abbreviation |
|---|---|---|---|
| Binary representation of protein sequence | BPS | 3-Class Secondary Structure | SS3 |
| Relative Solvent Accessibility | RSA | Permutation Vectors | PERM |
| PK1 a-COOH [25] | PK1Known | | |

## 8 Applications

The precise prediction of B-cell epitopes (in particular 3D conformational B-cell epitopes) leads to major improvements in diagnostics, drug design and vaccine development in the fields of autoimmunity, infectious-disease, and cancer. Examples are provided below.

### 8.1 Therapeutic antibody mapping

The binding event of an antibody to an antigen is a key event in activating the humoral B Cell immune response against any pathogenic threat, such as viral and bacterial infections, in addition to cancer antigens in malignant tumour cells. B Cell epitope mapping is essential in therapeutic antibody development. The prediction of true B-cell epitopes by the present invention not only provides a functional understanding of the critical residues involved antibody-antigen binding; it also aids in the selection of therapeutically relevant antibodies to the prioritized predicted epitopes for further development as therapeutic antibodies.

### 8.2 Vaccine development

In antibody driven vaccine design, *in silico* predictions are performed to predict BCEs that could trigger humoral immune responses. Previous *in silico* guided vaccine-design initiatives tend to selectively apply BCE prediction with a focus on surface-exposed sites among the antigens. However, by using the approach of the present invention (in particular training on unbound protein structures and the use of candidate BCE encodings ("permutation vectors") mapped to the antigen protein sequence), we can identify with precision, BCEs to guide Ab based vaccine design with far higher fidelity. BCE vaccines guided by the approach of the present invention can advantageously identify at scale specific antibody epitope interactions capable of avoiding undesirable immune responses, in addition to potentially generating long lasting, potent and universal immunity. Moreover, the method described here bypasses the need for years of target discovery and laborious expensive time-consuming work at a fraction of the cost and time.

### 8.3 Immuno-diagnostics and immune monitoring

Prediction of B cell epitopes is of direct help to the design of immuno-diagnostic and immune monitoring of therapy strategies and reagents. Integrating prediction approaches for the accurate and comprehensive detection of BCEs can greatly facilitate diagnosis. One example of this would be the rapid and accurate detection of BCE epitopes on the spike protein of beta-coronaviruses, allowing for the diagnosis of COVID in a manner that can distinguish infections caused by common cold viruses from the SARS-CoV-2 virus causing COVID. Along the same vein, the BCE prediction technology can be used to identify immunodominant or humoral reactive epitopes in tumour associated antigens or neoantigens in immuno-diagnostic and immune monitoring clinical applications.

### 8.4 Example Systems

Figure 4 schematically illustrates an example of a system suitable for implementing embodiments of the method. The system 1100 comprises at least one server 1110 which is in communication with a reference data store 1120. The server may also be in communication with an automated peptide synthesis device 1130, for example over a communications network 1140.

In certain embodiments the server may obtain, for example using from the reference data store, an amino acid sequence of one or more query proteins. The server may then predict whether the query protein comprises a BCE that is likely to instigate a binding event with an antibody (e.g. instigate an immunogenic response).

Query proteins predicted to contain one or more BCEs may be sent to the automated peptide synthesis device 1130 to synthesise the query protein or parts thereof. Techniques for automated peptide synthesis are well known in the art and it will be understood that any known technique may be used. Typically, the query protein or epitope is synthesized using standard solid phase synthetic peptide chemistry and purified using reverse-phase high performance liquid chromatography before being formulated into an aqueous solution. If used for vaccination, prior to administration the peptide solution is usually admixed with an adjuvant before being administered to the patient.

Peptide synthesis technology has existed for more than 20 years but has undergone rapid improvements in recent years to the point where synthesis now takes just a few minutes on commercial machines. For brevity we do not describe in detail such machines but their operation would be understood to one skilled in the art and such conventional machines may be adapted to receive a candidate region or epitope from the server.

The server may comprise the functions described above to identify query proteins predicted to contain one or more viable BCEs. It will of course be understood that these functions may be subdivided across different processing entities of a computer network and different processing modules in communication with one another.

The techniques for identifying BCE-containing proteins may integrate into a wider ecosystem for customised vaccine development. Example vaccine development ecosystems are well known in the art and are described at a high-level for context, but for brevity we do not describe the ecosystem in detail.

In an example ecosystem, a first, sample, step may be to isolate DNA from a tumor biopsy and matched healthy tissue control. In a second, sequence, step, the data is sequenced and the variants identified i.e. the mutations. In an immune profiler step the associated mutated peptides may be generated «in silico».

Using the associated mutated peptides, and the techniques described here, a candidate protein may be predicted and selected and target epitopes identified for vaccine design. That is, the candidate peptide sequence is chosen based on its predicted binding affinity determined using the technique described herein.

The target epitopes are then generated synthetically using conventional techniques as described above. Prior to administration the peptide solution is usually admixed with an adjuvant before being administered to the patient (vaccination). In alternatives, the target epitopes can be engineered into DNA or RNA, or engineered into the genome of a bacteria or virus, as with any conventional vaccine.

The proteins predicted by the methods described herein may also be used to create other types of vaccine other than peptide based vaccines. For example the proteins (or predicted epitopes therein) could be encoded into the corresponding DNA or RNA sequence and used to vaccinate the patient. Note that the DNA is usually inserted in to a plasmid construct. Alternatively, the DNA can be incorporated into the genome of a bacterial or viral delivery system (can be RNA also - depending on the viral delivery system) - which can be used to vaccinate the patient - so the manufactured vaccine in a genetically engineered virus or bacteria which manufactures the targets post immunisation in the patient i.e. in vivo.

An example of a suitable server 1110 is shown in Figure 5. In this example, the server includes at least one microprocessor 1200, a memory 1201, an optional input/output device 1202, such as a keyboard and/or display, and an external interface 1203, interconnected via a bus 1204 as shown. In this example the external interface 1203 can be utilised for connecting the server 1110 to peripheral devices, such as the communications networks 1140, reference data store 1120, other storage devices, or the like. Although a single external interface 1203 is shown, this is for the purpose of example only, and in practice multiple interfaces using various methods (e.g. Ethernet, serial, USB, wireless or the like) may be provided.

In use, the microprocessor 1200 executes instructions in the form of applications software stored in the memory 1201 to allow the required processes to be performed, including communicating with the reference data store 1120 in order to receive and process input data, and/or with a client device to receive sequence data for one or more query proteins, and to generate immunogenic potential predictions according to the methods described above. The applications software may include one or more software modules, and may be executed in a suitable execution environment, such as an operating system environment, or the like.

Accordingly, it will be appreciated that the server 1200 may be formed from any suitable processing system, such as a suitably programmed client device, PC, web server, network server, or the like. In one particular example, the server 1200 is a standard processing system such as an Intel Architecture based processing system, which executes software applications stored on non- volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 1200 is a shown as a single entity, it will be appreciated that the server 1200 can be distributed over a number of geographically separate locations, for example by using processing systems and/or databases 1201 that are provided as part of a cloud based environment. Thus, the above described arrangement is not essential and other suitable configurations could be used.

9. Appendix

[1] T P Hopp and K R Woods. Prediction of protein antigenic determinants from amino acid sequences. Proceedings of the National Academy of Sciences of the United States of America, 78(6):3824-3828, 06 1981.

[2] R. Grantham. Amino acid difference formula to help explain protein evolution. Science, 185(4154):862, 09 1974.

[3] JOEL JANIN. Surface and inside volumes in globular proteins. Nature, 277:491 EP -, 02 1979.

[4] H R Guy. Amino acid side-chain partition energies and distribution of residues in soluble proteins. Biophys J, 47(1):61-70, Jan 1985.

[5] J. K. Mohana Rao and Patrick Argos. A conformational preference parameter to predict helices in integral membrane proteins. Biochimica et Biophysica Acta (BBA) - Protein Structure and Molecular Enzymology, 869(2):197-214, 1986.

[6] Sanzo Miyazawa and Robert L. Jernigan. Estimation of effective interresidue contact energies from protein crystal structures: quasi-chemical approximation. Macromolecules, 18(3):534-552, 03 1985.

[7] Gang Zhao and Erwin London. An amino acid "transmembrane tendency" scale that approaches the theoretical limit to accuracy for prediction of transmembrane helices: relationship to biological hydrophobicity. Protein science : a publication of the Protein Society, 15(8):1987-2001, 08 2006.

[8] Cyrus Chothia. The nature of the accessible and buried surfaces in proteins. Journal of Molecular Biology, 105(1):1-12, 1976.

[9] GD Rose, AR Geselowitz, GJ Lesser, RH Lee, and MH Zehfus. Hydrophobicity of amino acid residues in globular proteins. Science, 229(4716):834, 08 1985.

[10] P Y Chou and G D Fasman. Prediction of the secondary structure of proteins from their amino acid sequence. Adv Enzymol Relat Areas Mol Biol, 47:45-148, 1978.

[11] Shneior Lifson and Christian Sander. Antiparallel and parallel -strands differ in amino acid residue preferences. Nature, 282(5734):109-111, 1979.

[12] M. Cooper and Robert E. Hausman. The cell: a molecular approach, volume 4. Washington, DC ASM Press, 2007.

[13] E A Emini, J V Hughes, D S Perlow, and J Boger. Induction of hepatitis a virus-neutralizing antibody by a virus-specific synthetic peptide. Journal of virology, 55(3):836-839, 09 1985.

[14] G. Deleage and B. Roux. An algorithm for protein secondary structure prediction based on class prediction. Protein Engineering, Design and Selection, 1(4):289-294, 08 1987.

[15] P. MANAVALAN and P. K. PONNUSWAMY. Hydrophobic character of amino acid residues in globular proteins. Nature, 275(5681):673-674, 1978.

[16] Michael Levitt. Conformational preferences of amino acids in globular proteins. Biochemistry, 17(20):4277-4285, 10 1978.

[17] Serafin Fraga. Theoretical prediction of protein antigenic determinants from amino acid sequences. Canadian Journal of Chemistry, 60(20):2606-2610, 2019/03/01 1982.

[18] Gjalt W. Welling, Wicher J. Weijer, Ruurd van der Zee, and Sytske Welling-Wester. Prediction of sequential antigenic regions in proteins. FEBS Letters, 188(2):215-218, 2019/02/28 1985.

[19] Henry B. Bull and Keith Breese. Surface tension of amino acid solutions: A hydrophobicity scale of the amino acid residues. Archives of Biochemistry and Biophysics, 161(2):665-670, 1974.

[20] C. A. Browne, H. P. J. Bennett, and S. Solomon. The isolation of peptides by high-performance liquid chromatography using predicted elution positions. Analytical Biochemistry, 124(1):201-208, 1982.

[21] R. Wolfenden, L. Andersson, P. M. Cullis, and C. C. B. Southgate. Affinities of amino acid side chains for solvent water. Biochemistry, 20(4):849-855, 02 1981.

[22] J L Meek. Prediction of peptide retention times in high-pressure liquid chromatography on the basis of amino acid composition. Proceedings of the National Academy of Sciences of the United States of America, 77(3):1632-1636, 03 1980.

[23] R. BHASKARAN and P. K. PONNUSWAMY. Positional flexibilities of amino acid residues in globular proteins. International Journal of Peptide and Protein Research, 32(4):241-255, 2019/02/28 1988.

[24] Schwartz R.M. Dayhoff, M.O. and B.C. Orcutt. A model of evolutionary change in proteins, volume 5. Atlas of Protein Sequence and Structure. Natl. Biomed. Res. Found., Washington DC, 1978.

[25] David L. (David Lee) Nelson and Michael M. Cox. Lehninger principles of biochemistry. Principles of biochemistry. W.H.Freeman, New York, seventh edition, edition, 2017.

[26] K JWilson, A Honegger, R P Stotzel, and G J Hughes. The behaviour of peptides on reverse-phase supports during high-pressure liquid chromatography. The Biochemical journal, 199(1):31-41, 10 1981.

[27] A. S. Kolaskar and Prasad C. Tongaonkar. A semi-empirical method for prediction of antigenic determinants on protein antigens. FEBS Letters, 276(1-2):172-174, 2018/12/07 December 10, 1990.

[28] Peter McCaldon and Patrick Argos. Oligopeptide biases in protein sequences and their use in predicting protein coding regions in nucleotide sequences. Proteins: Structure, Function, and Bioinformatics, 4(2):99-122, 2019/02/28 1988.

[29] J.M. Zimmerman, Naomi Eliezer, and R. Simha. The characterization of amino acid sequences in proteins by statistical methods. Journal of Theoretical Biology, 21(2):170-201,1968.

[30] Daniel D. Jones. Amino acid properties and side-chain orientation in proteins: A cross correlation approach, volume 50. 04 1975.

[31] D. Eisenberg, E. Schwarz, M. Komaromy, and R.Wall. Analysis of membrane and surface protein sequences with the hydrophobic moment plot. Journal of Molecular Biology, 179(1):125-142, 1984.

[32] J. M. R. Parker, D. Guo, and R. S. Hodges. New hydrophilicity scale derived from high-performance liquid chromatography peptide retention data: correlation of predicted surface residues with antigenicity and x-ray-derived accessible sites. Biochemistry, 25(19):5425-5432, 09 1986.

[33] Shaun D. Black and Diane R. Mould. Development of hydrophobicity parameters to analyze proteins which bear post- or cotranslational modifications. Analytical Biochemistry, 193(1):72-82, 1991.

[34] J L. Fauchere and V Pliska. Hydrophobic parameters II of amino acid side-chains from the partitioning of N-acetyl-amino acid amides, volume 18. 01 1983.

[35] Robert M. Sweet and David Eisenberg. Correlation of sequence hydrophobicities measures similarity in three-dimensional protein structure. Journal of Molecular Biology, 171(4):479-488, 1983.

[36] Jack Kyte and Russell F. Doolittle. A simple method for displaying the hydropathic character of a protein. Journal of Molecular Biology, 157(1):105-132, 1982.

[37] Charles. Tanford. Contribution of hydrophobic interactions to the stability of the globular conformation of proteins. Journal of the American Chemical Society, 84(22):4240-4247, 11 1962.

[38] Donald J. Abraham and Albert J. Leo. Extension of the fragment method to calculate amino acid zwitterion and side chain partition coefficients. Proteins: Structure, Function, and Bioinformatics, 2(2):130-152, 2019/02/28 1987.

[39] Richard Cowan and R GWhittaker. Hydrophobicity indices for amino acid residues as determined by HPLC, volume 3. 01 1990.

[40] Mark A. Roseman. Hydrophilicity of polar amino acid side-chains is markedly reduced by flanking peptide bonds. Journal of Molecular Biology, 200(3):513-522, 1988.

[41] A. A. Aboderin. An empirical hydrophobicity scale for alpha-amino-acids and some of its applications. International Journal of Biochemistry, 2:537-544, 1971.

## Claims

1. A computer-implemented method of predicting whether a protein comprises a B-cell epitope that is likely to instigate a binding event with an antibody, the method comprising:

   (a) accessing the amino acid sequence of the protein;
   (b) accessing one or more structure and/or surface characteristics of the protein;
   (c) generating one or more candidate encodings of respective one or more candidate B-cell epitopes on the protein, wherein each candidate encoding represents the respective candidate B-cell epitope as a plurality of data elements corresponding to the amino acids of the amino acid sequence; and
   (d) using a trained first machine learning model to predict whether any of the candidate B-cell epitope(s) is likely to be a true B-cell epitope on the protein; wherein the input to the trained first machine learning model comprises the amino acid sequence of the protein, the one or more structure and/or surface characteristics of the protein, and the one or more candidate encodings.

2. The method of claim 1, wherein the output of the trained first machine learning model is a probability that each of the candidate encoding(s) represents a true B-cell epitope on the protein.

3. The method of claim 1 or claim 2, wherein the one or more candidate encodings represent all possible combinations of the amino acids within the amino acid sequence.

4. The method of any of the preceding claims, wherein the first machine leaning model comprises a long short-term memory network, LSTM, preferably a bi-directional long short-term memory network, BLSTM.

5. The method of any of the preceding claims, wherein the one or more structure and/or surface characteristics include one or more of: the secondary structure of the protein; the relative solvent accessibility, RSA; the half-sphere exposure, HSA.

**6.** The method of any of the preceding claims, wherein the first machine learning model is trained using a first reference dataset that comprises:

(i) a plurality of amino acid sequences of respective first reference proteins, each first reference protein comprising at least one true B-cell epitope,
(ii) a plurality of reference encodings of each true B-cell epitope, wherein each reference encoding represents the respective true B-cell epitope as a plurality of data elements corresponding to the amino acids of the amino acid sequence; and
(iii) one or more structure and/or surface characteristics of each first reference protein.

**7.** The method of claim 6, wherein the one or more structure and/or surface characteristics of each first reference protein are one or more structure and/or surface characteristics of each first reference protein in an unbound state.

**8.** The method of any of claim 6 or claim 7, wherein the one or more structure and/or surface characteristics of each first reference protein, are predicted by applying one or more trained second machine learning model(s) to the corresponding amino acid sequence, wherein
the one or more trained second machine learning models are trained on a second reference dataset that comprises a plurality of amino acid sequences of respective proteins and their corresponding structure and/or surface characteristics.

**9.** The method of any of the preceding claims, wherein the one or more structure and/or surface characteristics of the protein accessed in step (b) are predicted by applying one or more trained second machine learning model(s) to the corresponding amino acid sequence, wherein
the one or more trained second machine learning models are trained on a second reference dataset that comprises a plurality of amino acid sequences of respective proteins and their corresponding structure and/or surface characteristics.

**10.** The method of claim 8 or claim 9, wherein the one or more second machine learning model(s) are trained on a second reference dataset that comprises a plurality of amino acid sequences of respective second reference proteins and their corresponding structure and/or surface characteristics in an unbound state.

**11.** The method of any of claims 7 to 10, wherein at least some of the first reference proteins are obtained by:

(i) accessing a plurality of protein complexes comprising at least three different protein chains;
(ii) filtering the plurality of protein complexes to leave only those having a protein chain mapped as a valid heavy ($V_H$) chain of an antibody, a protein chain mapped as a valid light ($V_L$) chain of an antibody, and a protein chain mapped as an antigen;
(iii) pairing the $V_H$ and $V_L$ chains to form $V_H$ and $V_L$ chain pairs; and
(iv) for each $V_H$ and $V_L$ chain pair, defining a true B-cell epitope on the corresponding antigen; wherein

at least some of the first reference proteins of the first reference dataset correspond to an antigen mapped in step (ii) that comprises at least one true B-cell epitope defined in step (iv).

**12.** The method of any of claims 7 to 11, further comprising generating pseudo-random data based on the plurality of reference encodings of each true B-cell epitope of the first reference dataset, and wherein the first reference dataset comprises said pseudo-random data as negative data.

**13.** The method of any of the preceding claims, wherein the B-cell epitope is a conformational B-cell epitope.

**14.** The method of any of the preceding claims, wherein the protein comprises an antigen.

**15.** A method of training a machine learning model, comprising:

generating a reference dataset, the reference dataset comprising:

a plurality of amino acid sequences of respective reference proteins, each reference protein comprising at least one true B-cell epitope;
a plurality of reference encodings of the true B-cell epitope(s), wherein each reference encoding represents the respective true B-cell epitope as a plurality of data elements corresponding to the amino acids of the amino acid sequence; and
one or more structure and/or surface characteristics of each reference protein; and

training the machine learning model using the reference dataset to learn a relationship between the candidate encodings, the structure and/or surface characteristic(s), and the corresponding true B-cell epitopes.

**16.** The method of claim 15, wherein the one or more structure and/or surface characteristics of each reference protein are one or more structure and/or surface characteristics of each first reference protein in an unbound state.

**17.** A method of creating a vaccine, comprising:

predicting that a protein comprises a B-cell epitope by a method according to any of claims 1 to 14; and
synthesising the protein and/or the predicted B-cell epitope, or encoding at least one of the protein, the predicted B-cell epitope, B-cell epitope variant, B-cell epitope predicted or simulated variant, into a corresponding protein, peptide, DNA or RNA sequence.

**18.** A method of creating a diagnostic assay to determine whether a patient has or has had a cancer or prior infection with a pathogen, wherein the diagnostic assay is carried out on a biological sample obtained from a subject, comprising identifying at least one protein of the pathogen or tumor that is predicted to comprise a B-cell epitope, using a method according to any of claims 1 to 14; wherein
the diagnostic assay comprises the utilisation or identification within the biological sample of the identified at least one protein and/or B-cell epitope.

**19.** A system for predicting whether a protein comprises a B-cell epitope that is likely to instigate a binding event with an antibody, comprising at least one processor in communication with at least one memory device, the at least one memory device having stored thereon instructions for causing the at least one processor to perform a method according to any of claims 1 to 14.

**20.** A computer program product comprising instructions which, when executed by a computer, cause the computer to perform the method of any of claims 1 to 19.

**21.** Use of a machine learning model trained using the method of claim 15 or claim 16.

**22.** A machine learning model trained using the method of claim 15 or claim 16.

**23.** A method of synthesising a protein, comprising: predicting that a protein comprises a B-cell epitope that is likely to instigate a binding event with an antibody using a method according to any of claims 1 to 14; and
synthesising the protein.

**24.** A protein synthesised using the method of claim 23.

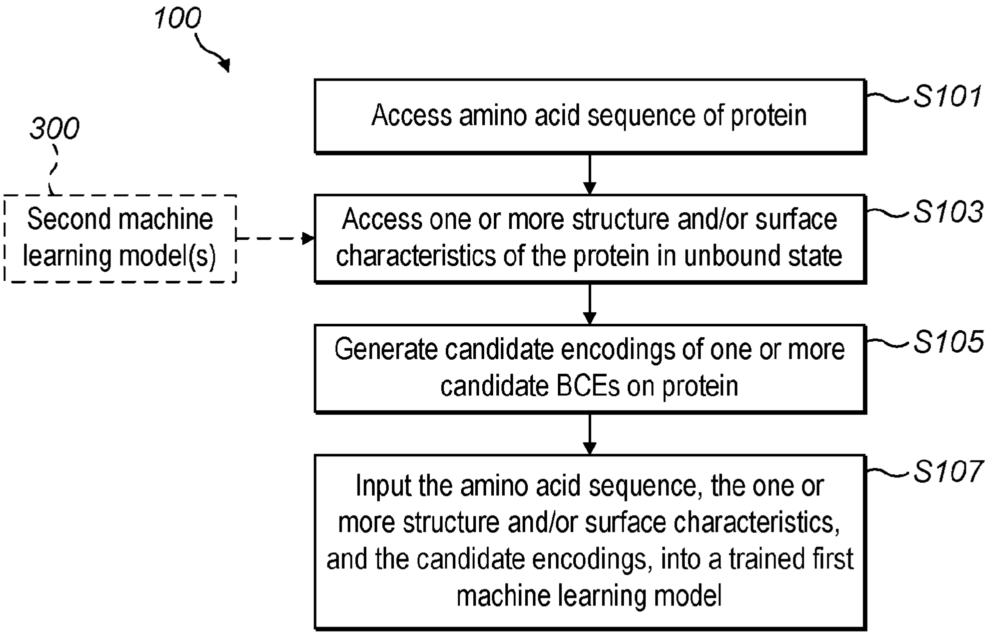
100
300
Second machine learning model(s)
Access amino acid sequence of protein
S101
Access one or more structure and/or surface characteristics of the protein in unbound state
S103
Generate candidate encodings of one or more candidate BCEs on protein
S105
Input the amino acid sequence, the one or more structure and/or surface characteristics, and the candidate encodings, into a trained first machine learning model
S107

FIG. 1

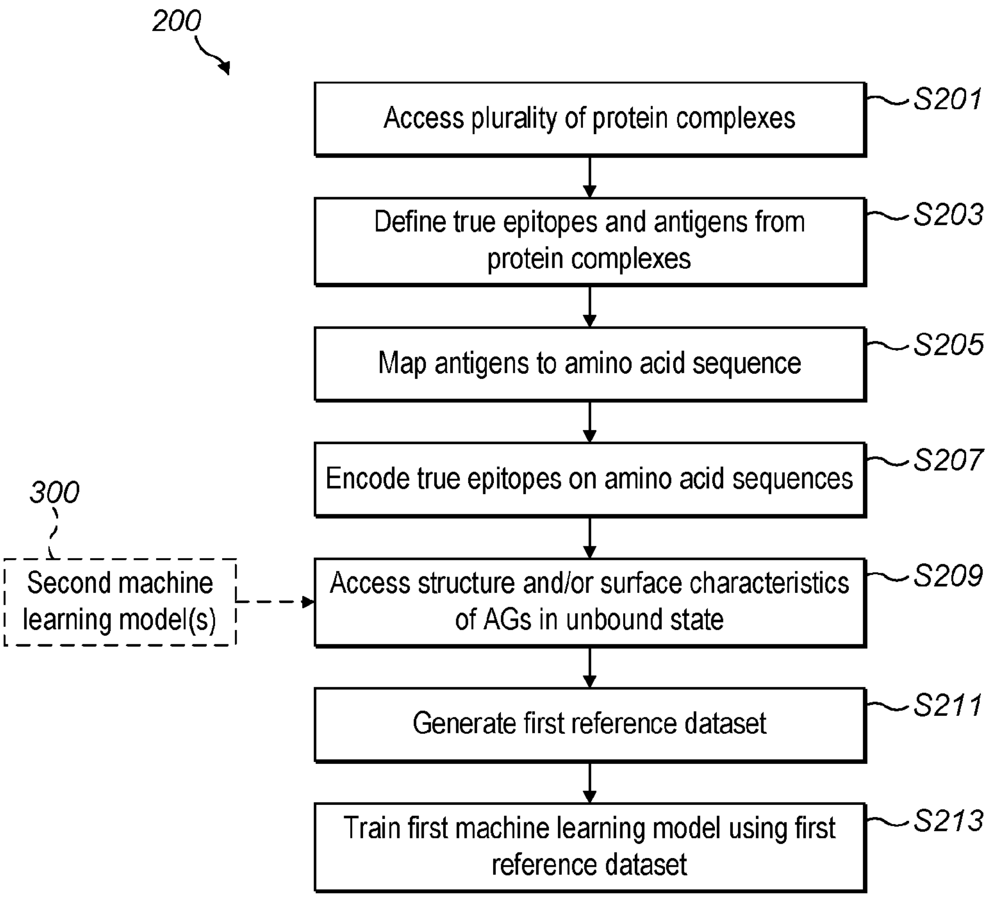
200
Access plurality of protein complexes
S201
Define true epitopes and antigens from protein complexes
S203
Map antigens to amino acid sequence
S205
Encode true epitopes on amino acid sequences
S207
300
Second machine learning model(s)
Access structure and/or surface characteristics of AGs in unbound state
S209
Generate first reference dataset
S211
Train first machine learning model using first reference dataset
S213

FIG. 2

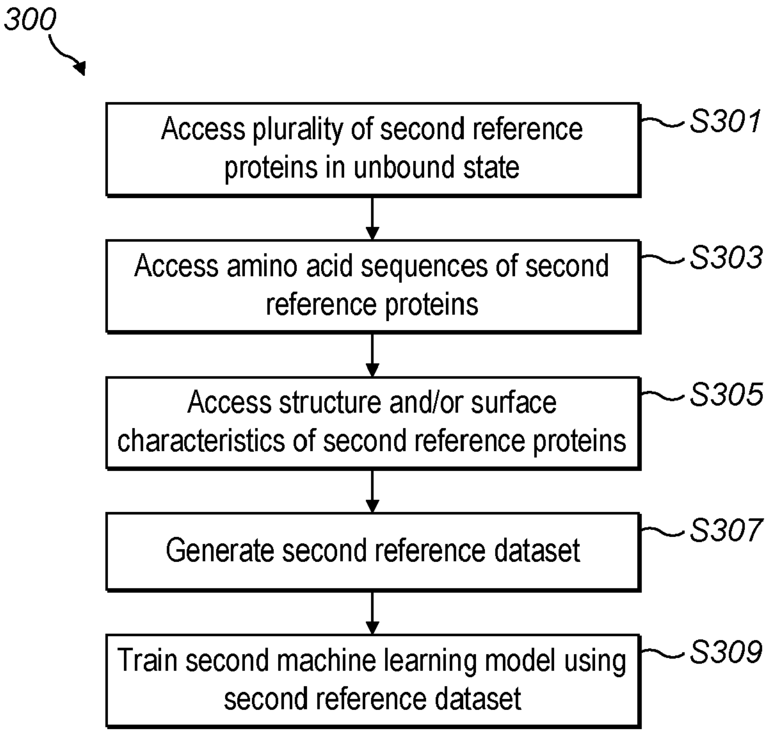
300
Access plurality of second reference proteins in unbound state
S301
Access amino acid sequences of second reference proteins
S303
Access structure and/or surface characteristics of second reference proteins
S305
Generate second reference dataset
S307
Train second machine learning model using second reference dataset
S309

FIG. 3

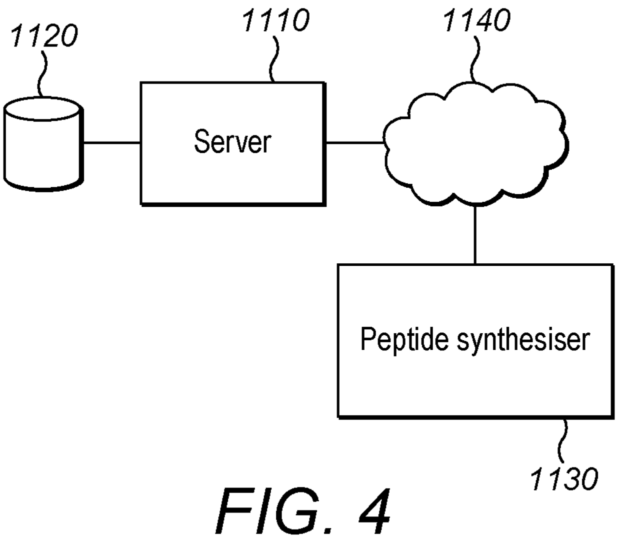
1120
1110
1140
Server
Peptide synthesiser
1130

FIG. 4

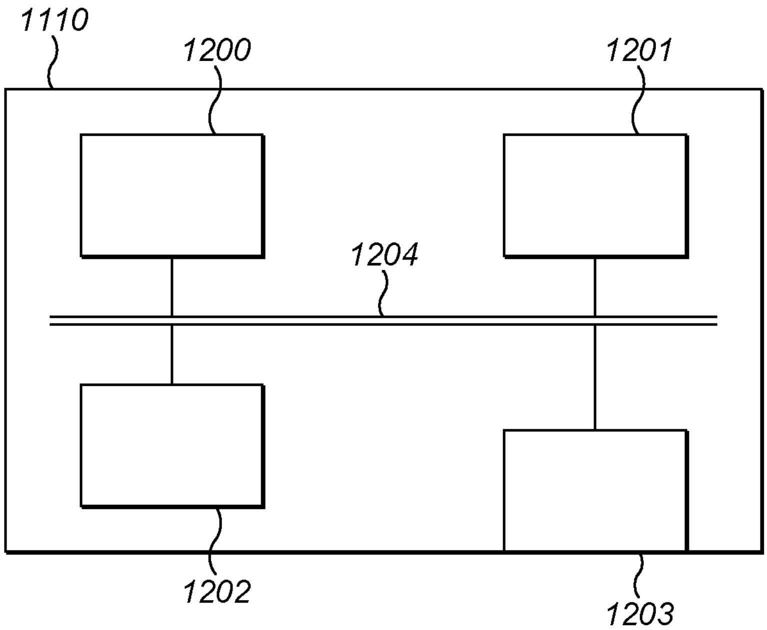


FIG. 5

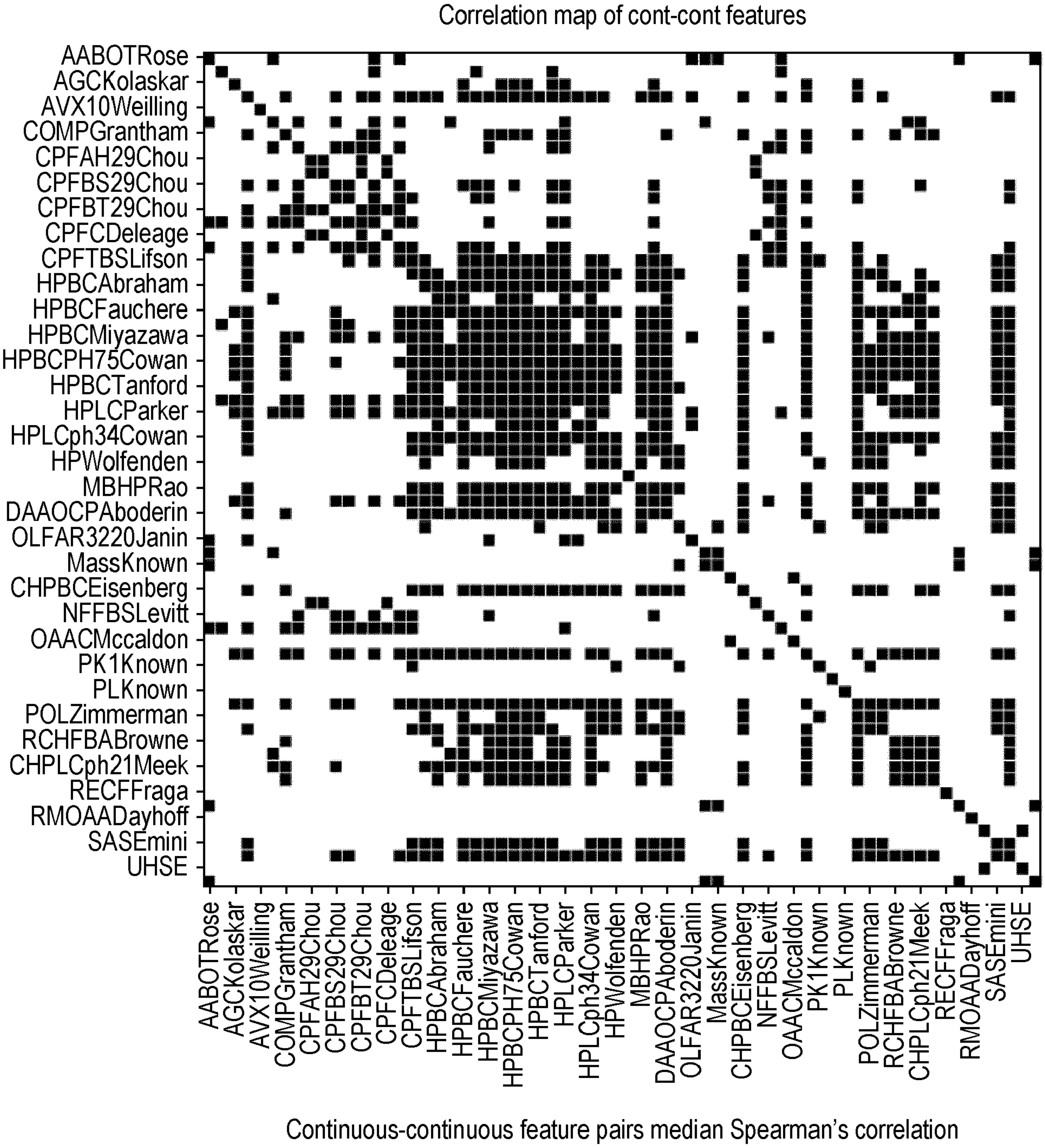
Correlation map of cont-cont features
AABOTRose
AGCKolaskar
AVX10Weilling
COMPGrantham
CPFAH29Chou
CPFBS29Chou
CPFBT29Chou
CPFCDeleage
CPFTBSLifson
HPBCAbraham
HPBCFauchere
HPBCMiyazawa
HPBCPH75Cowan
HPBCTanford
HPLCParker
HPLCph34Cowan
HPWolfenden
MBHPRao
DAAOCPAboderin
OLFAR3220Janin
MassKnown
CHPBCEisenberg
NFFBSLevitt
OAACMccaldon
PK1Known
PLKnown
POLZimmerman
RCHFBABrowne
CHPLCph21Meek
RECFFraga
RMOAADayhoff
SASEmini
UHSE
Continuous-continuous feature pairs median Spearman's correlation

FIG. 6(a)

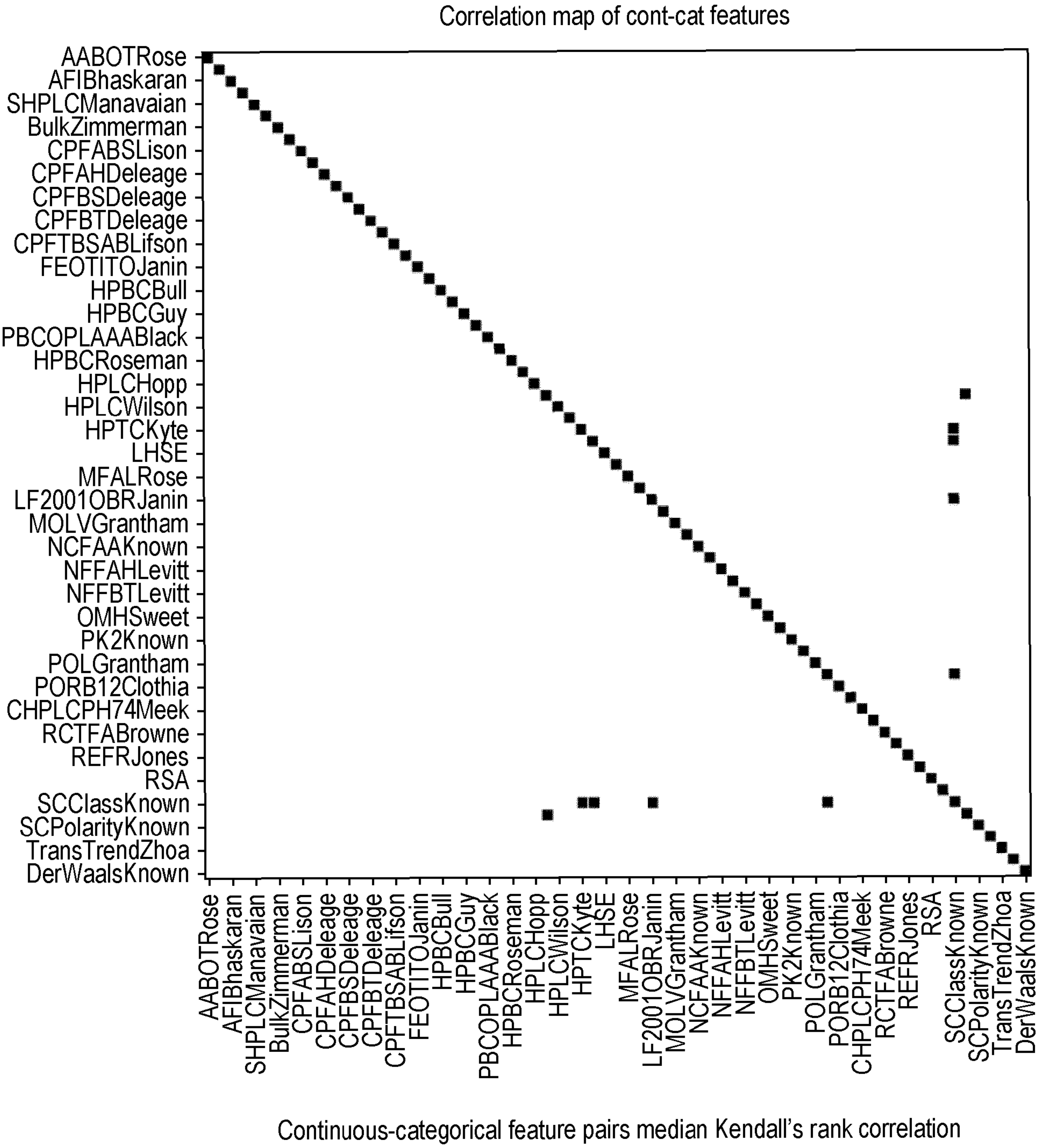
Correlation map of cont-cat features
AABOTRose
AFIBhaskaran
SHPLCManavaian
BulkZimmerman
CPFABSLison
CPFAHDeleage
CPFBSDeleage
CPFBTDeleage
CPFTBSABLifson
FEOTITOJanin
HPBCBull
HPBCGuy
PBCOPLAAABlack
HPBCRoseman
HPLCHopp
HPLCWilson
HPTCKyte
LHSE
MFALRose
LF2001OBRJanin
MOLVGrantham
NCFAAKnown
NFFAHLevitt
NFFBTLevitt
OMHSweet
PK2Known
POLGrantham
PORB12Clothia
CHPLCPH74Meek
RCTFABrowne
REFRJones
RSA
SCClassKnown
SCPolarityKnown
TransTrendZhoa
DerWaalsKnown
Continuous-categorical feature pairs median Kendall's rank correlation

*FIG. 6(b)*

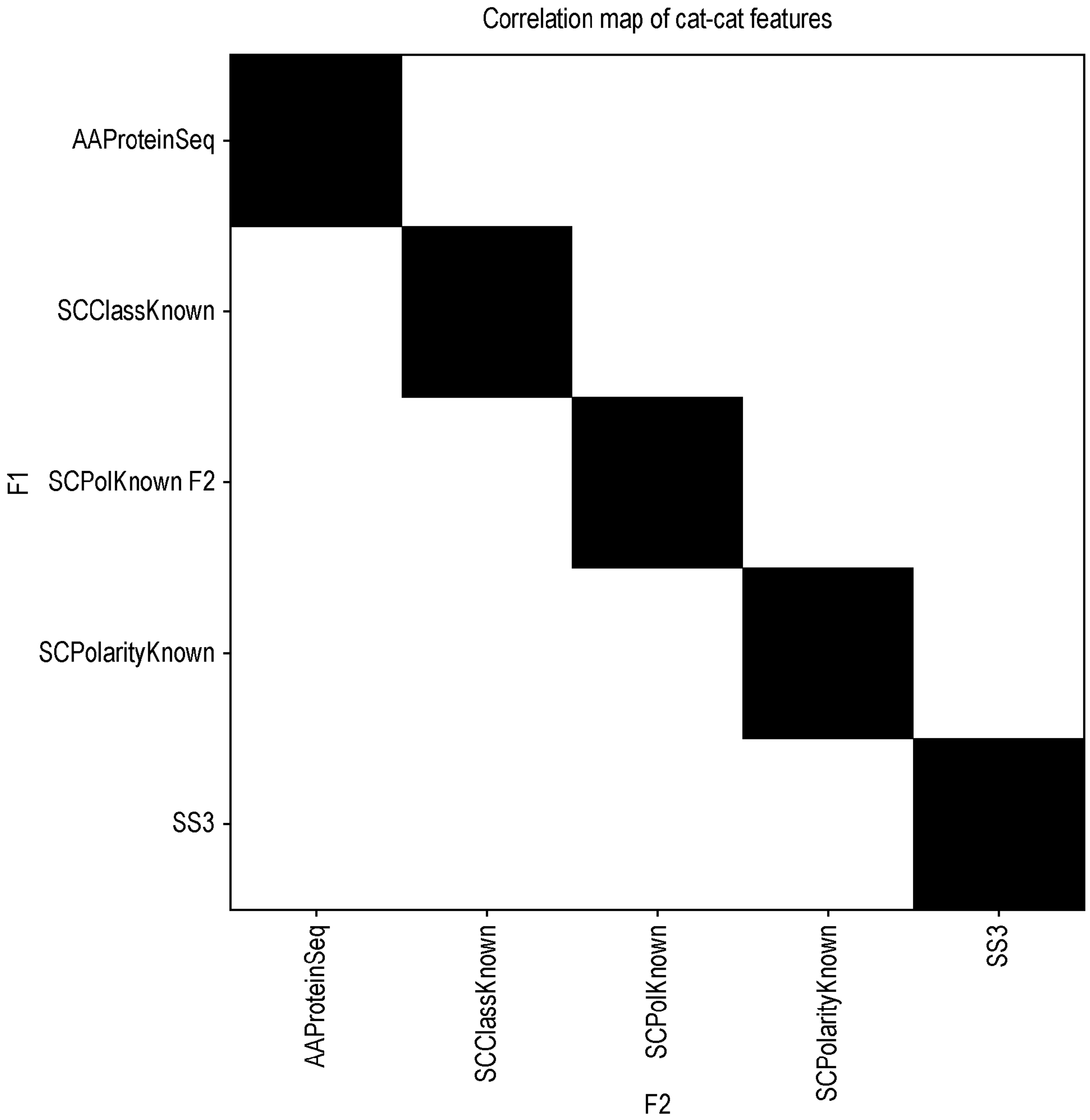


Categorical-categorical feature pairs median mutual information

*FIG. 6(c)*

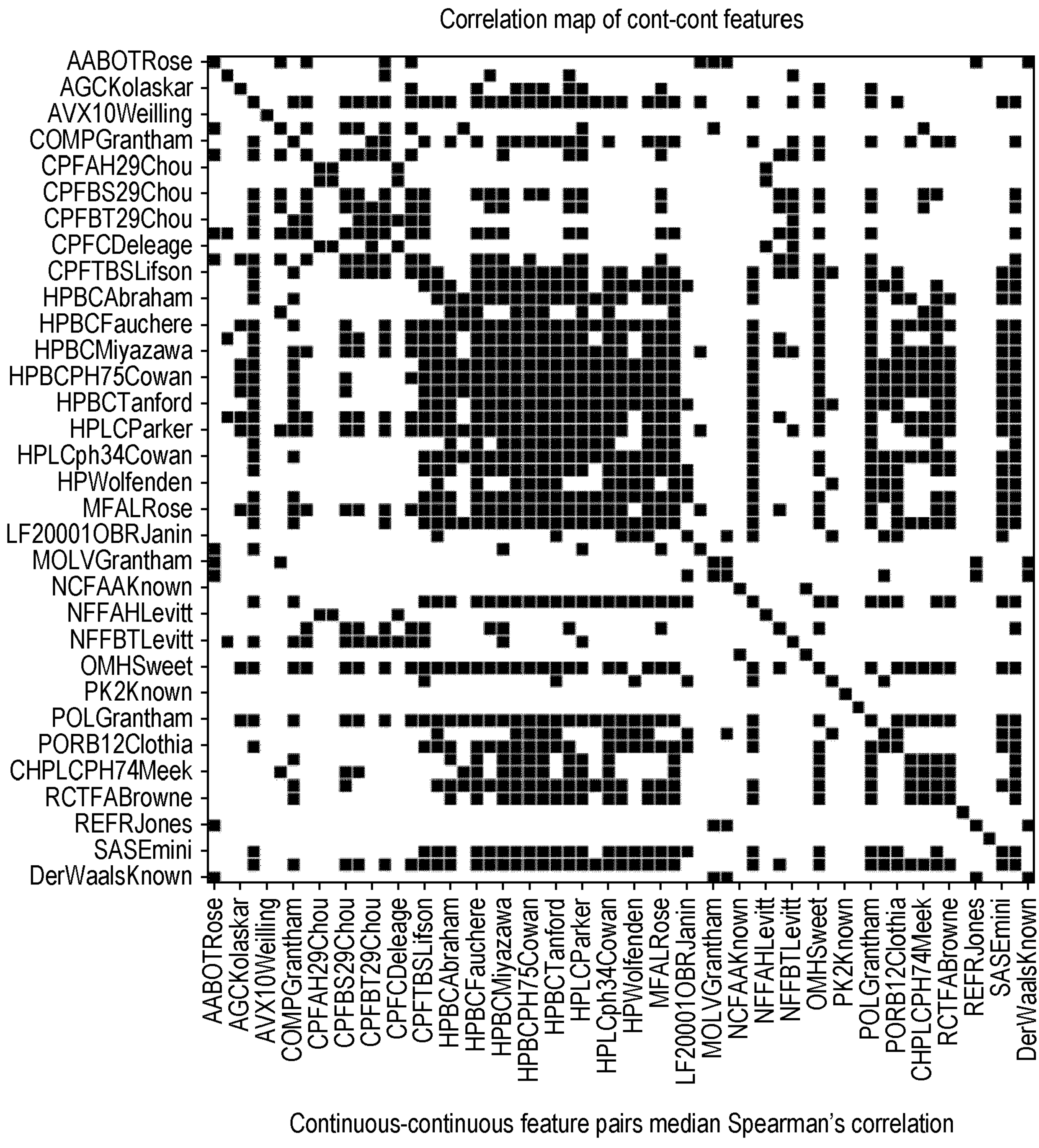
Correlation map of cont-cont features
AABOTRose
AGCKolaskar
AVX10Weilling
COMPGrantham
CPFAH29Chou
CPFBS29Chou
CPFBT29Chou
CPFCDeleage
CPFTBSLifson
HPBCAbraham
HPBCFauchere
HPBCMiyazawa
HPBCPH75Cowan
HPBCTanford
HPLCParker
HPLCph34Cowan
HPWolfenden
MFALRose
LF20001OBRJanin
MOLVGrantham
NCFAAKnown
NFFAHLevitt
NFFBTLevitt
OMHSweet
PK2Known
POLGrantham
PORB12Clothia
CHPLCPH74Meek
RCTFABrowne
REFRJones
SASEmini
DerWaalsKnown
Continuous-continuous feature pairs median Spearman's correlation

FIG. 6(d)

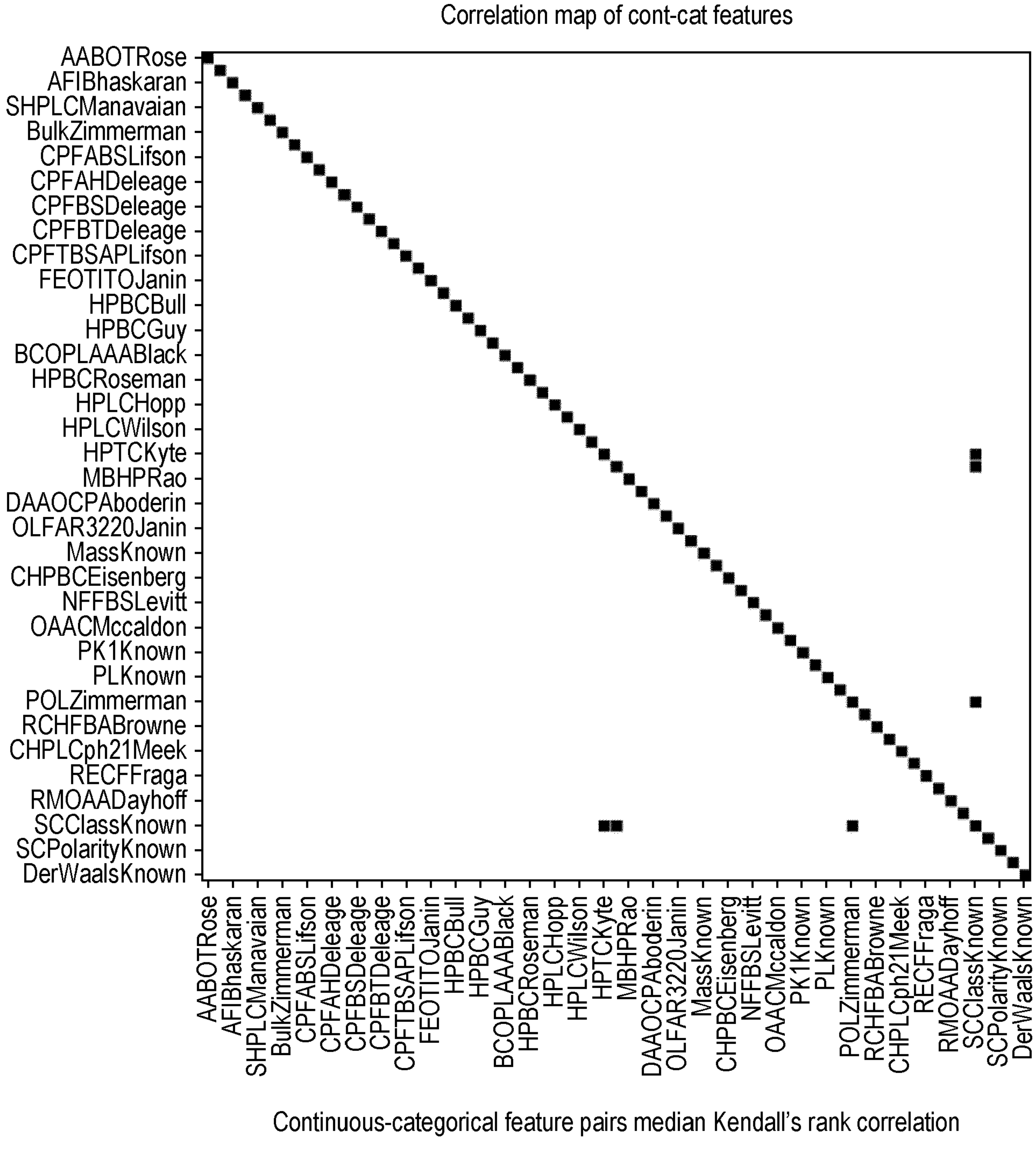
Correlation map of cont-cat features
AABOTRose
AFIBhaskaran
SHPLCManavaian
BulkZimmerman
CPFABSLifson
CPFAHDeleage
CPFBSDeleage
CPFBTDeleage
CPFTBSAPLifson
FEOTITOJanin
HPBCBull
HPBCGuy
BCOPLAAABlack
HPBCRoseman
HPLCHopp
HPLCWilson
HPTCKyte
MBHPRao
DAAOCPAboderin
OLFAR3220Janin
MassKnown
CHPBCEisenberg
NFFBSLevitt
OAACMccaldon
PK1Known
PLKnown
POLZimmerman
RCHFBABrowne
CHPLCph21Meek
RECFFraga
RMOAADayhoff
SCClassKnown
SCPolarityKnown
DerWaalsKnown
Continuous-categorical feature pairs median Kendall's rank correlation

FIG. 6(e)

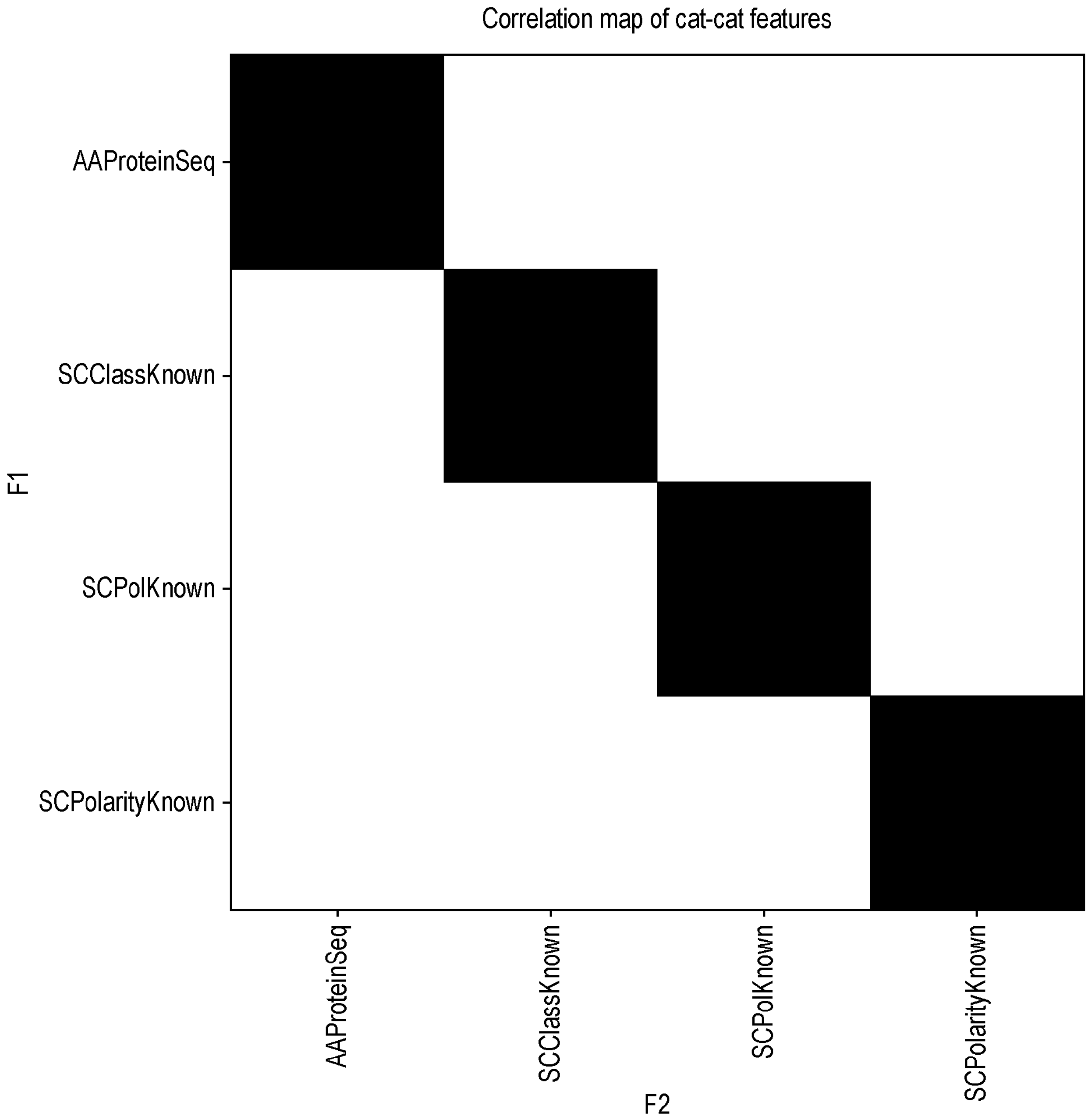


Categorical-categorical feature pairs median mutual information

FIG. 6(f)

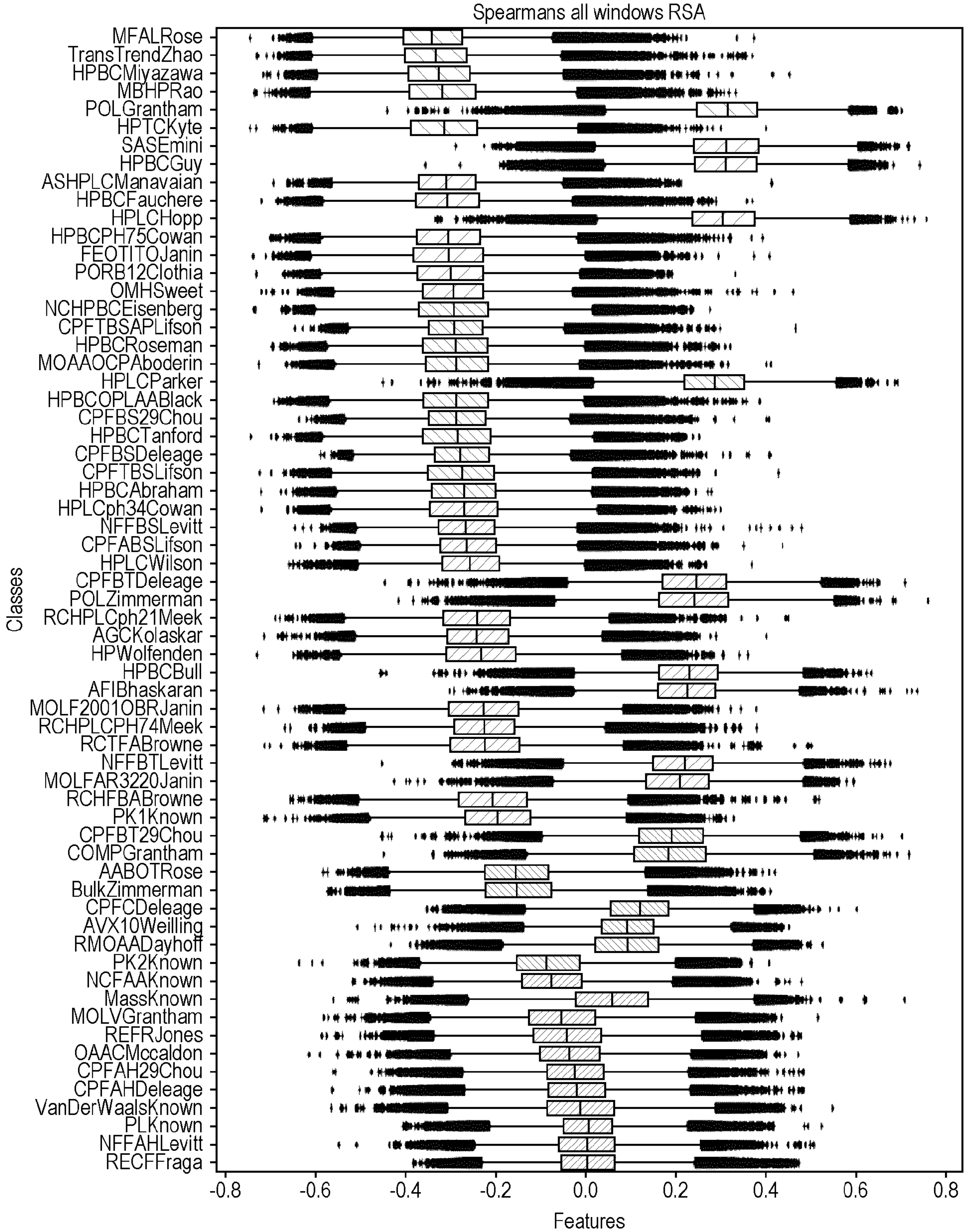


Continuous features with RSA using median Spearman's correlation

*FIG. 7(a)*

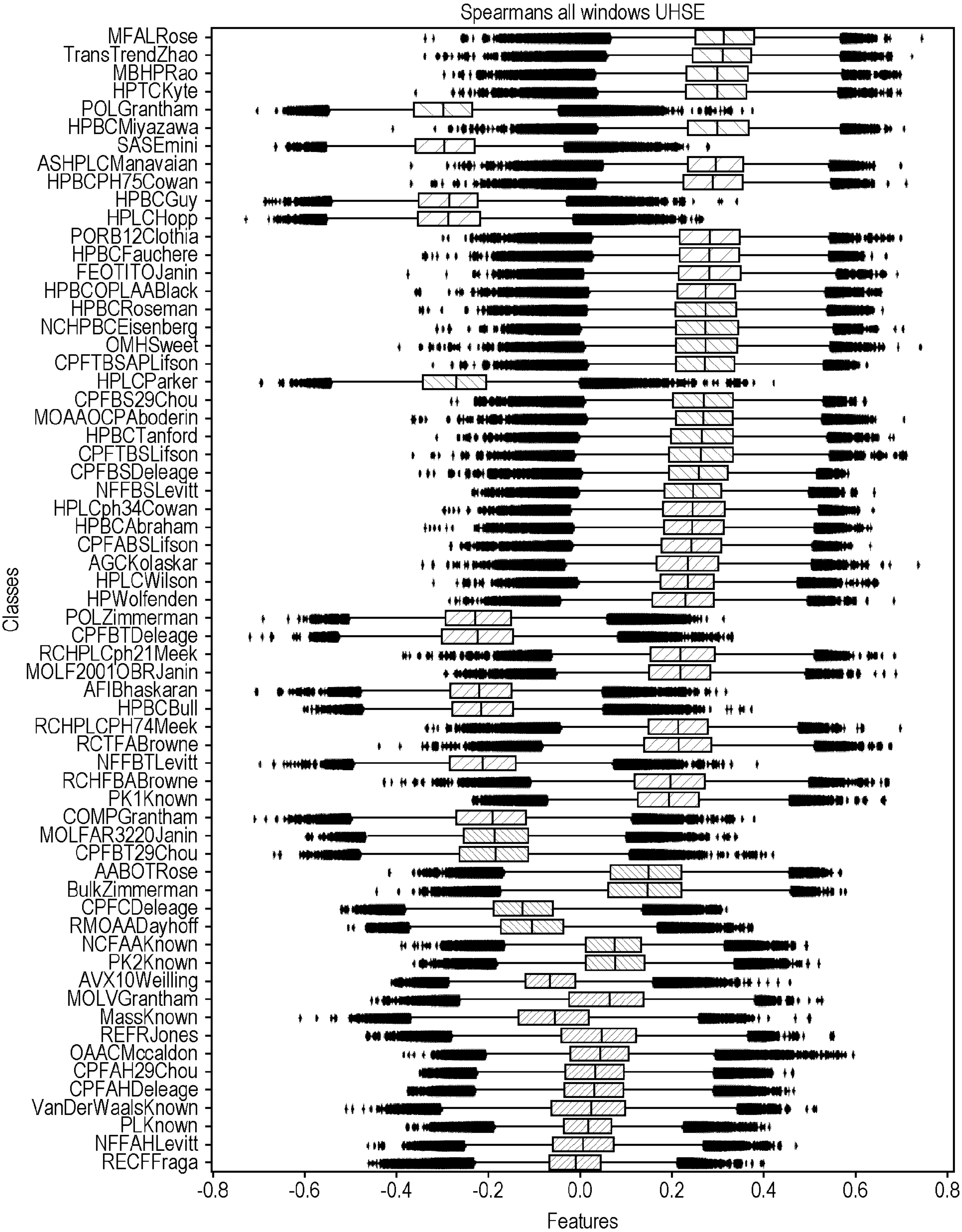


Continuous features with UHSE using median Spearman's correlation

*FIG. 7(b)*

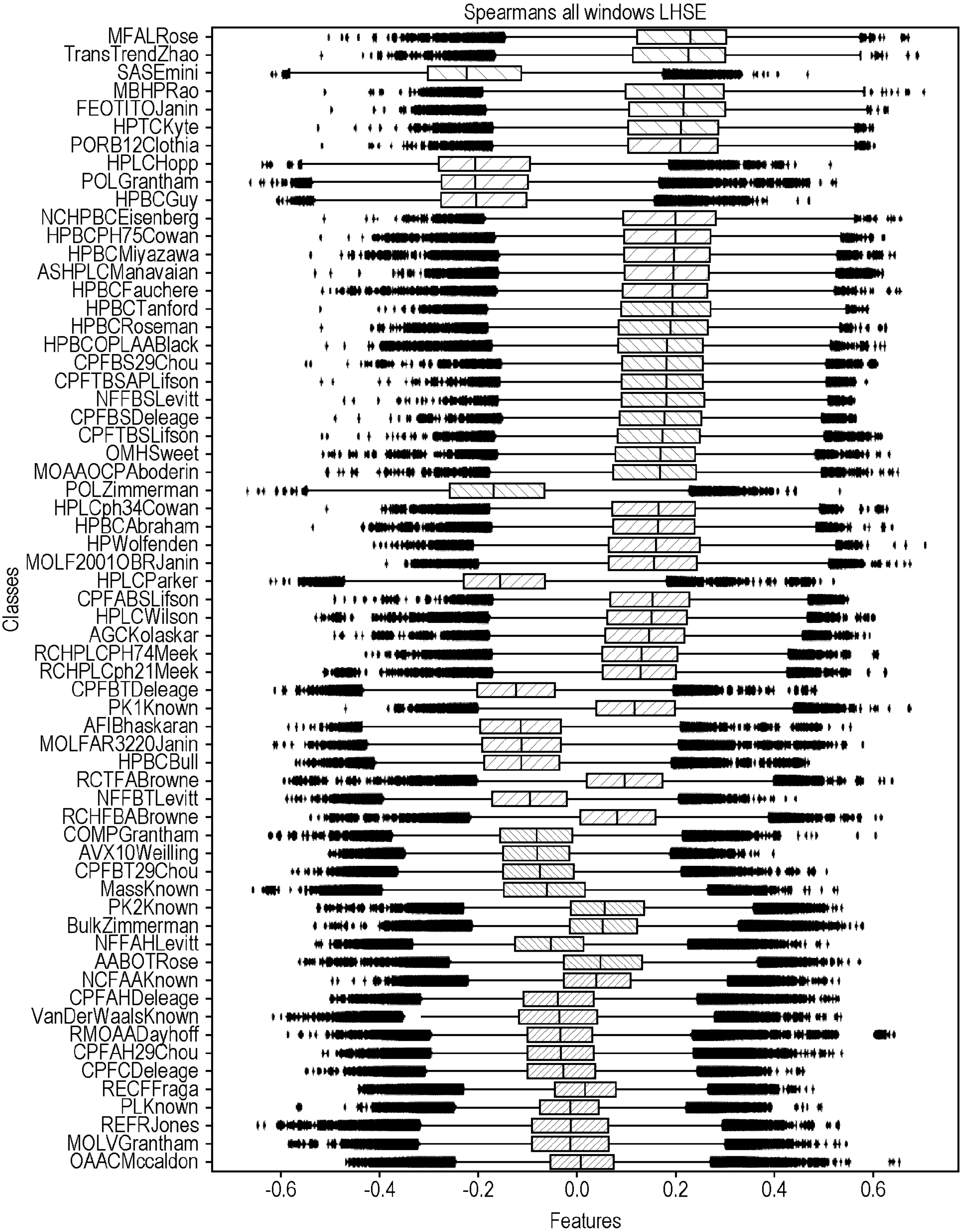


Continuous features with LHSE using median Spearman's correlation

*FIG. 7(c)*

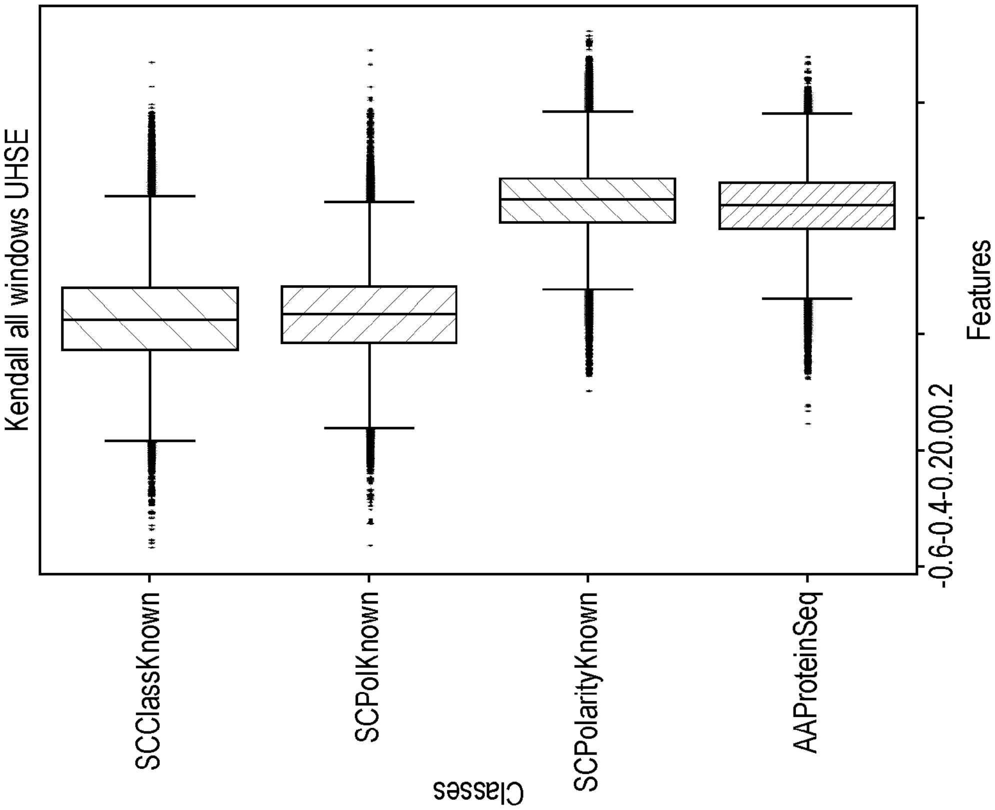


Categorical features with UHSE using median Kendall's rank correlation

*FIG. 8(b)*

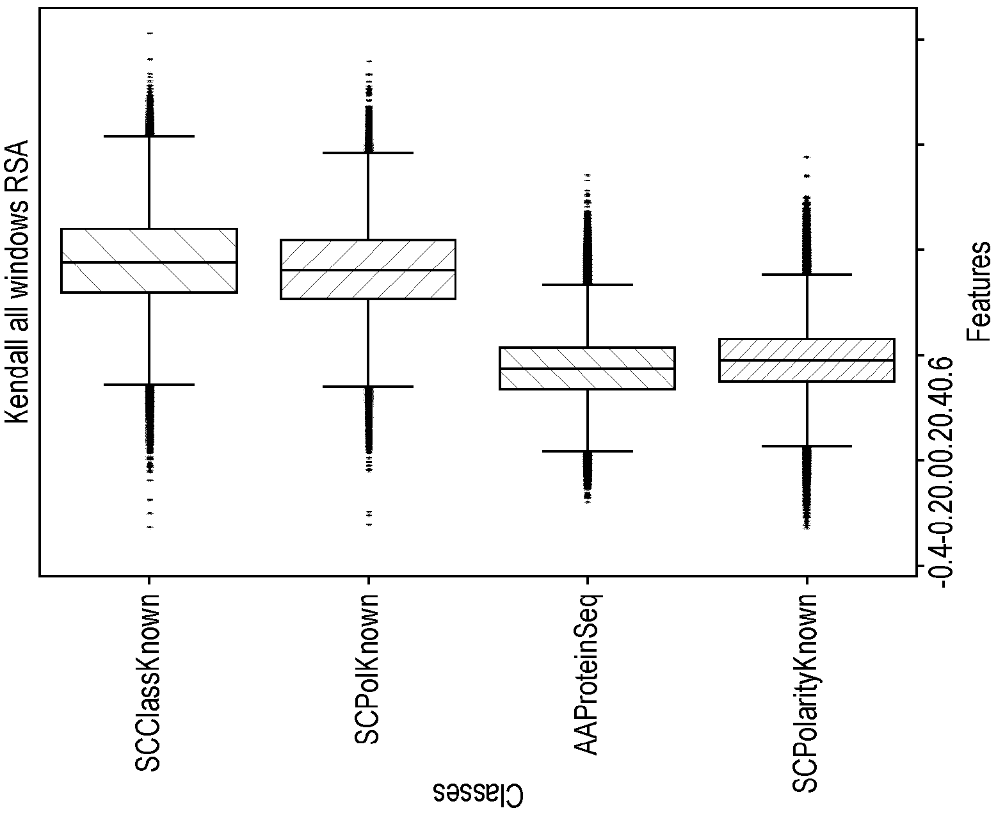


Categorical features with RSA using median Kendall's rank correlation

*FIG. 8(a)*

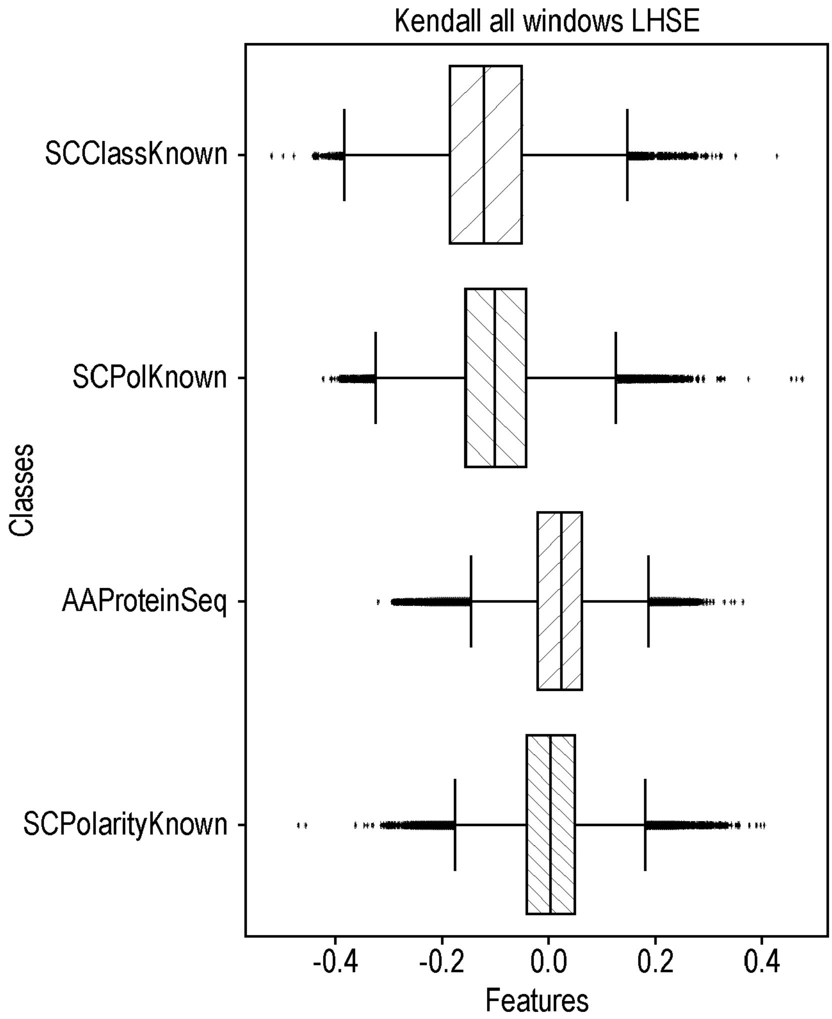


Categorical features with LHSE using median Kendall's rank correlation

*FIG. 8(c)*

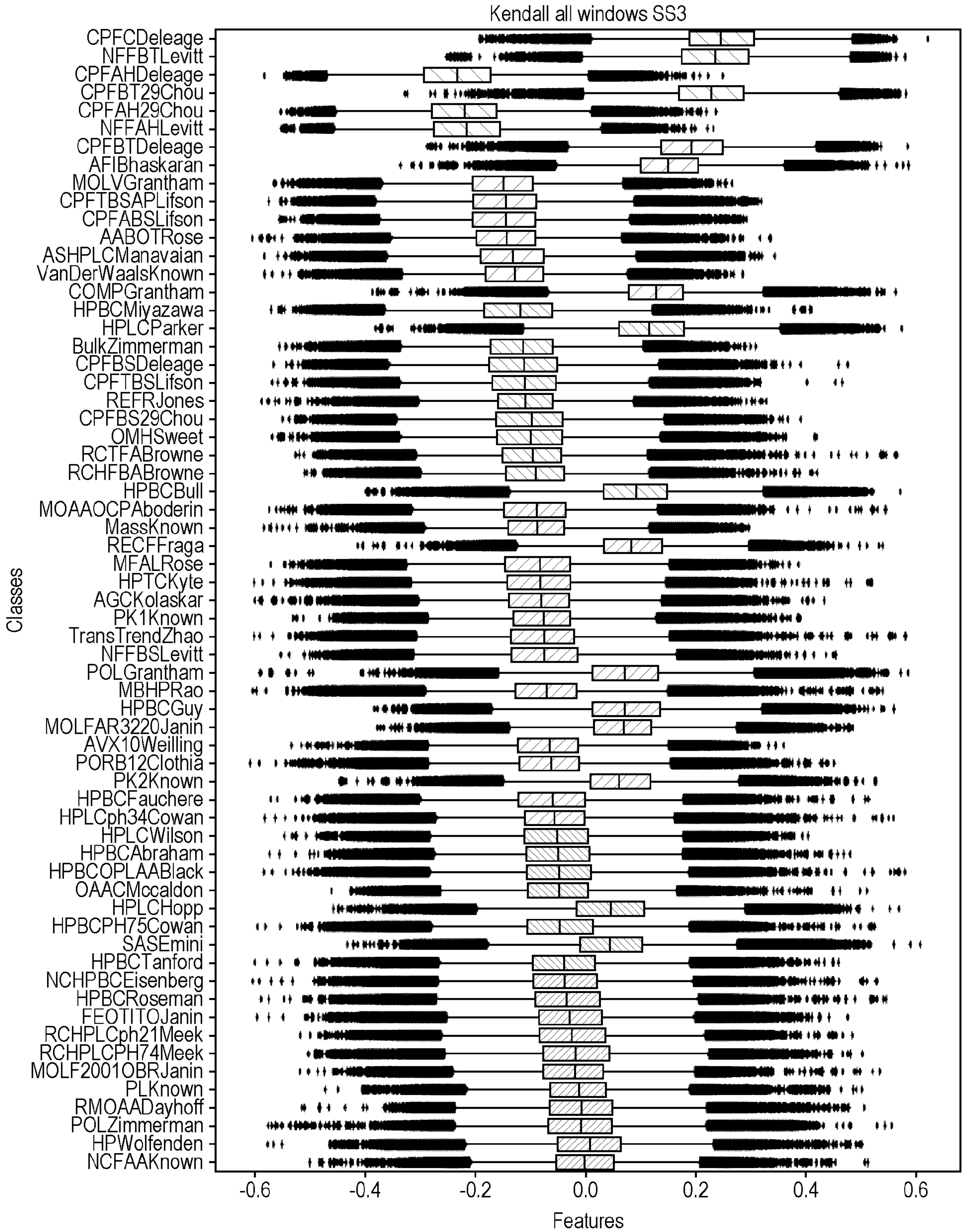


Continuous features with SS using median Kendall's rank correlation

*FIG. 9(a)*

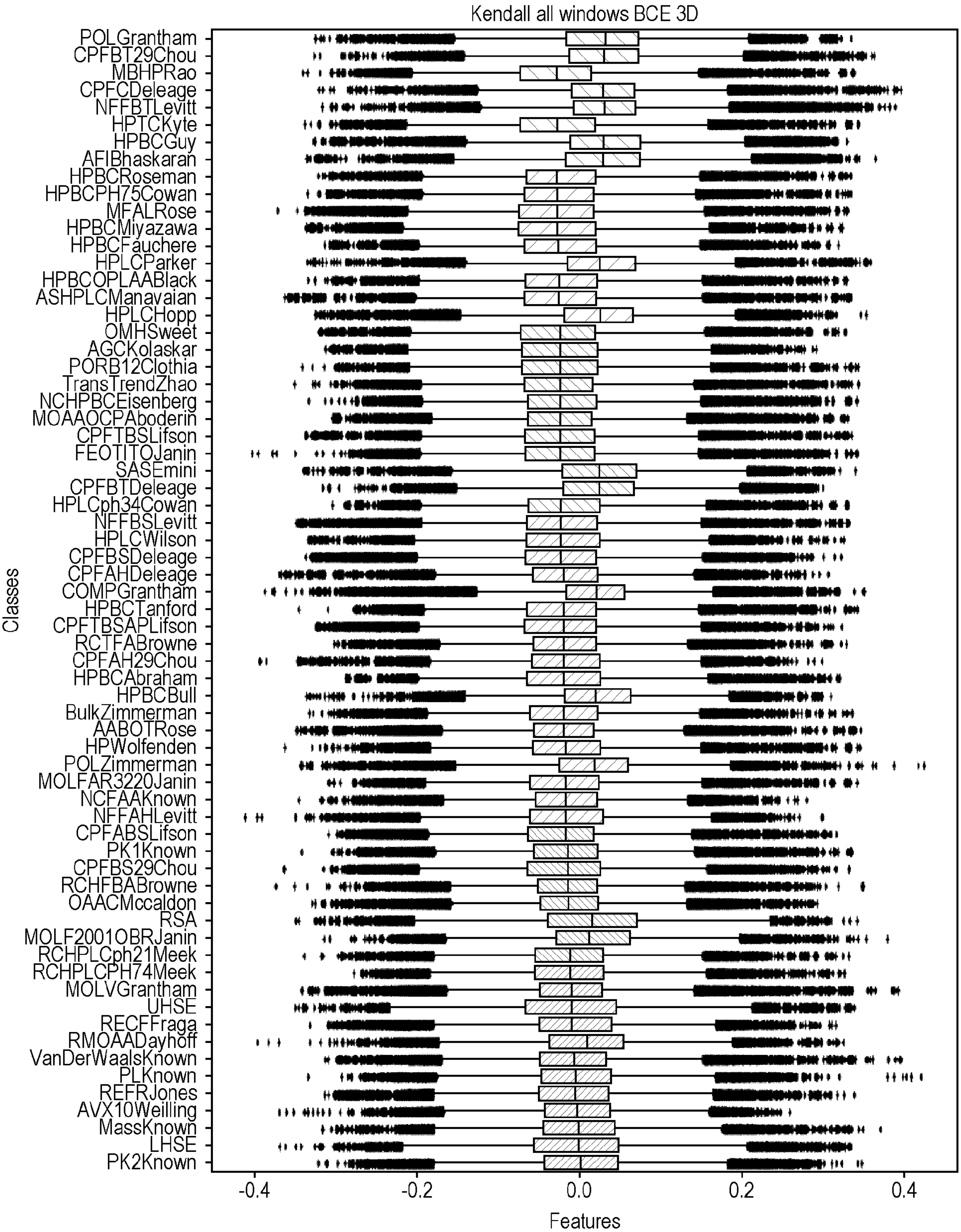


Continuous features with CBCE using median Kendall's rank correlation

*FIG. 9(b)*

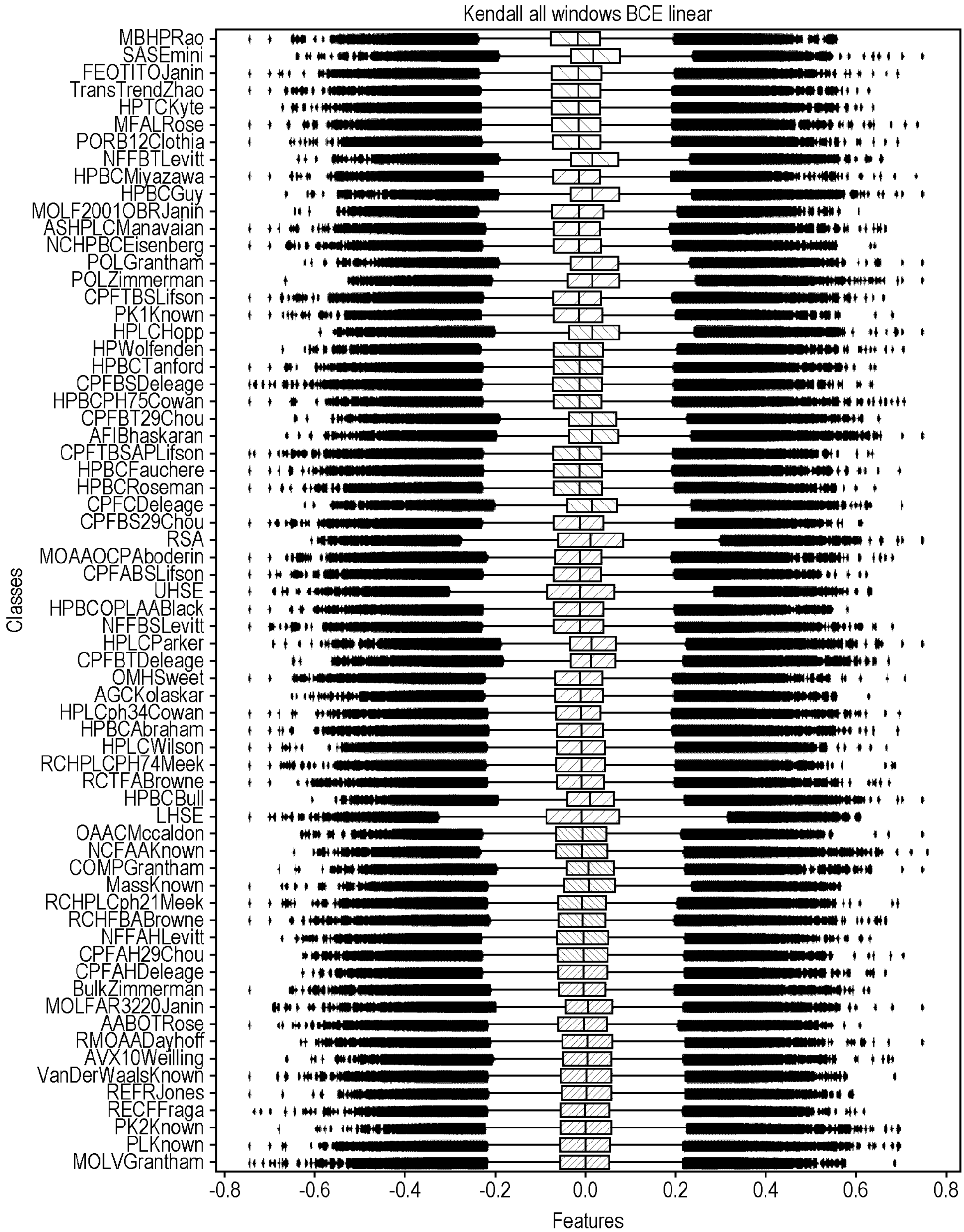


Continuous features with LBCE using median Kendall's rank correlation

*FIG. 9(c)*

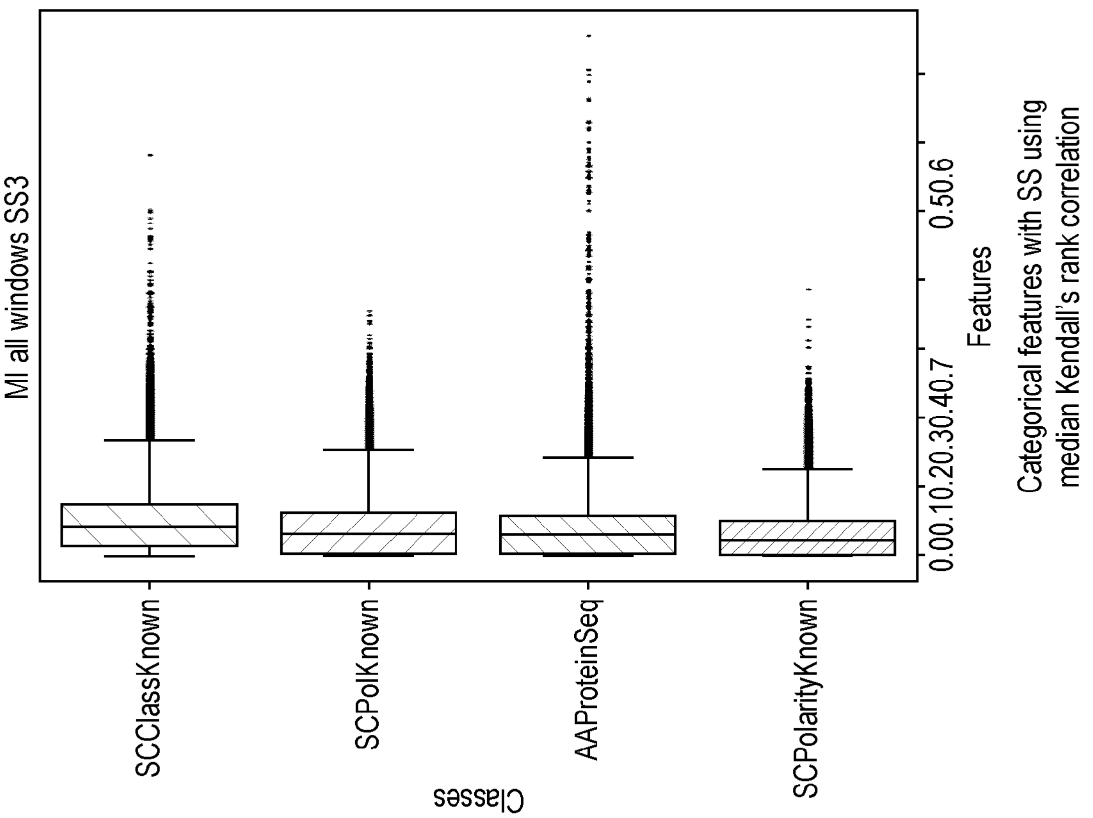


FIG. 10(a)

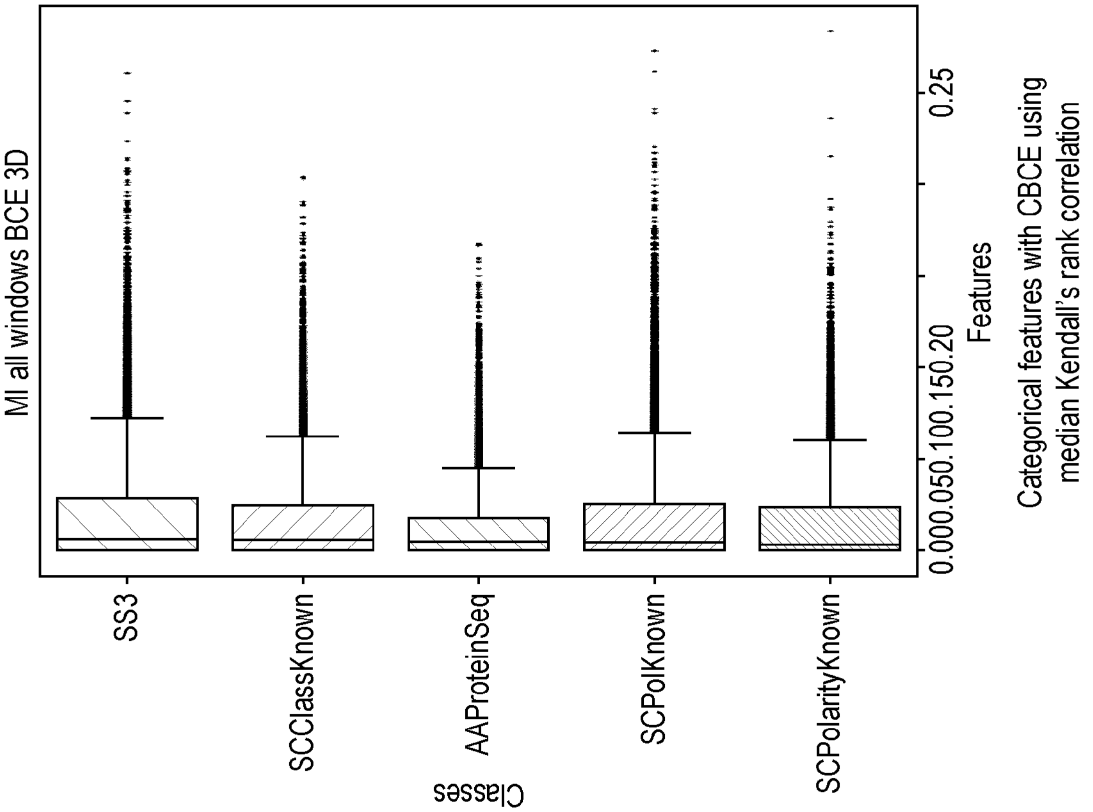


FIG. 10(b)

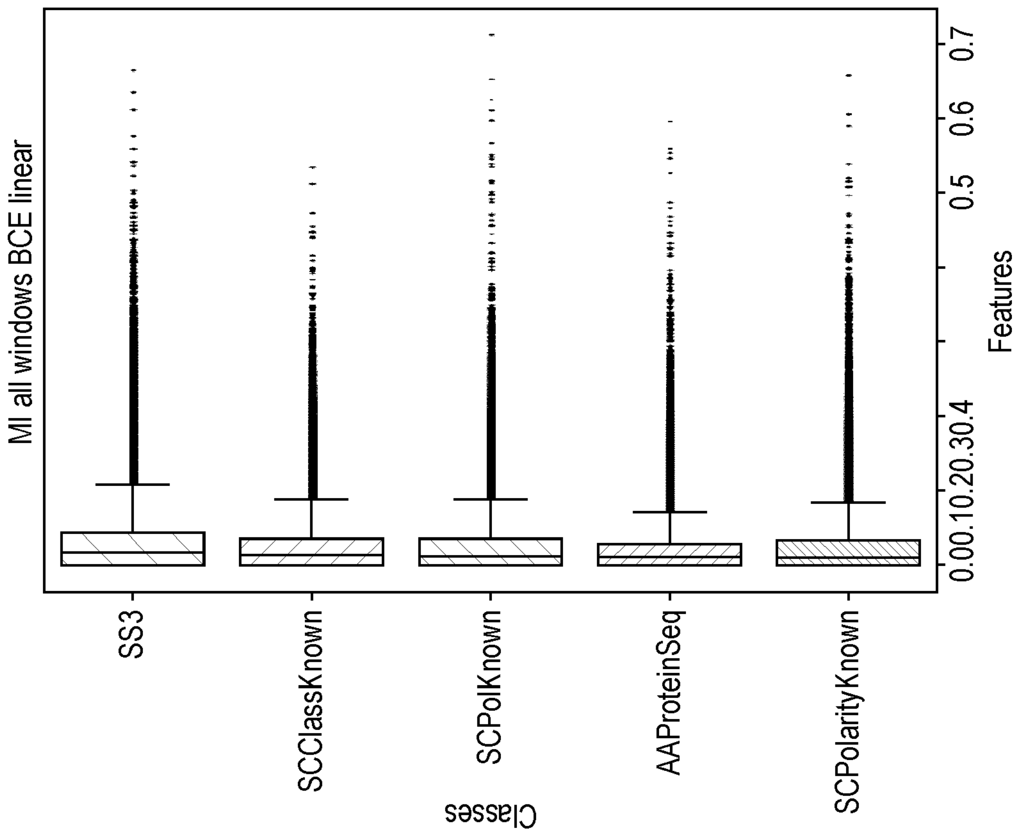


Categorical features with LBCE using median Kendall's rank correlation

FIG. 10(c)

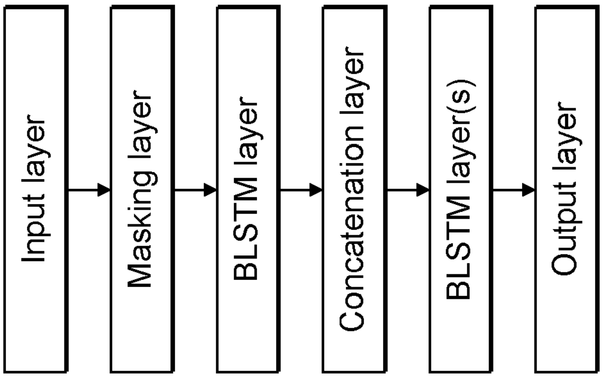


FIG. 11

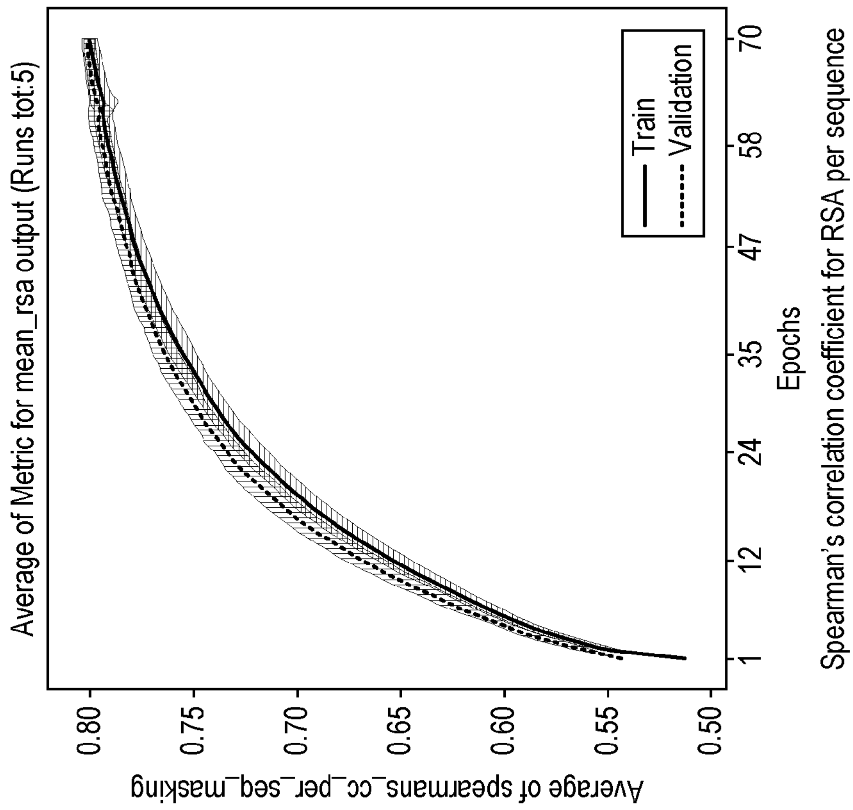


FIG. 12(a)

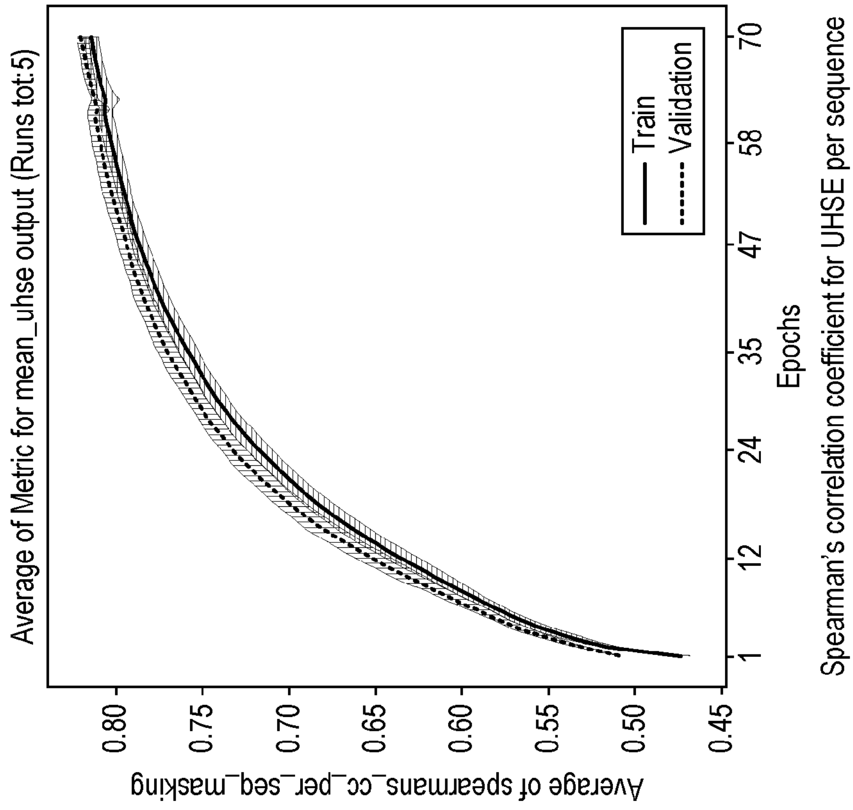


FIG. 12(b)

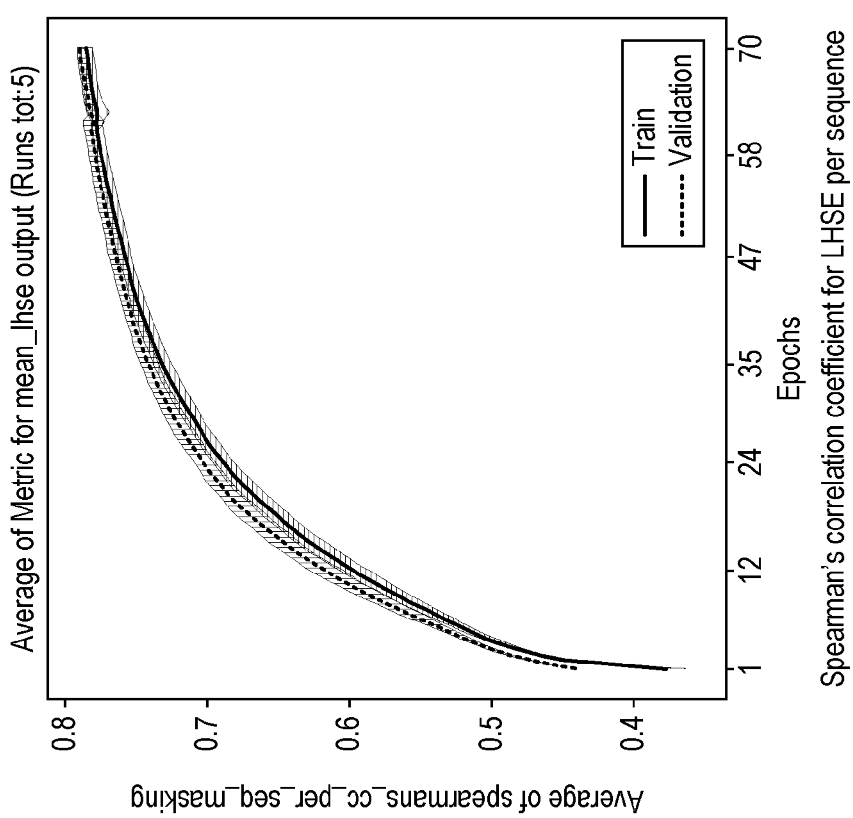


FIG. 12(c)

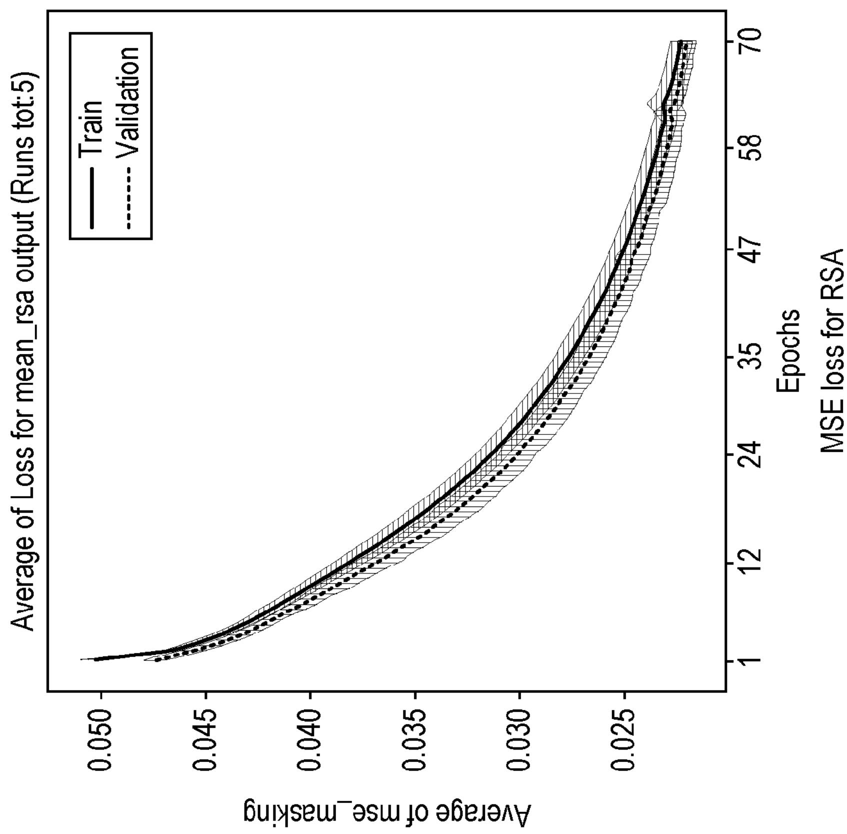


FIG. 12(d)

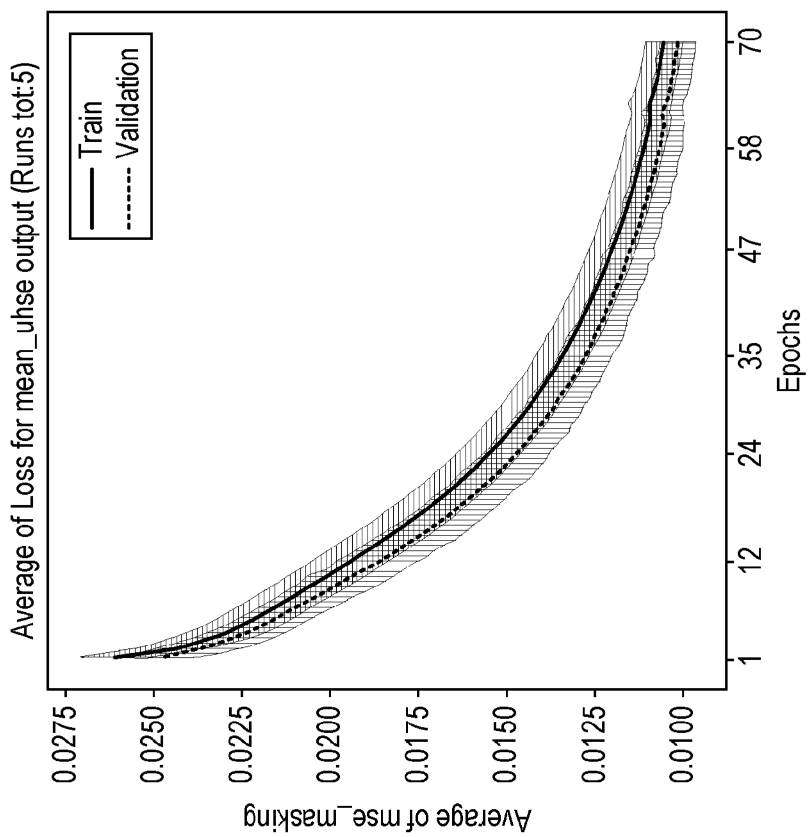


MSE loss for UHSE

FIG. 12(e)

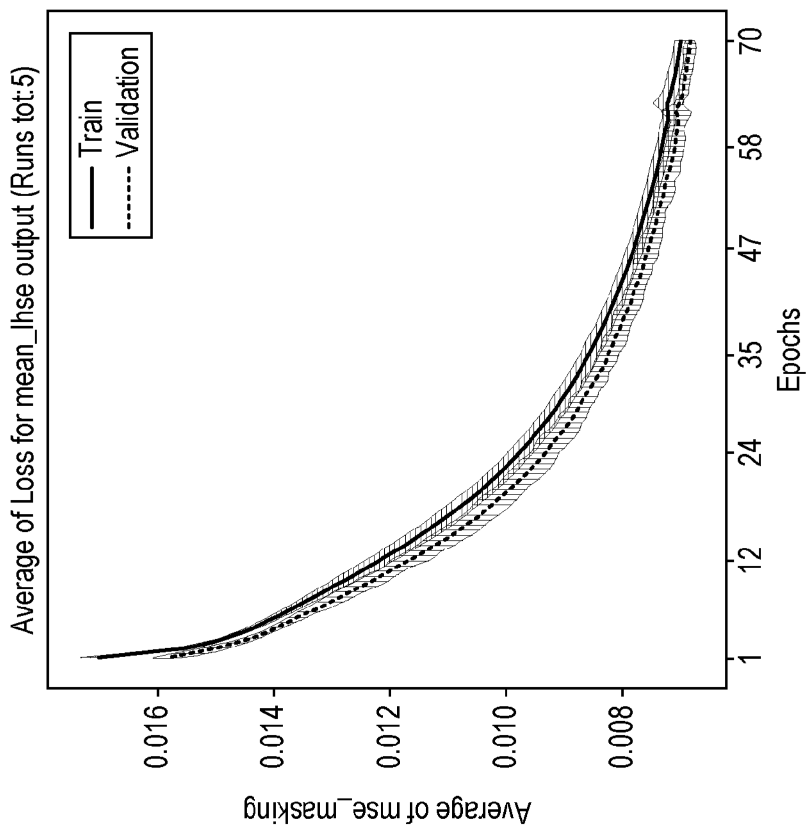


MSE loss for LHSE

FIG. 12(f)

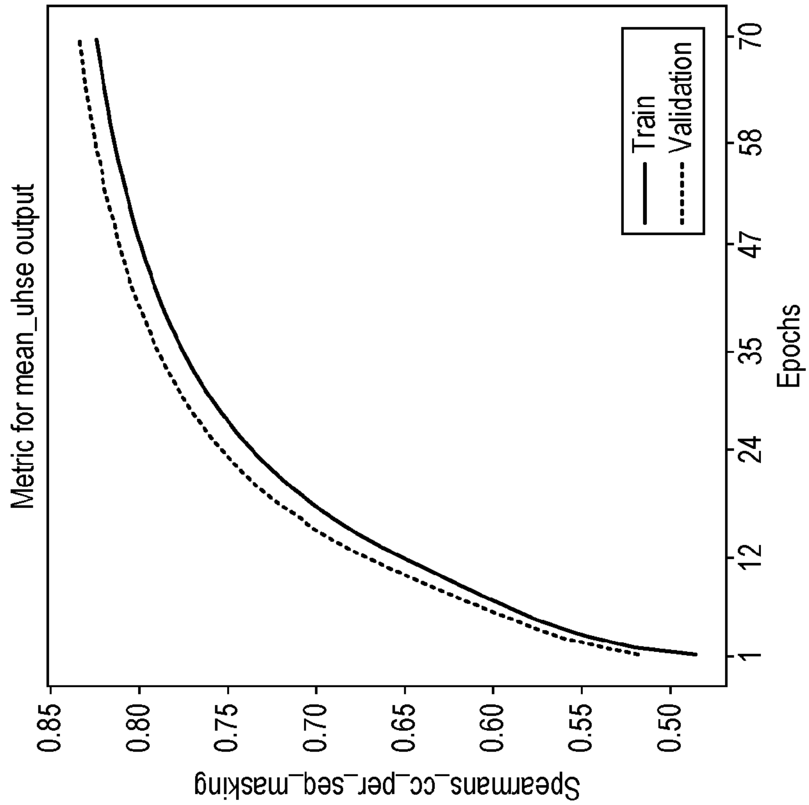


Spearman's correlation coefficient for UHSE per sequence

*FIG. 13(b)*

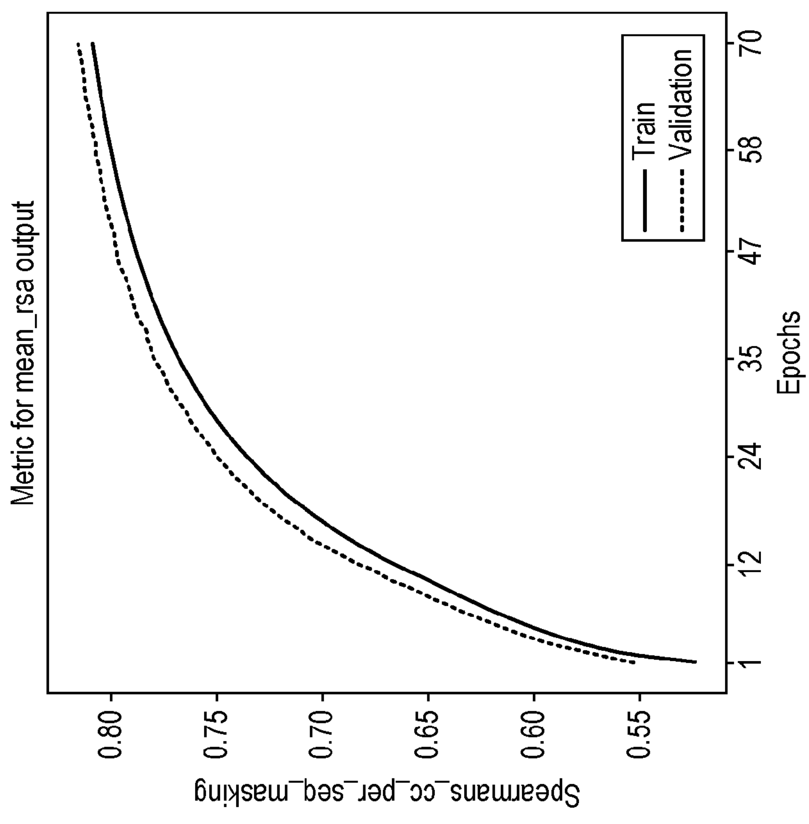


Spearman's correlation coefficient for RSA per sequence

*FIG. 13(a)*

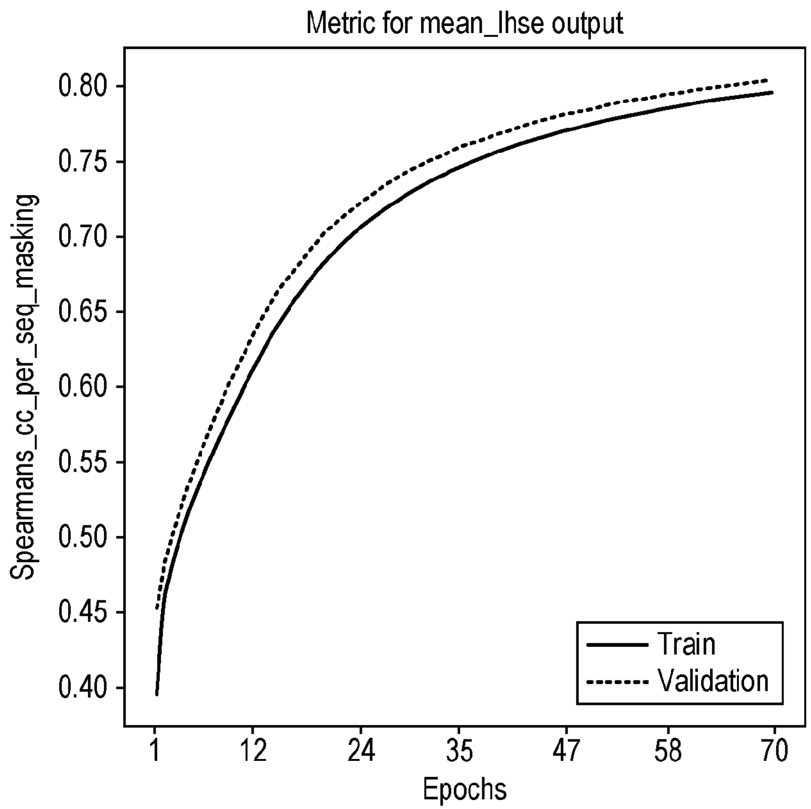

Spearman's correlation coefficient for LHSE per sequence

FIG. 13(c)

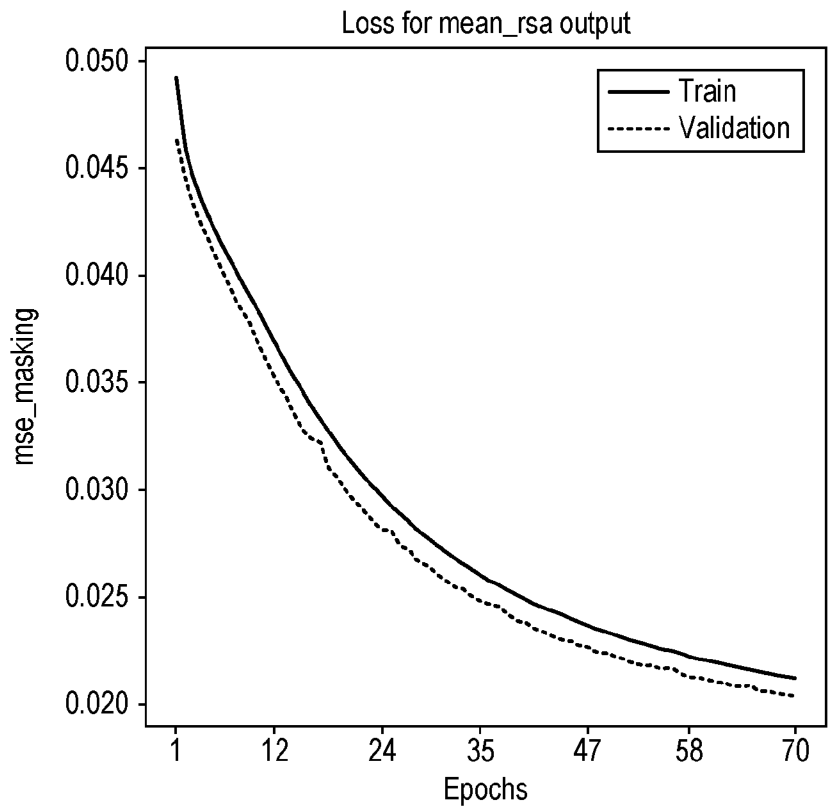

MSE loss for RSA

FIG. 13(d)

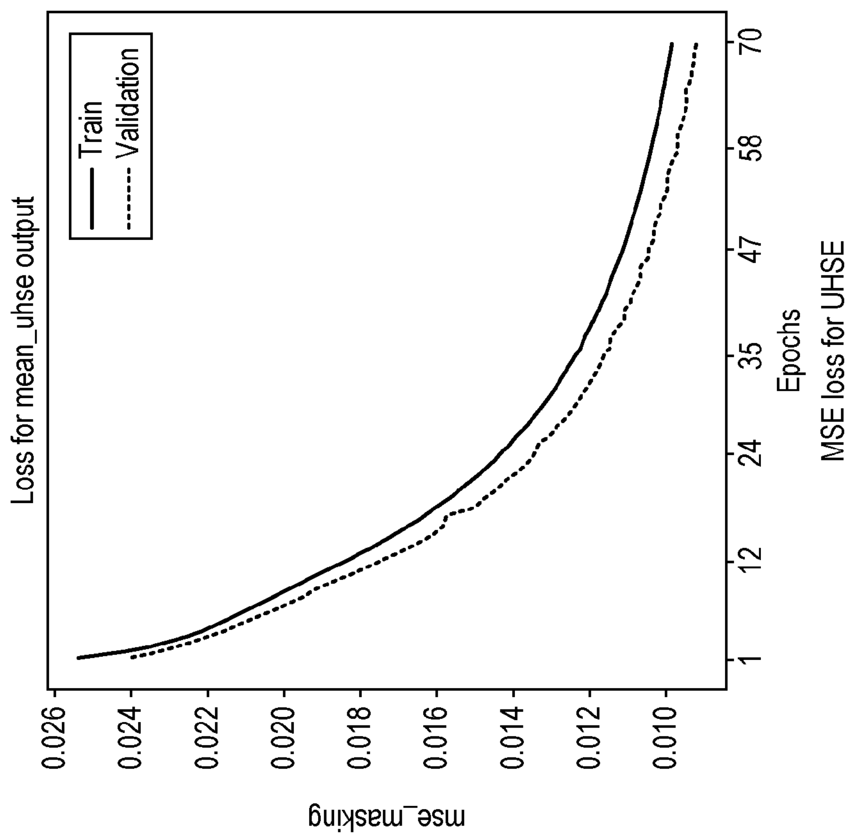


FIG. 13(e)

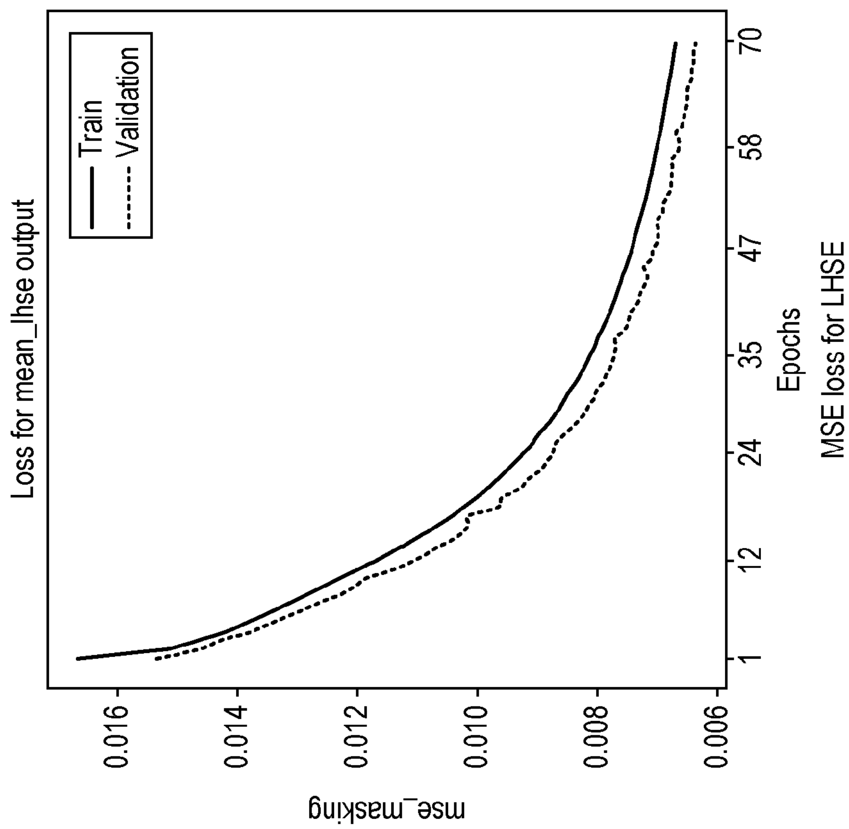


FIG. 13(f)

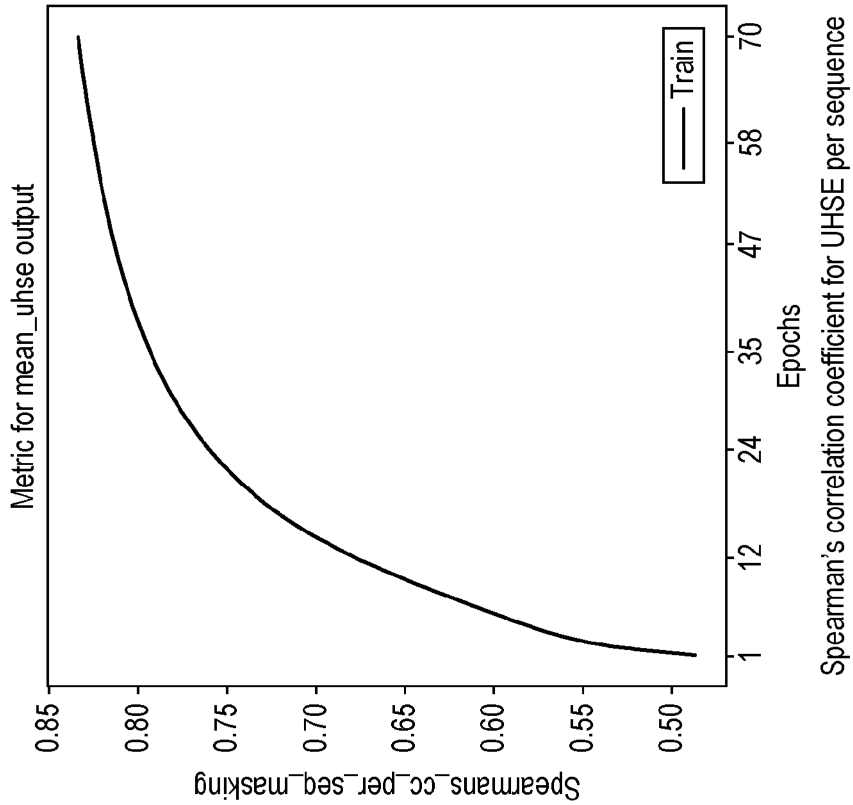


*FIG. 14(b)*

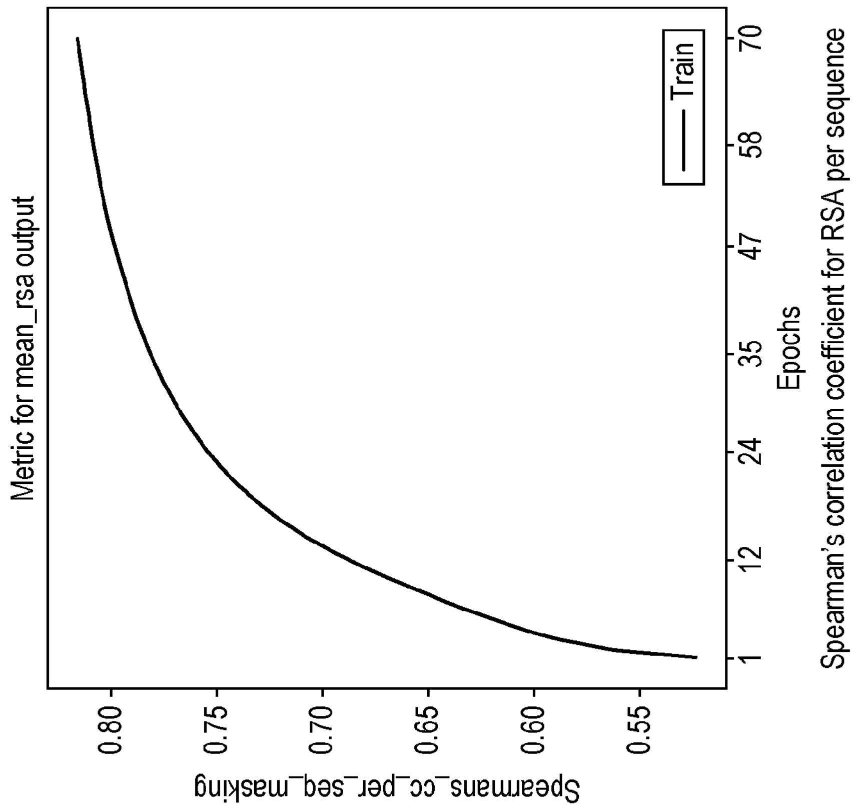


*FIG. 14(a)*

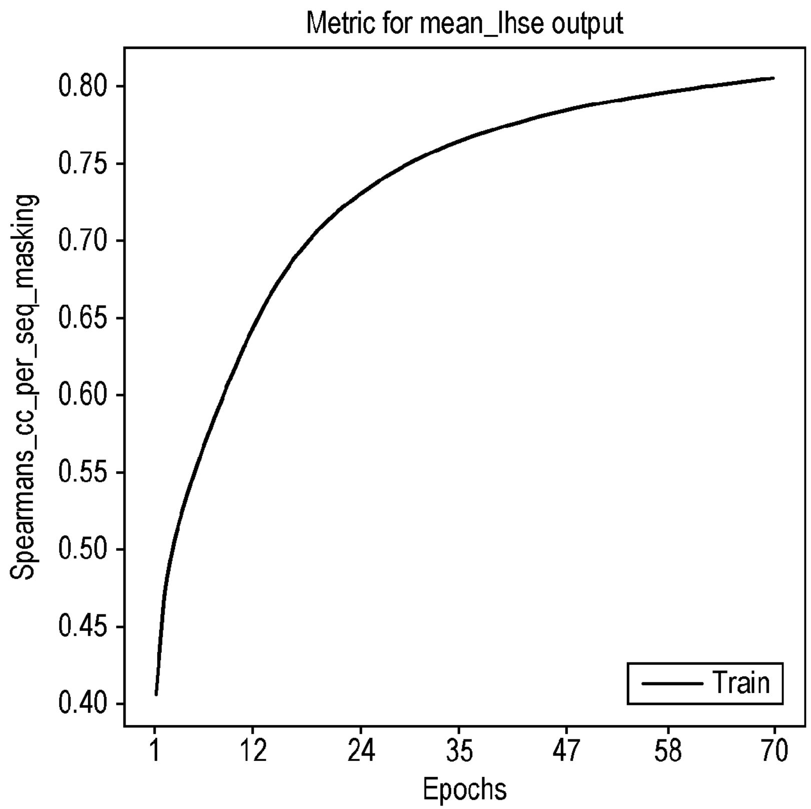


Spearman's correlation coefficient for LHSE per sequence

*FIG. 14(c)*

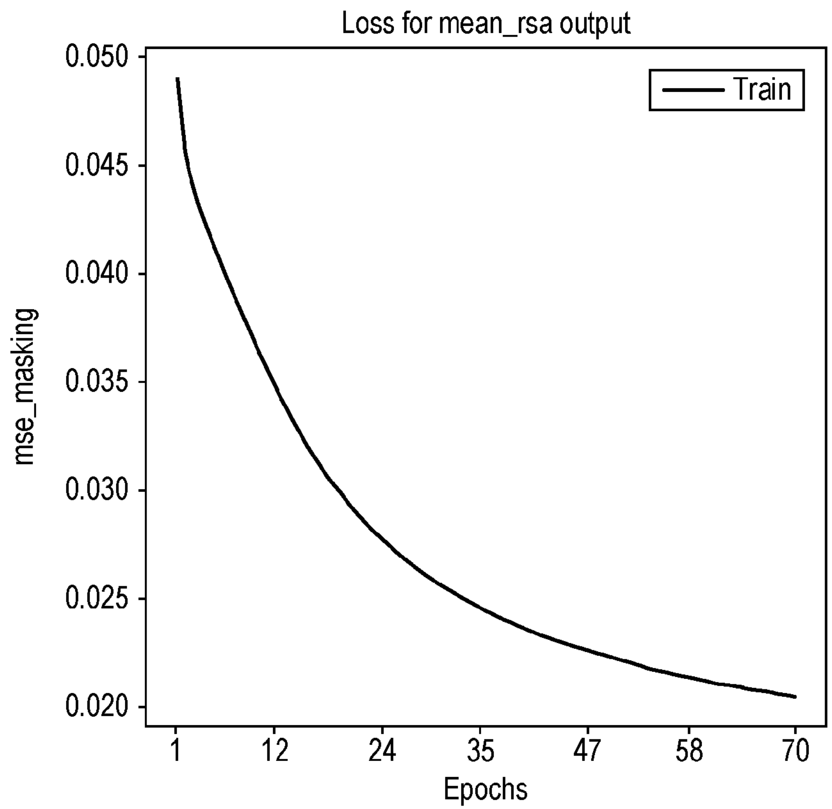


MSE loss for RSA

*FIG. 14(d)*

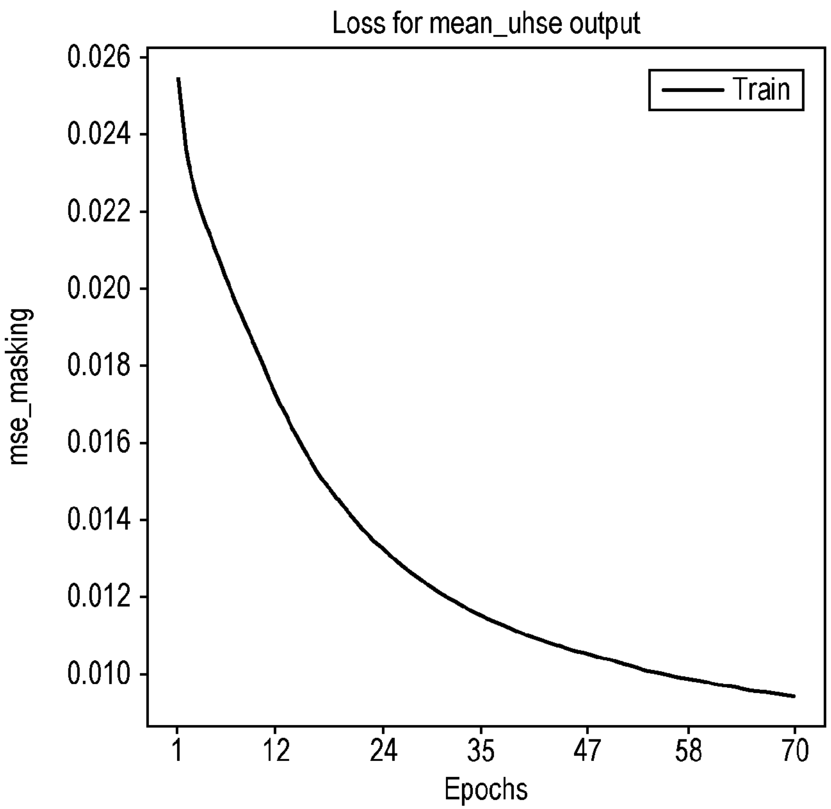


MSE loss for UHSE

FIG. 14(e)

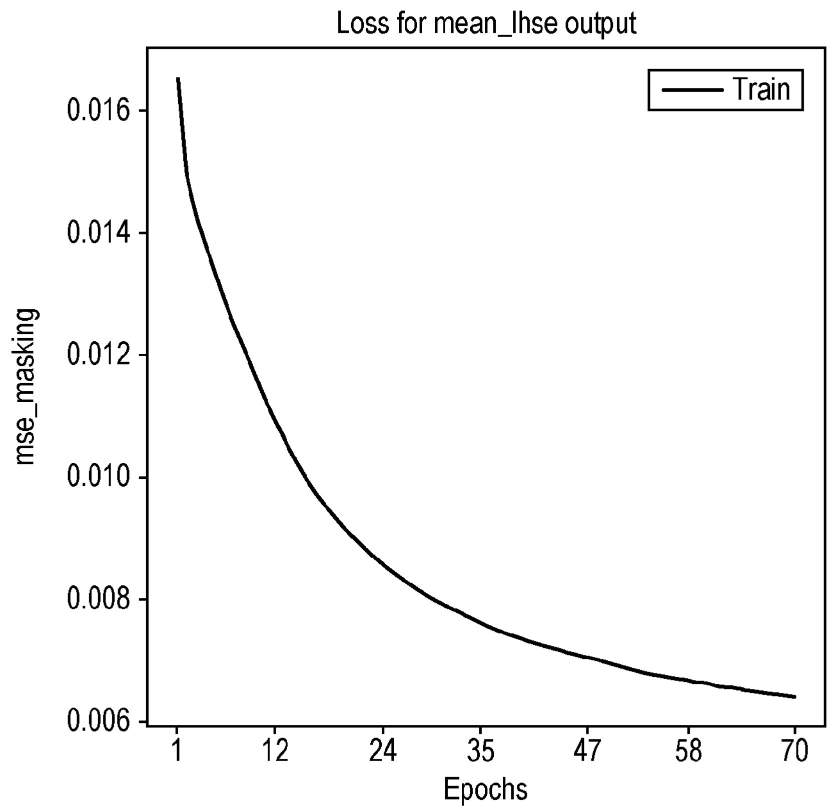


MSE loss for LHSE

FIG. 14(f)

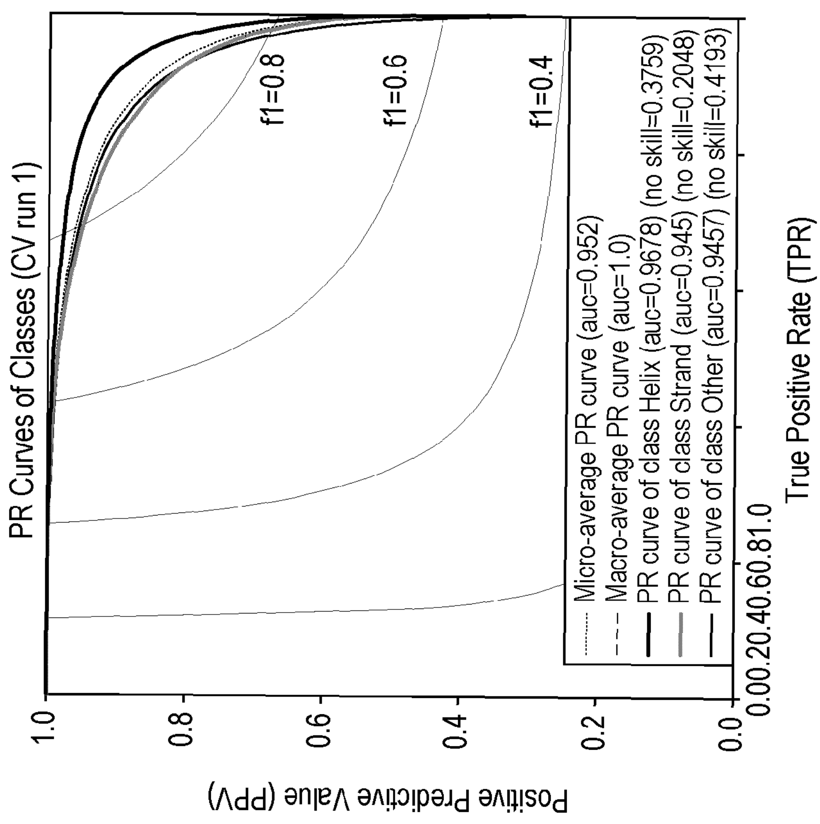


PR curve from cross-validation fold 1

FIG. 15(a)

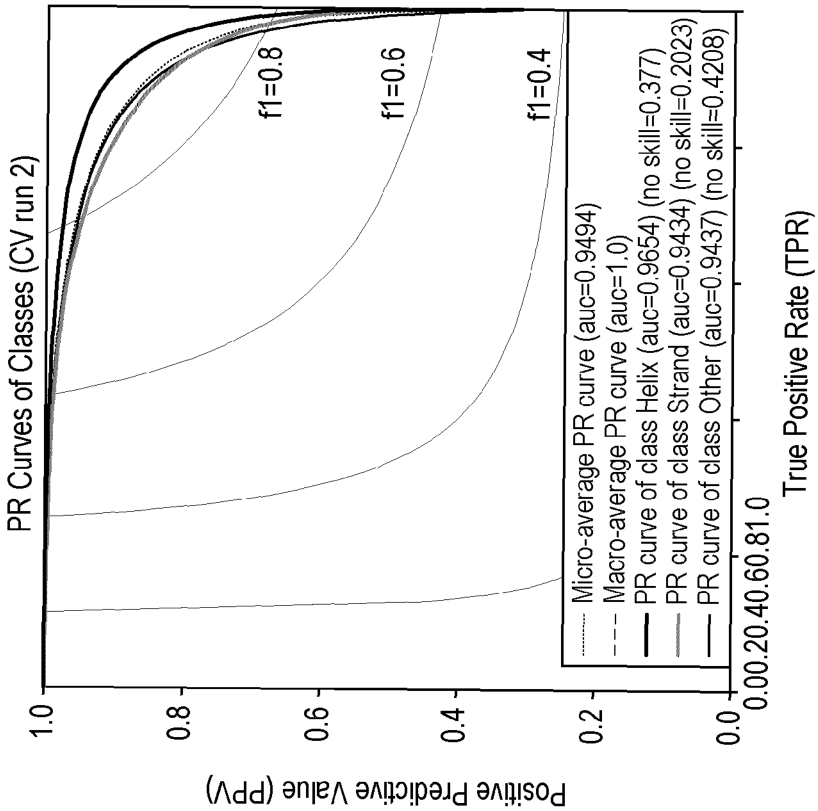


PR curve from cross-validation fold 2

FIG. 15(b)

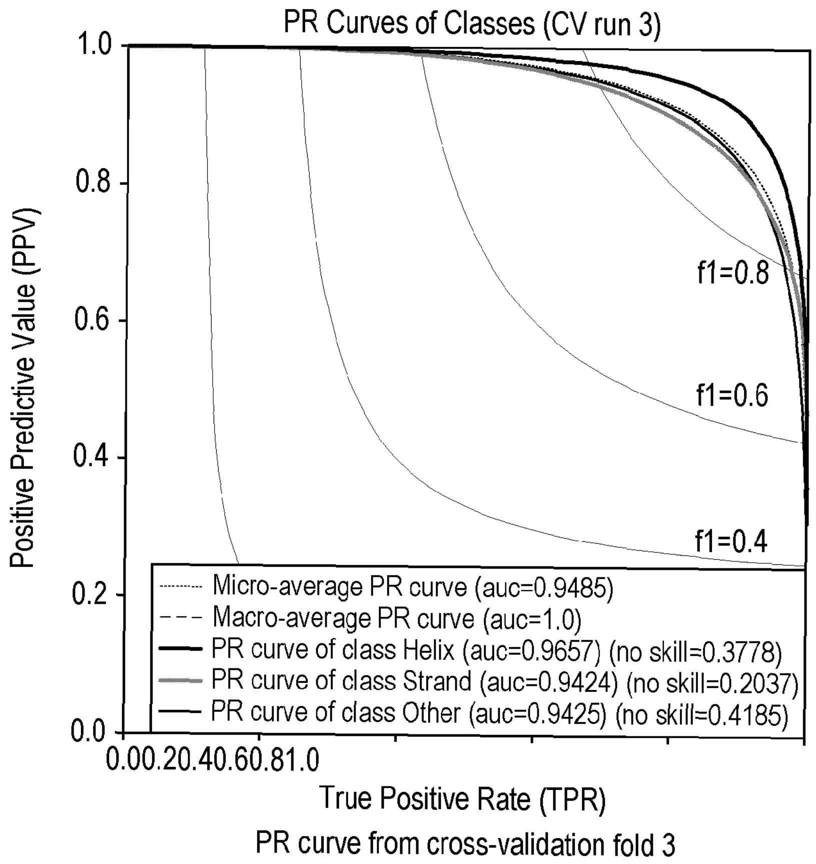


FIG. 15(c)

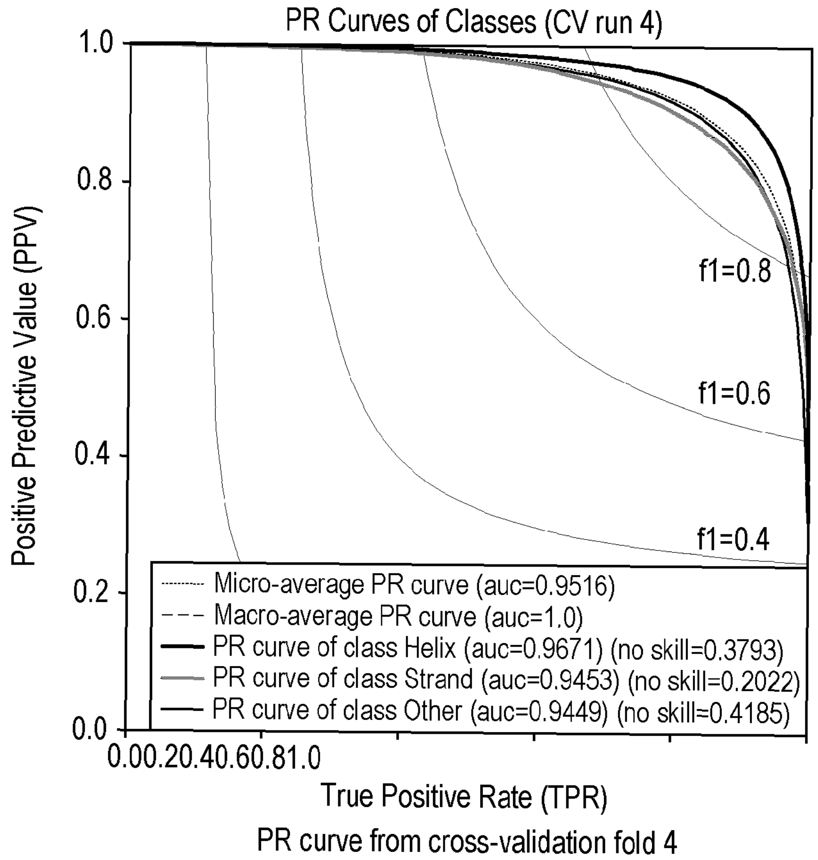


FIG. 15(d)

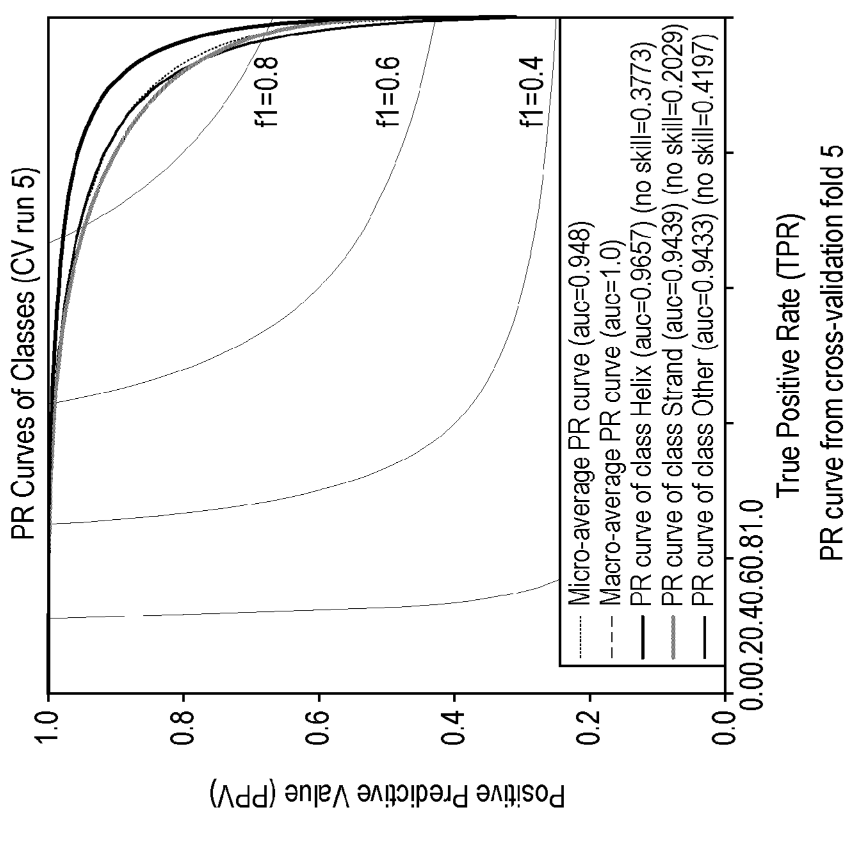


FIG. 15(e)

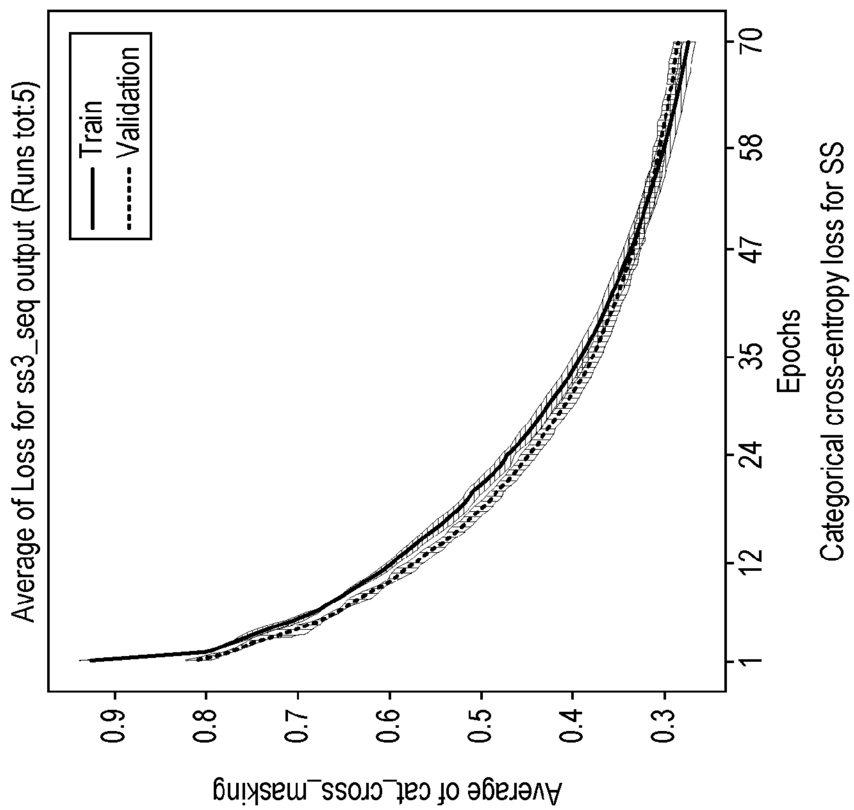


FIG. 15(f)

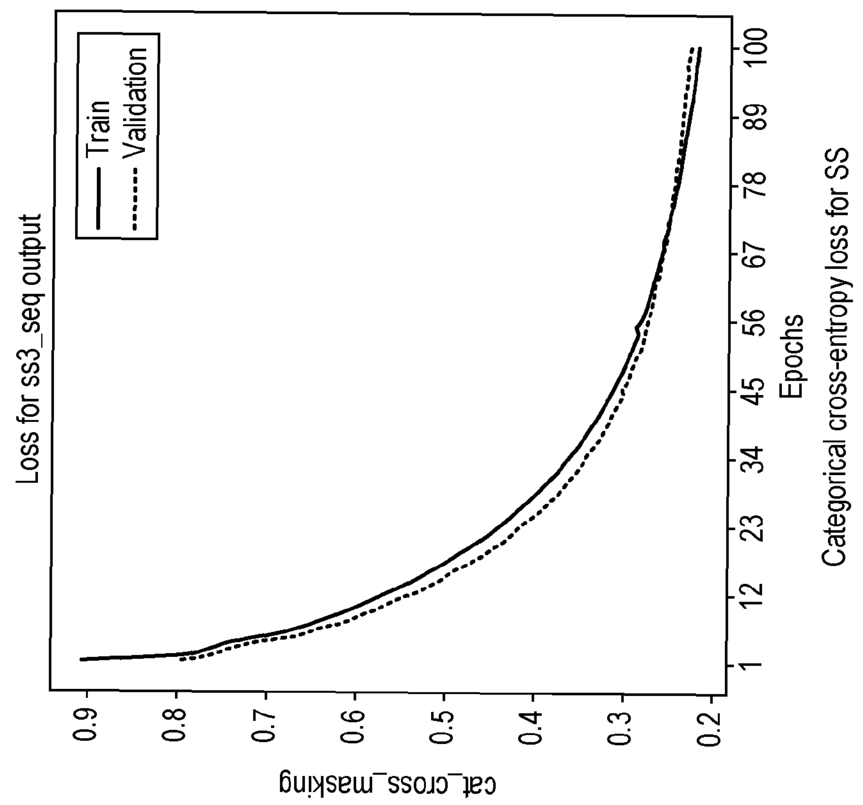


FIG. 16(b)

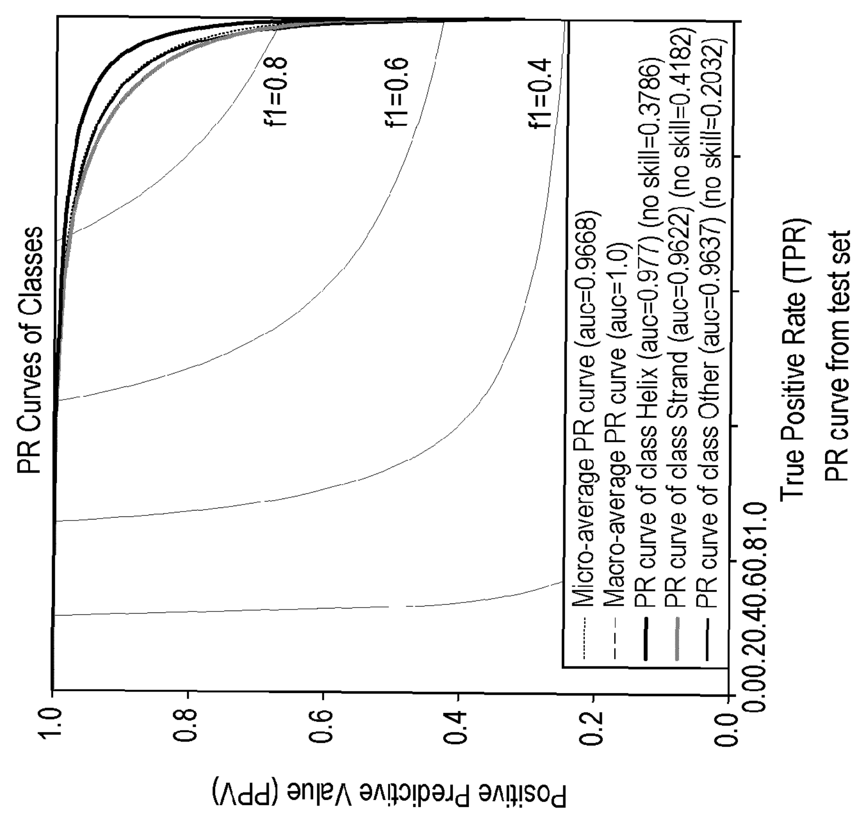


FIG. 16(a)

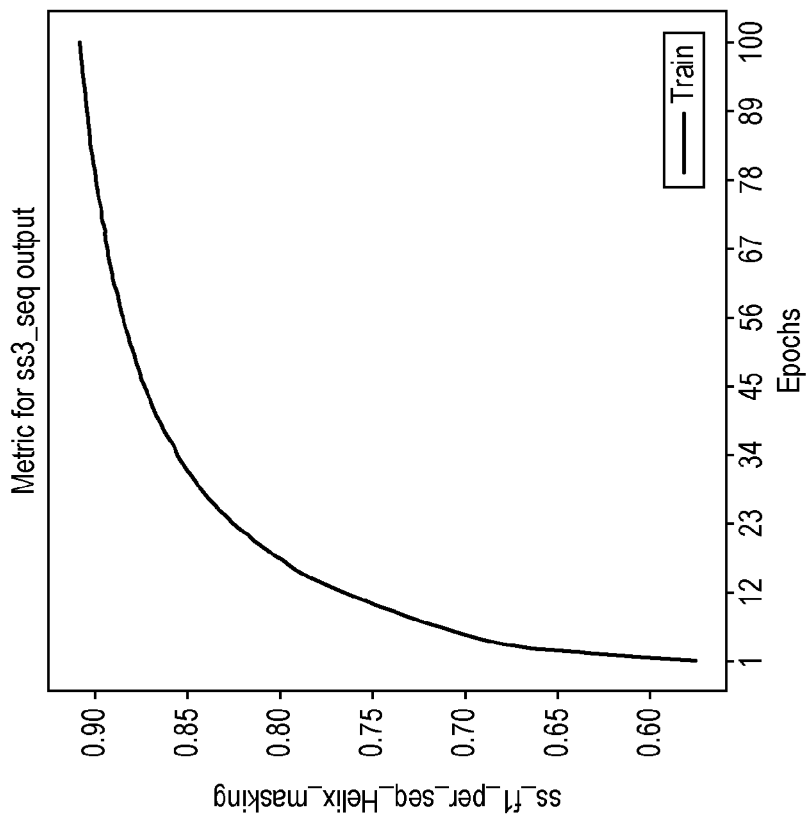


F1 curve for Helix for final estimates

FIG. 17(a)

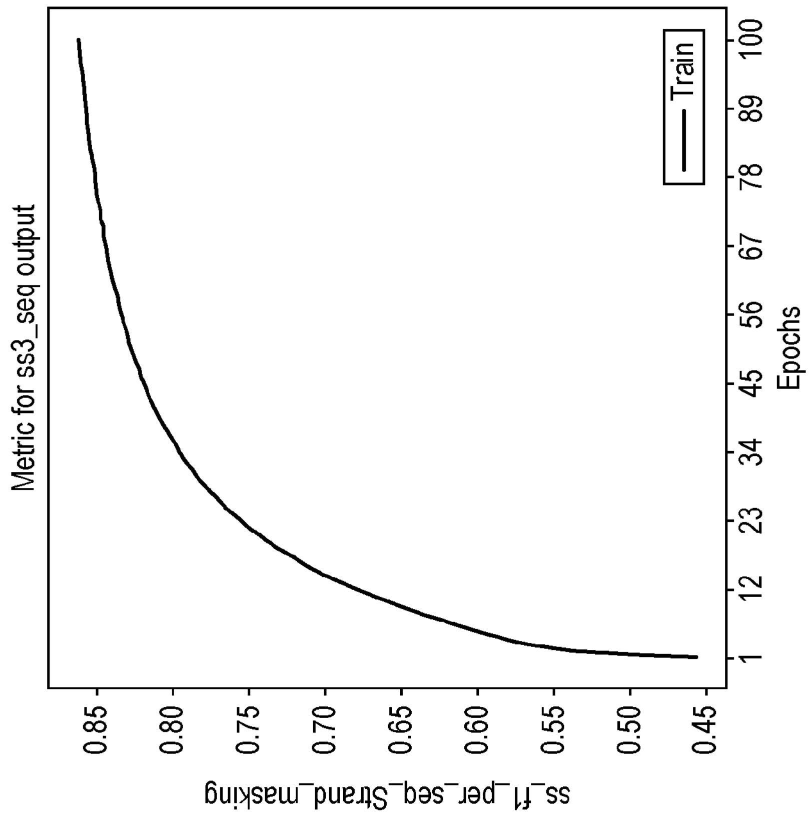


F1 curve for Strand for final estimates

FIG. 17(b)

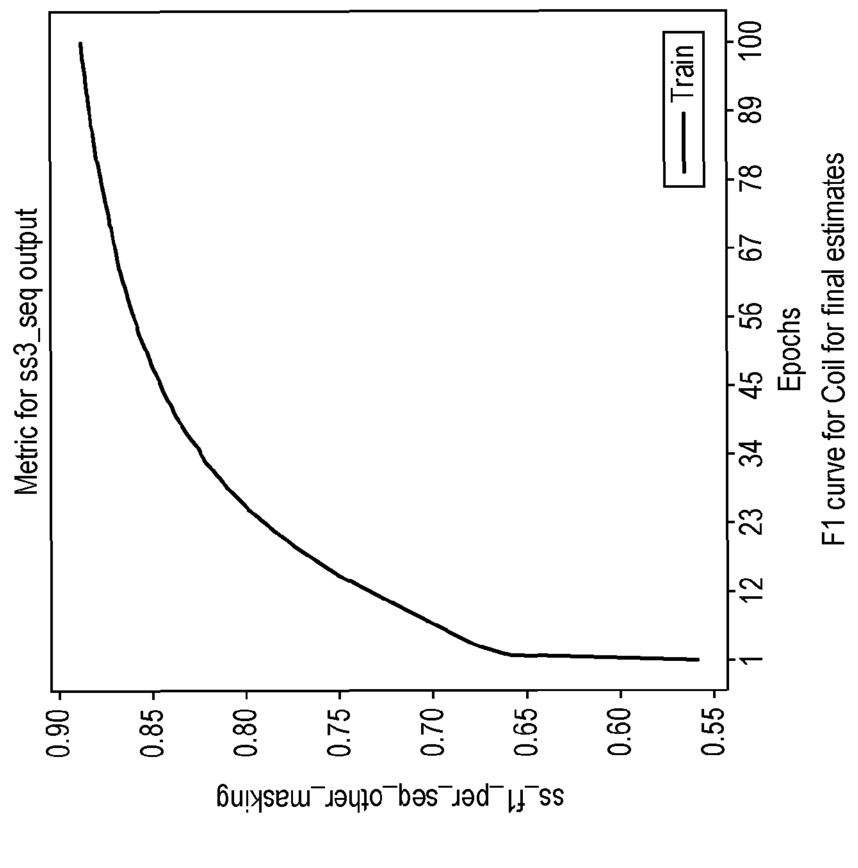


FIG. 17(c)

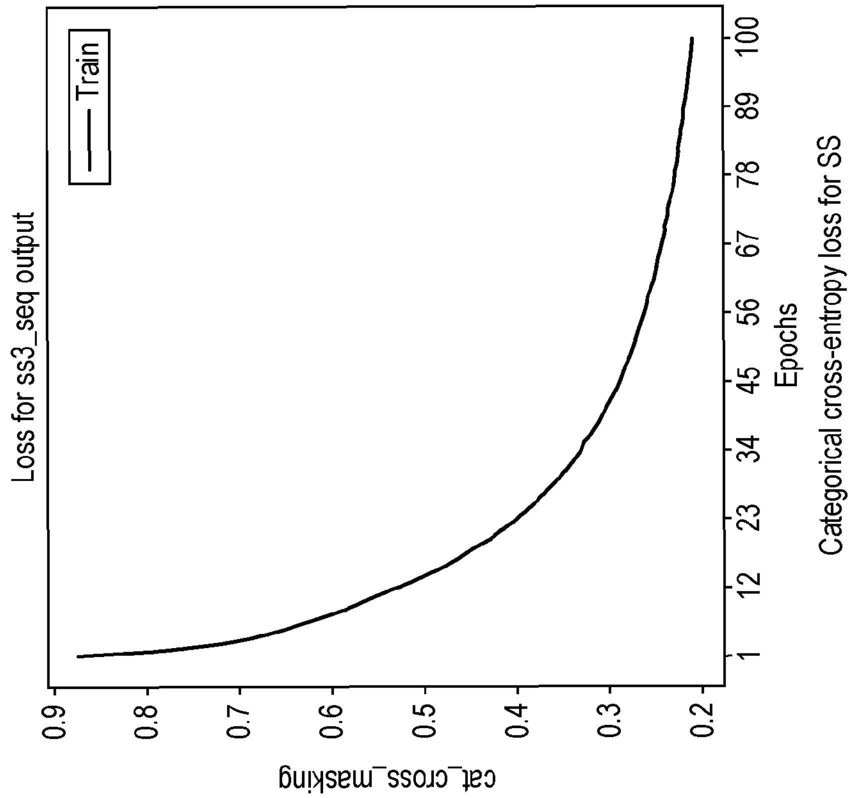


FIG. 17(d)

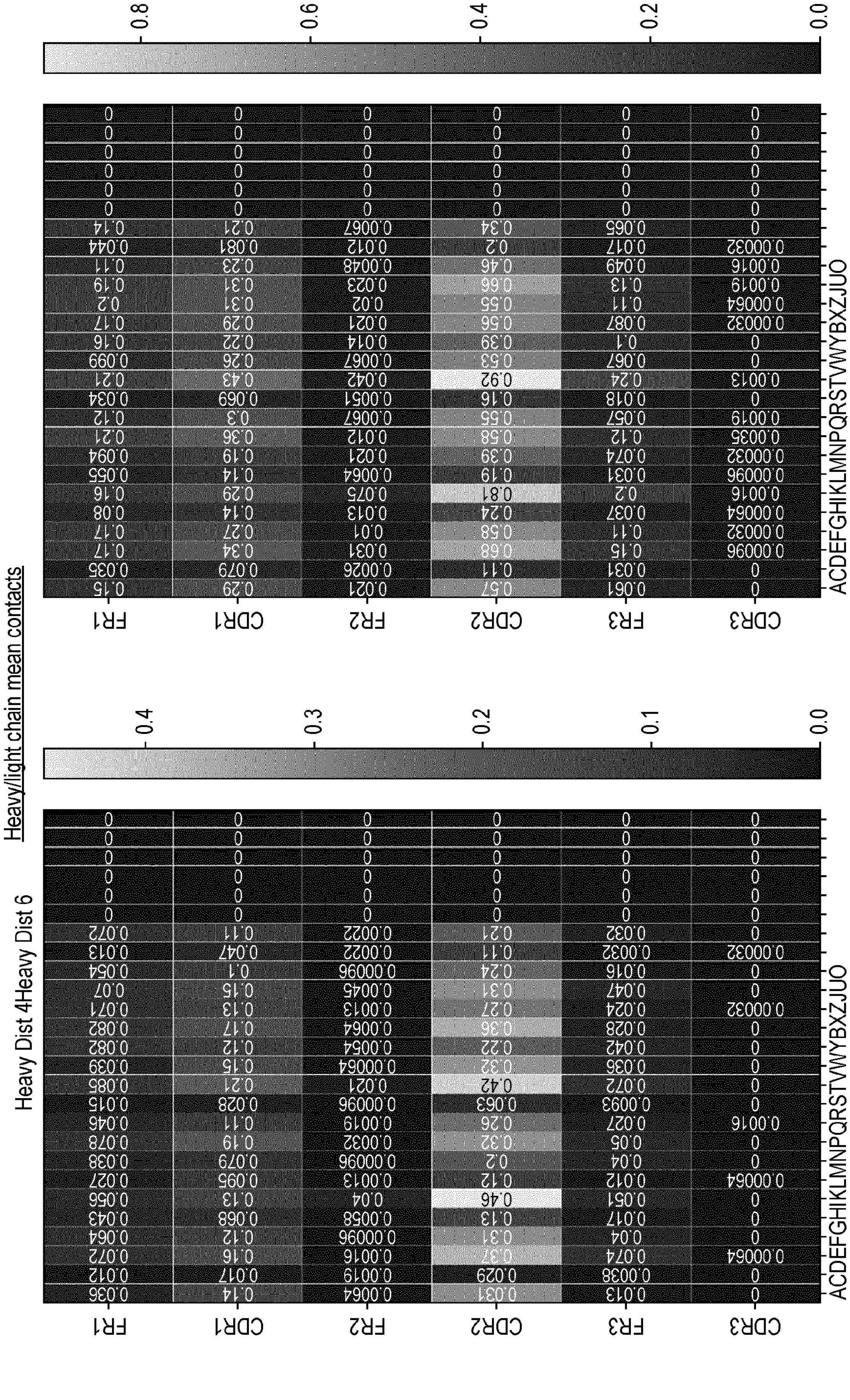
Heavy/light chain mean contacts
Heavy Dist 4Heavy Dist 6
FR1
CDR1
FR2
CDR2
FR3
CDR3
ACDEFGHIKLMNPQRSTVWYBXZJUO
0.0
0.1
0.2
0.3
0.4
0.0
0.2
0.4
0.6
0.8

FIG. 18(a)

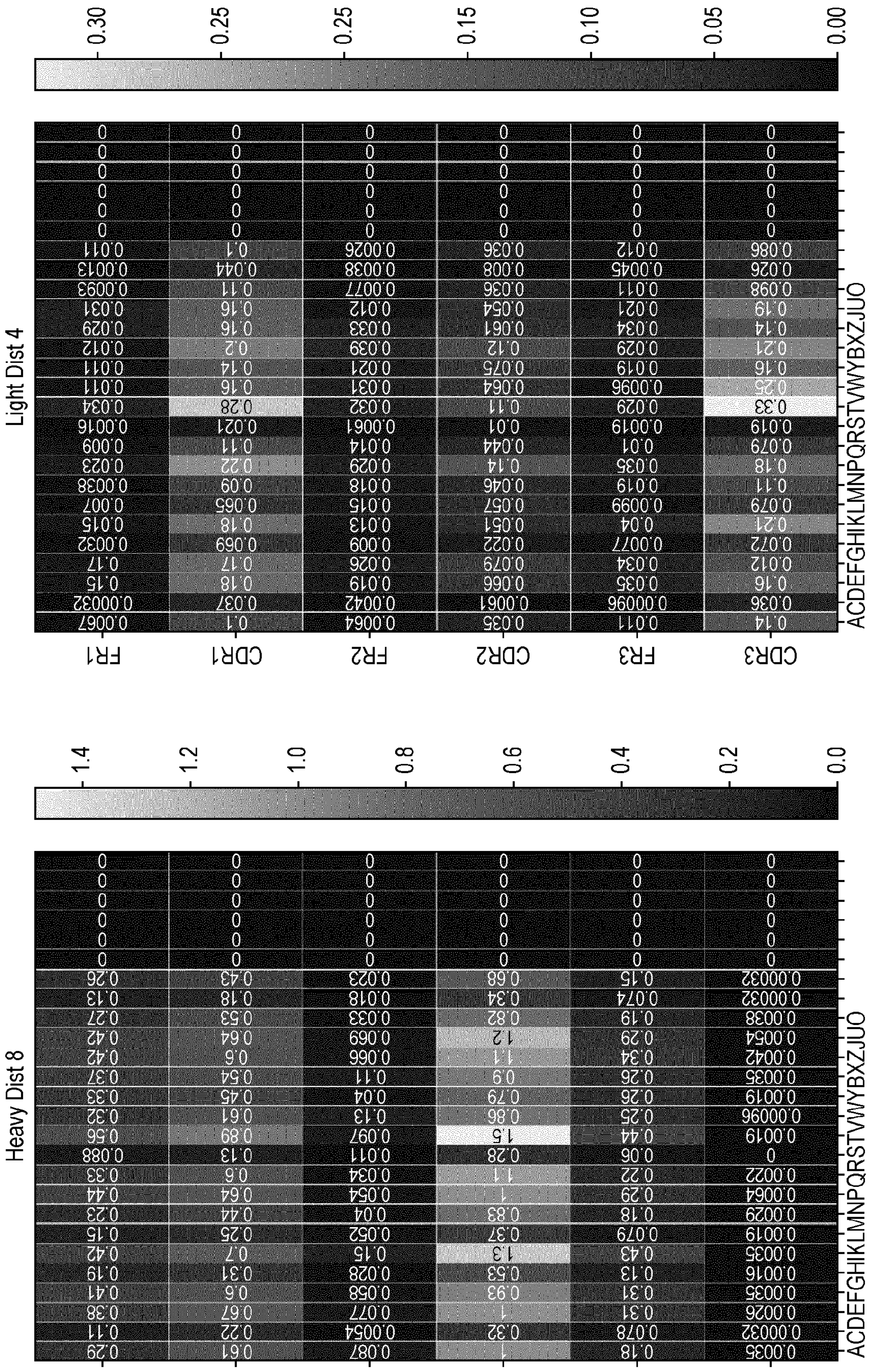


FIG. 18(a) Cont'd

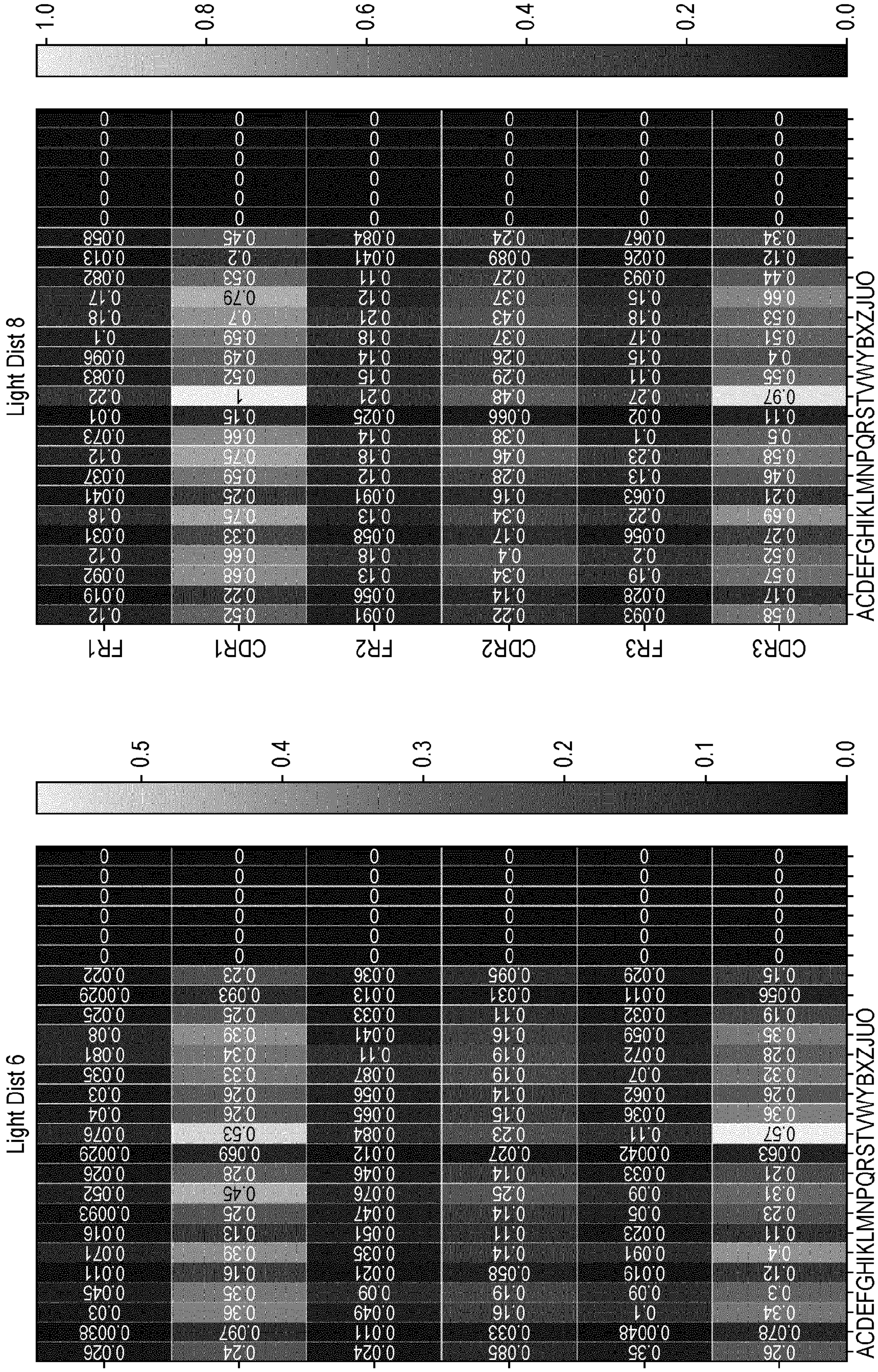


*FIG. 18(a) Cont'd*

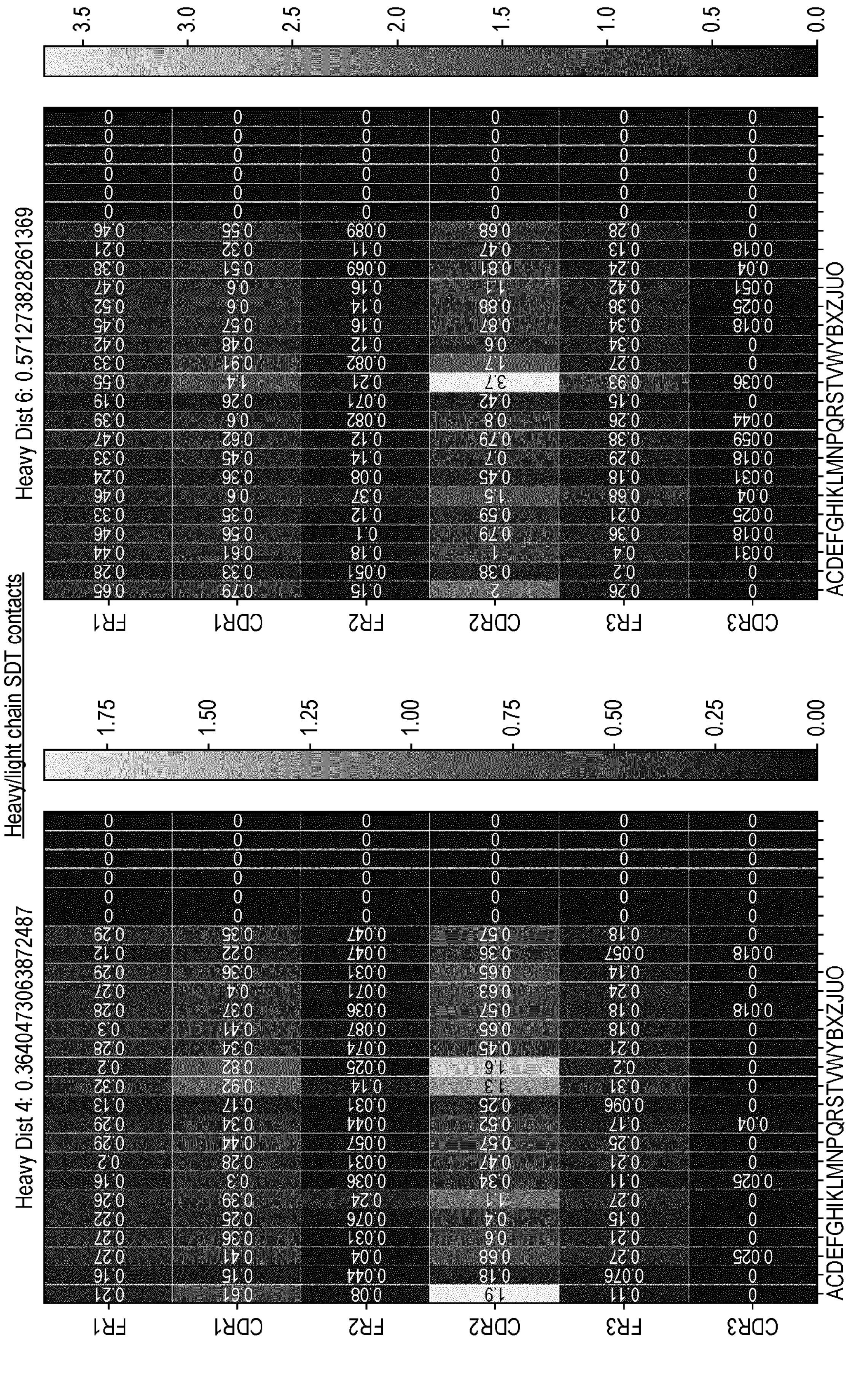
Heavy/light chain SDT contacts
Heavy Dist 4: 0.3640473063872487
Heavy Dist 6: 0.571273828261369
FR1
CDR1
FR2
CDR2
FR3
CDR3
ACDEFGHIKLMNPQRSTVWYBXZJUO

FIG. 18(b)

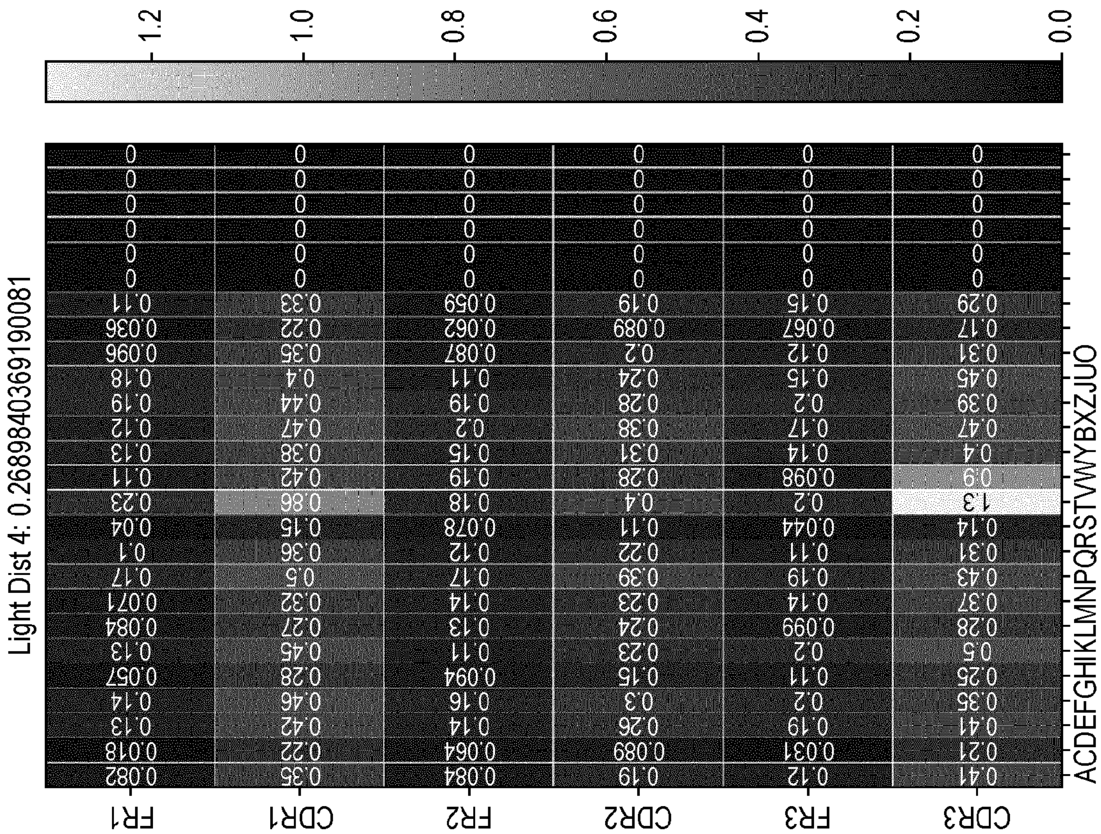


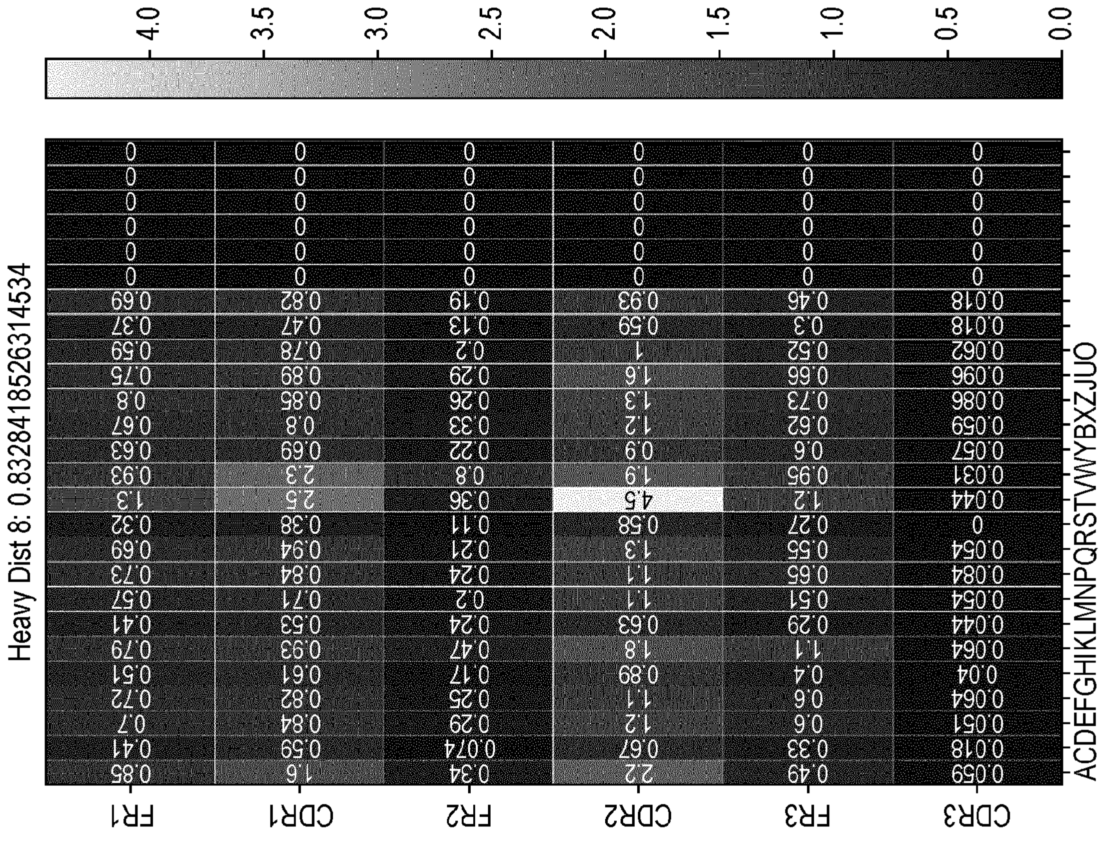


FIG. 18(b) Cont'd

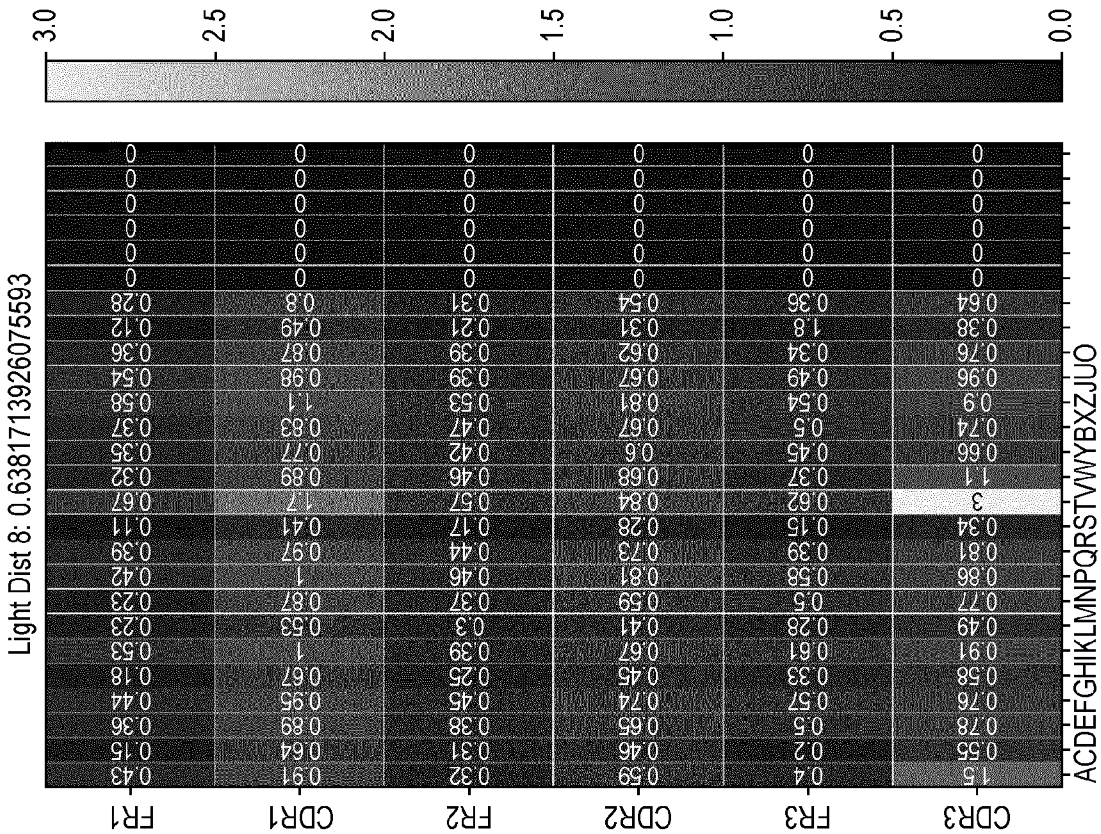


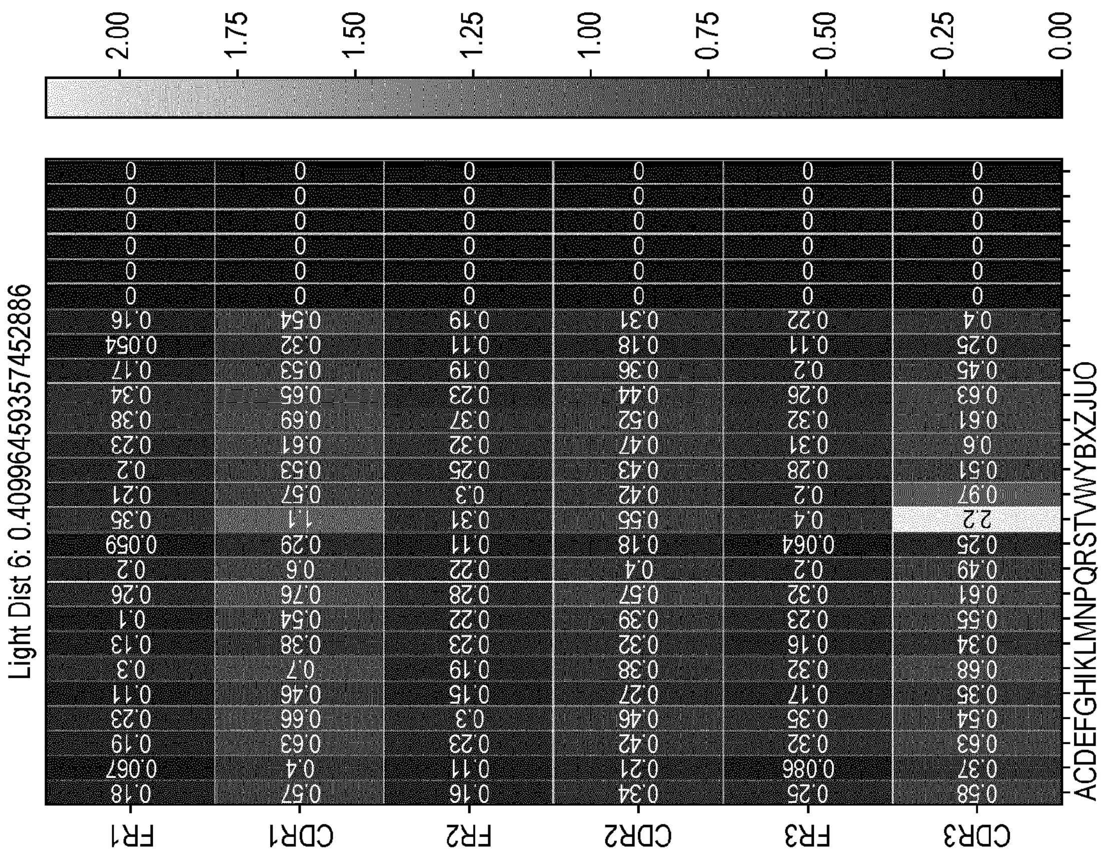


FIG. 18(b) Cont'd

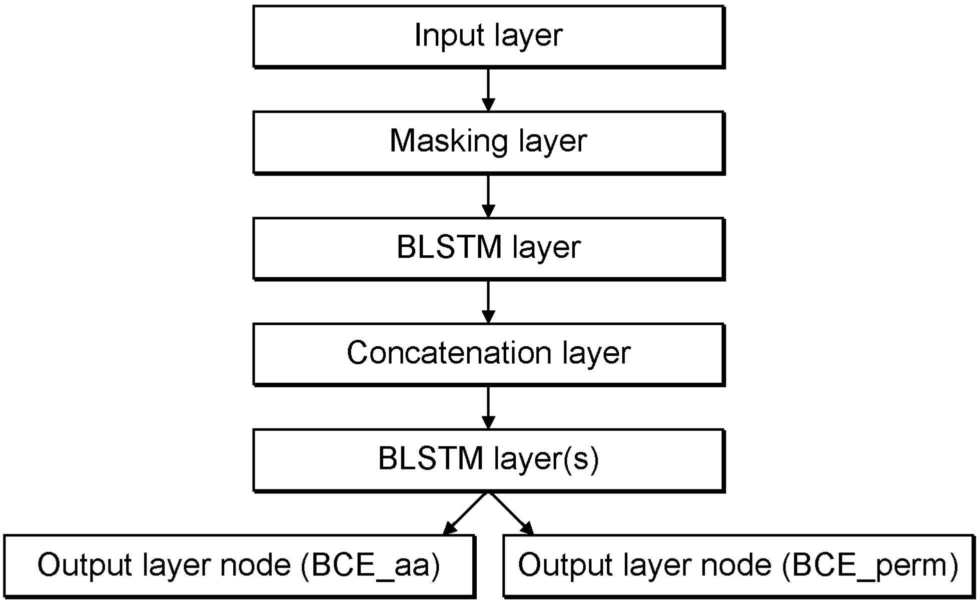


FIG. 19(a)

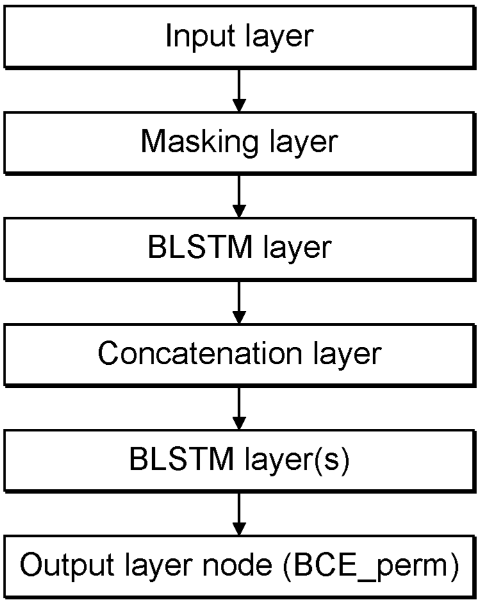


FIG. 19(b)

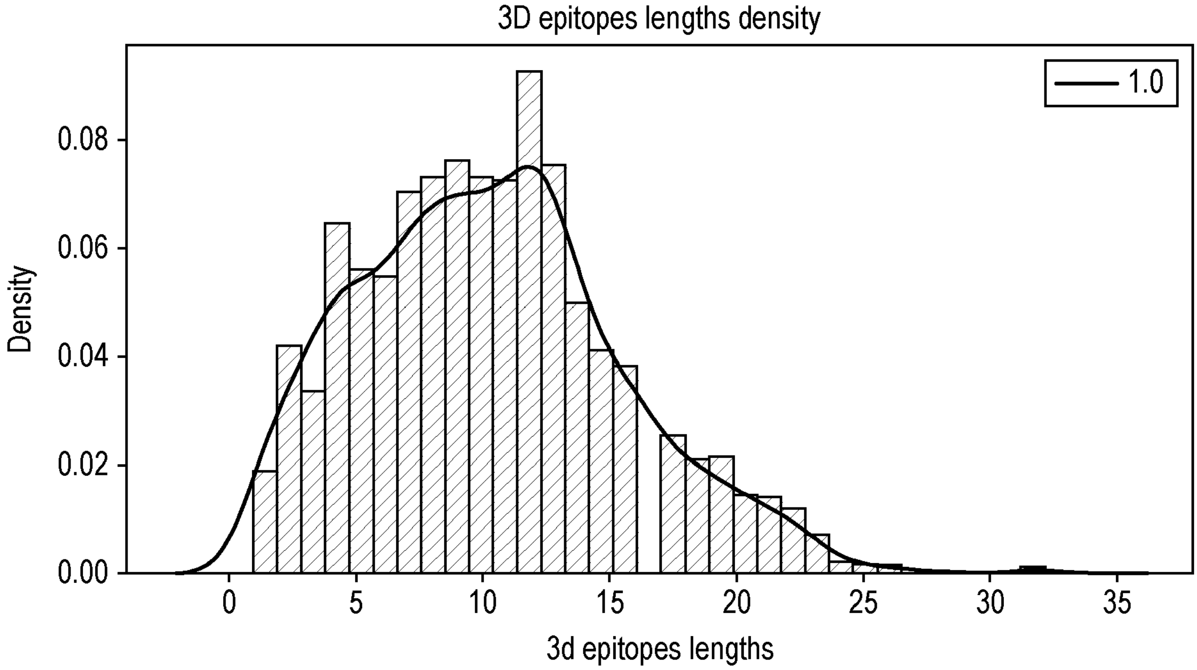


FIG. 20(a)

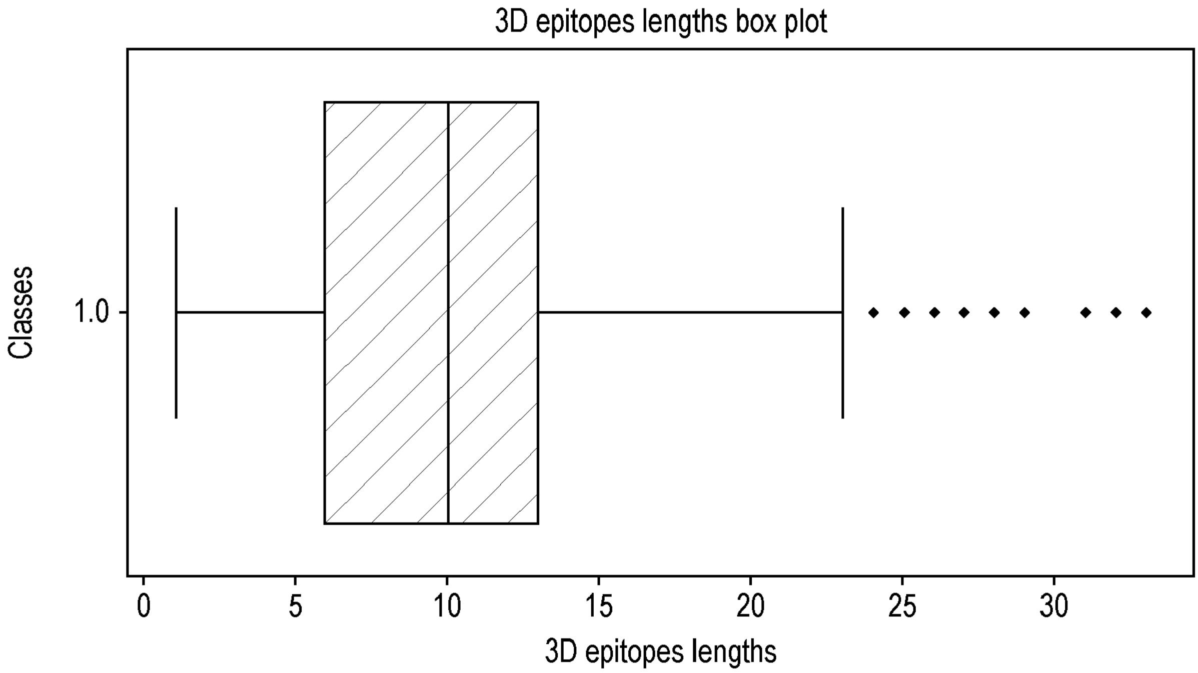


FIG. 20(b)

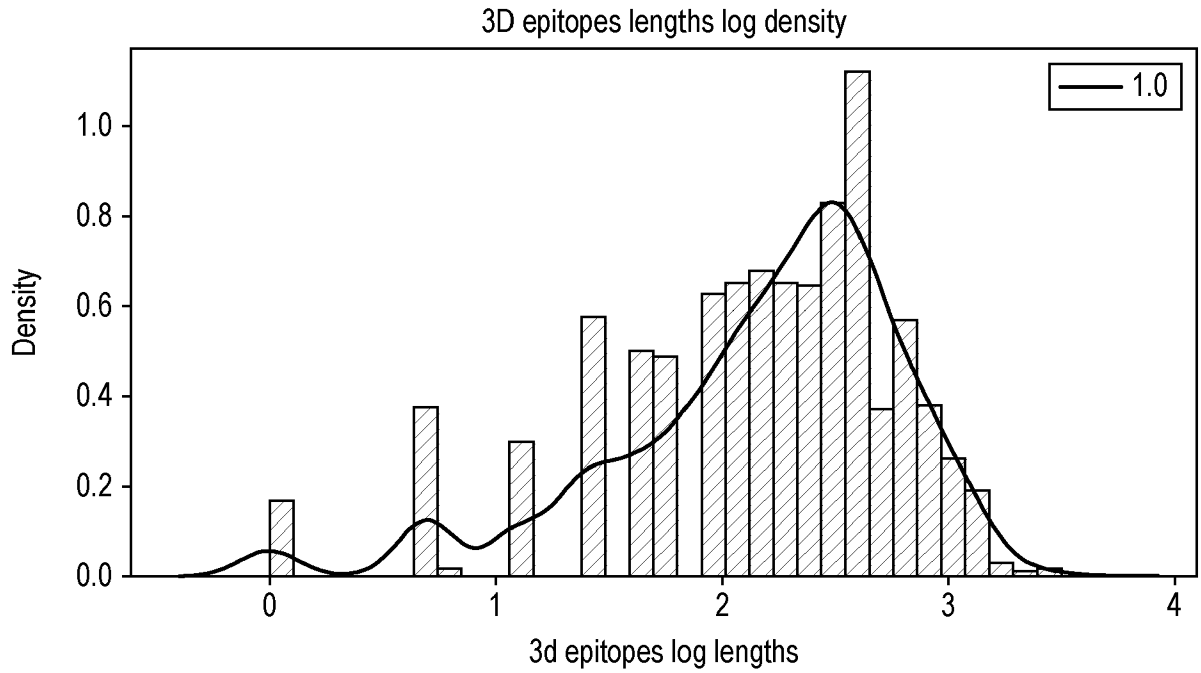


FIG. 20(c)

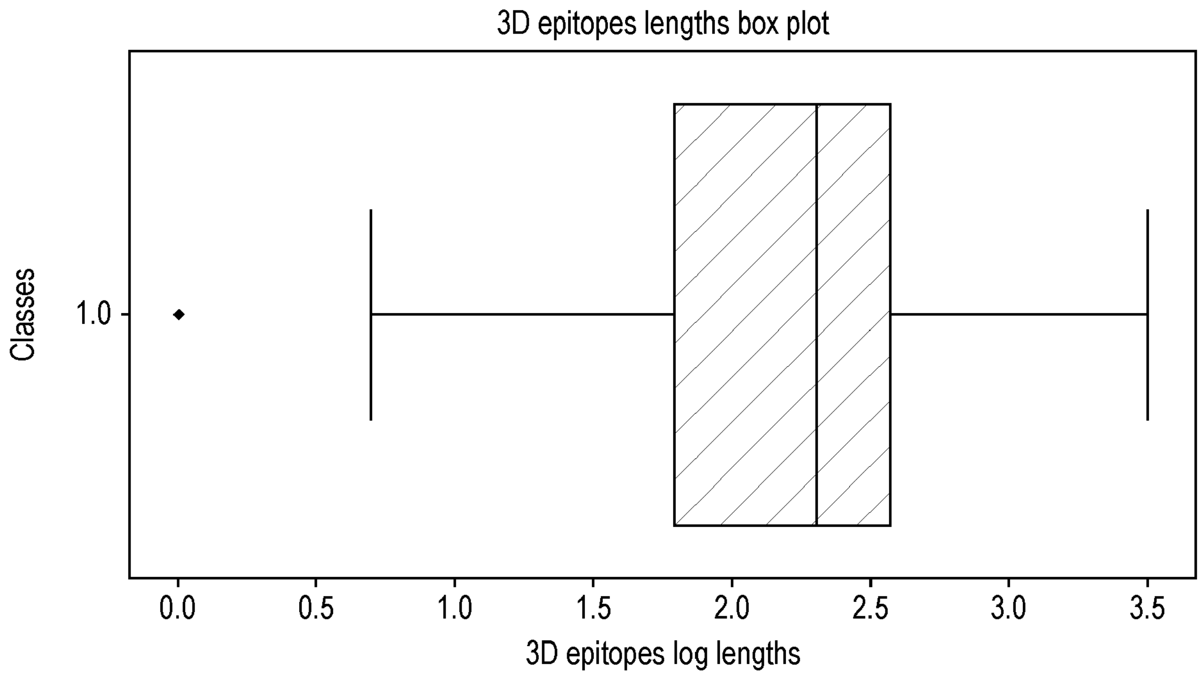


FIG. 20(d)

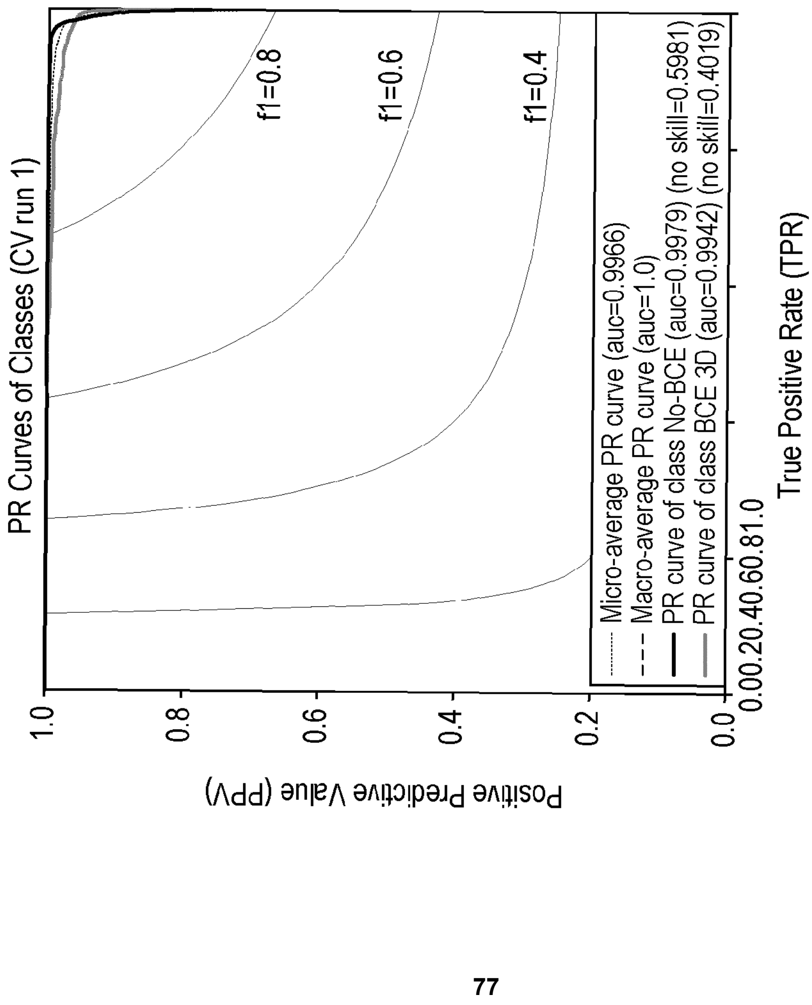


PR curve from cross-validation fold 1

FIG. 21(a)

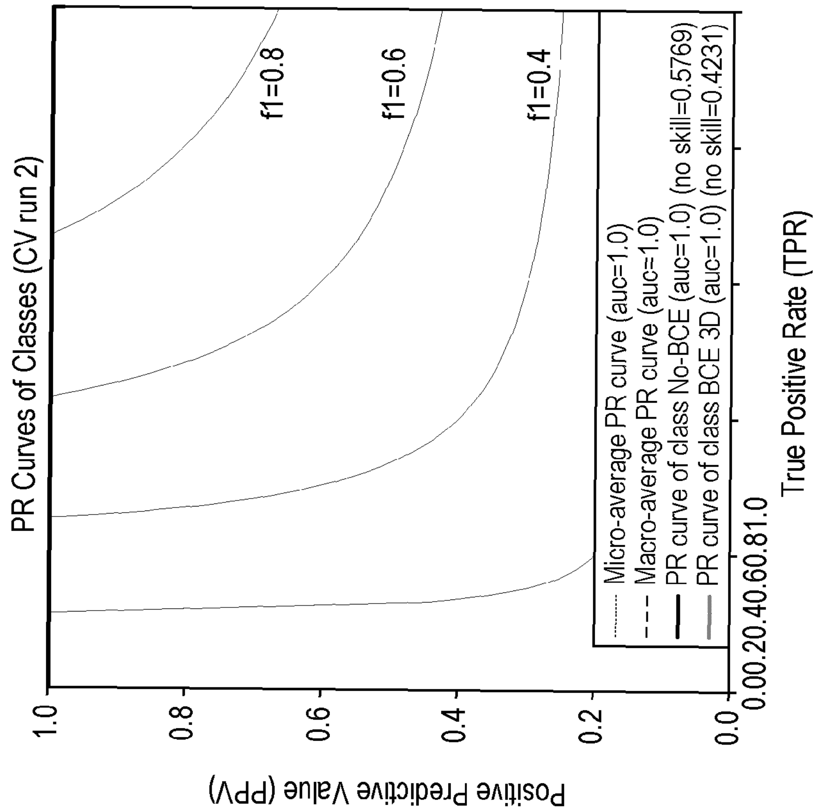


PR curve from cross-validation fold 2

FIG. 21(b)

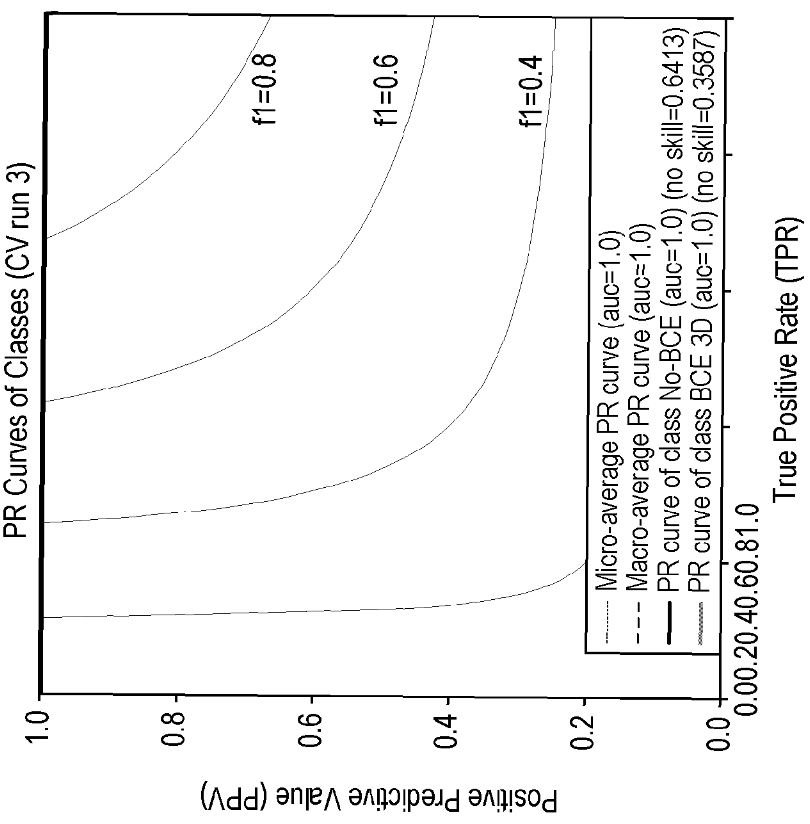


PR curve from cross-validation fold 3

*FIG. 21(c)*

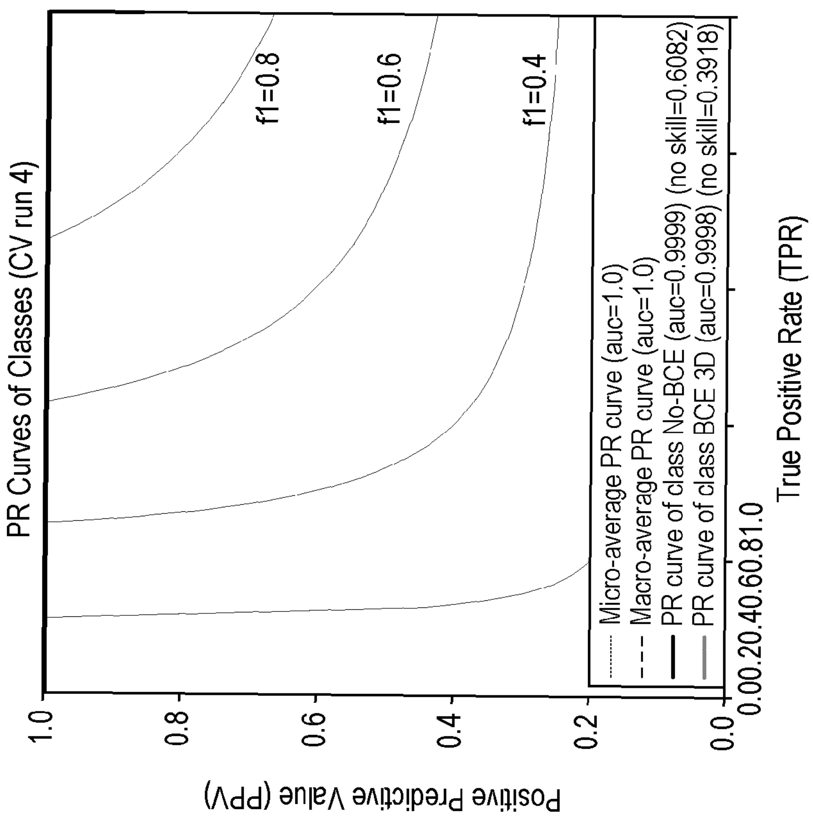


PR curve from cross-validation fold 4

*FIG. 21(d)*

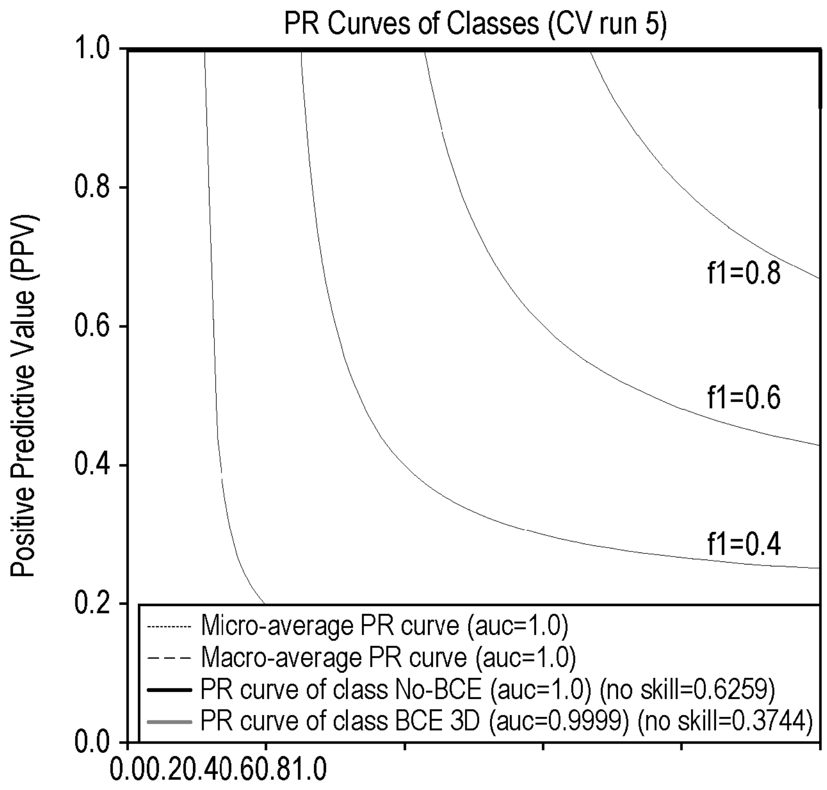


PR curve from cross-validation fold 5

FIG. 21(e)

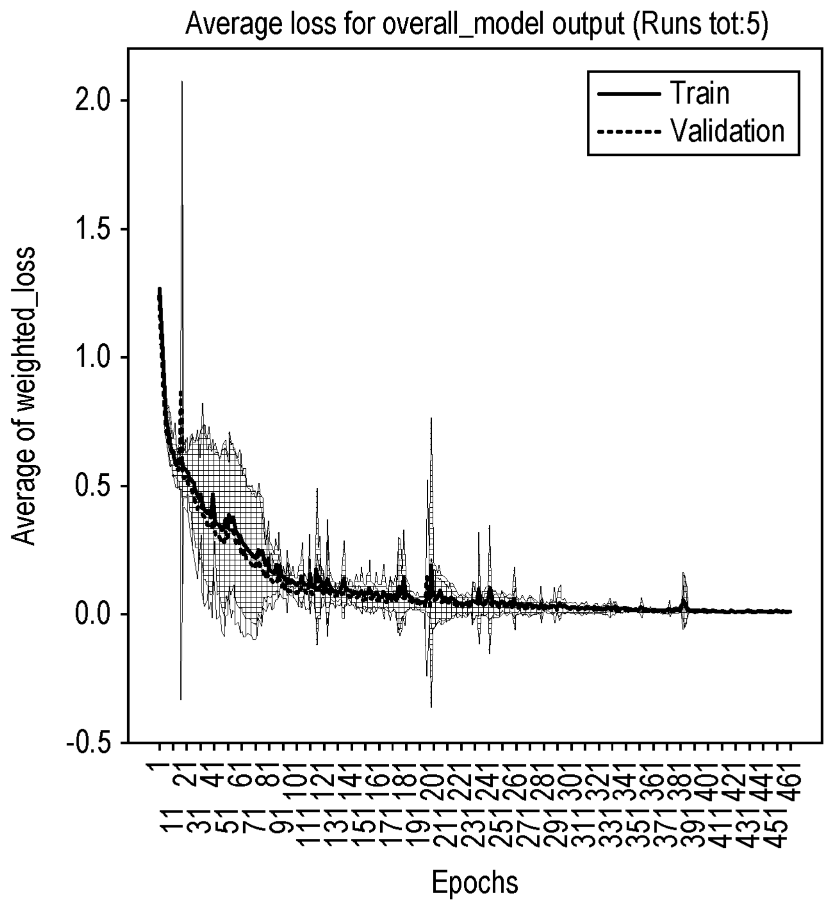


Overall model loss from all CV folds

FIG. 21(f)

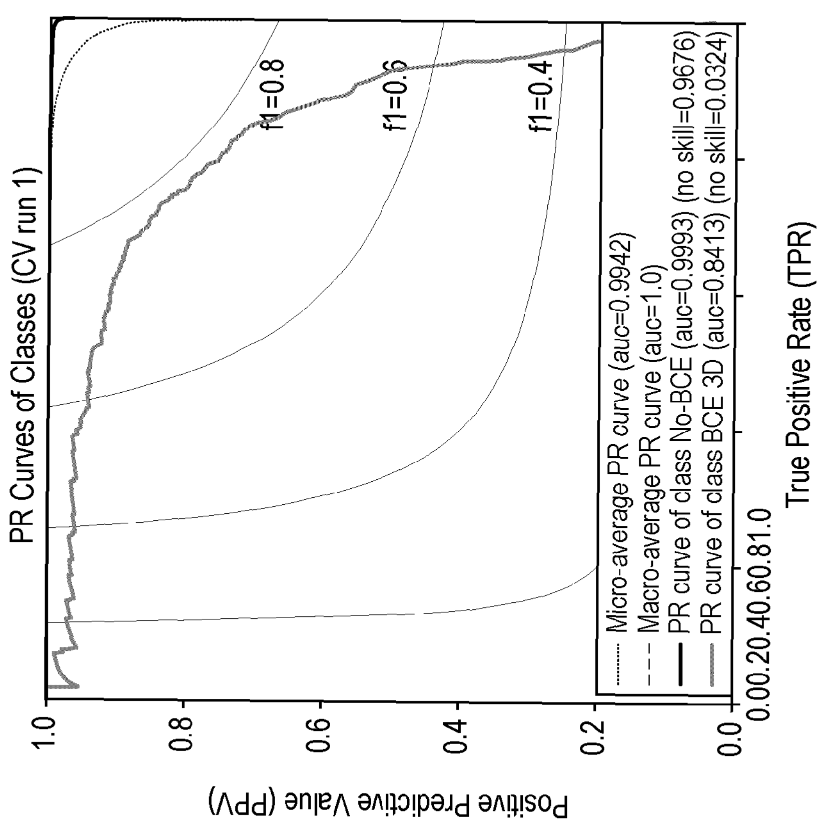


FIG. 22(a)

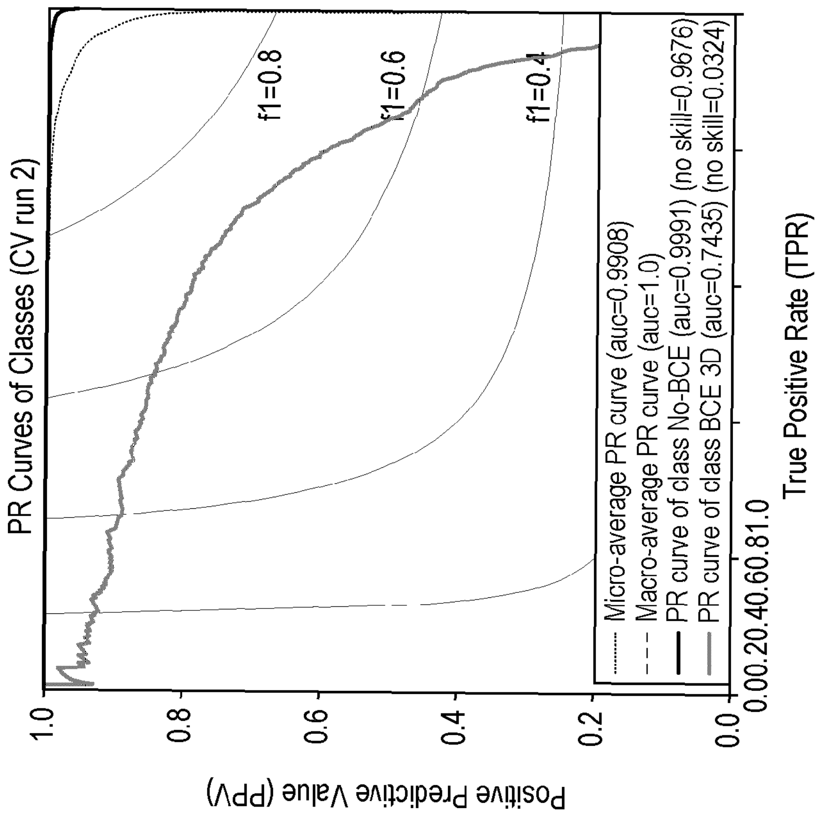


FIG. 22(b)

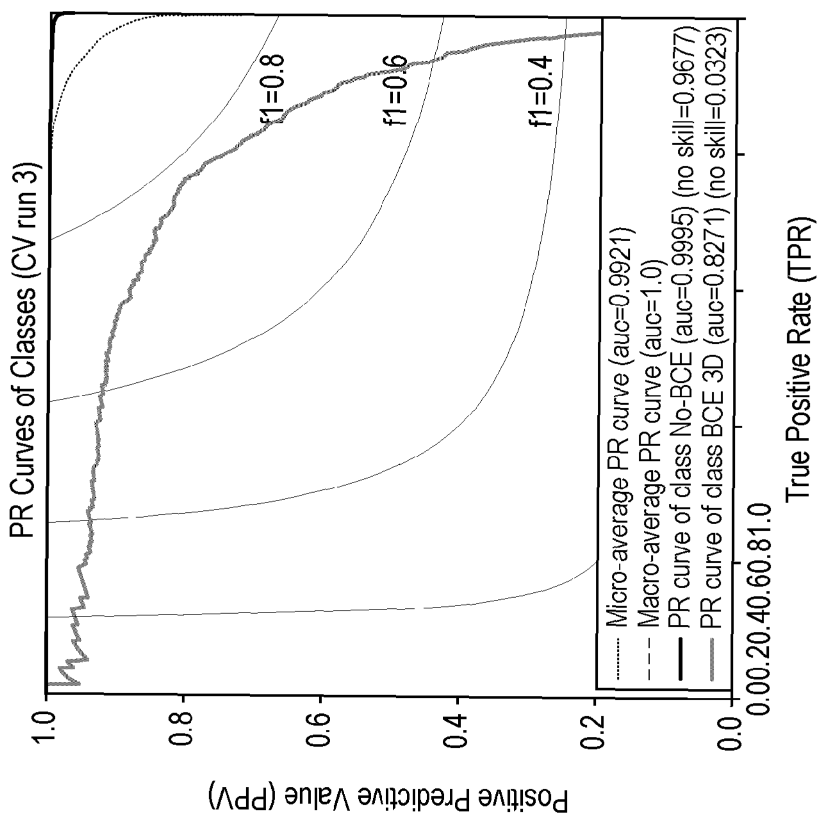


FIG. 22(c)

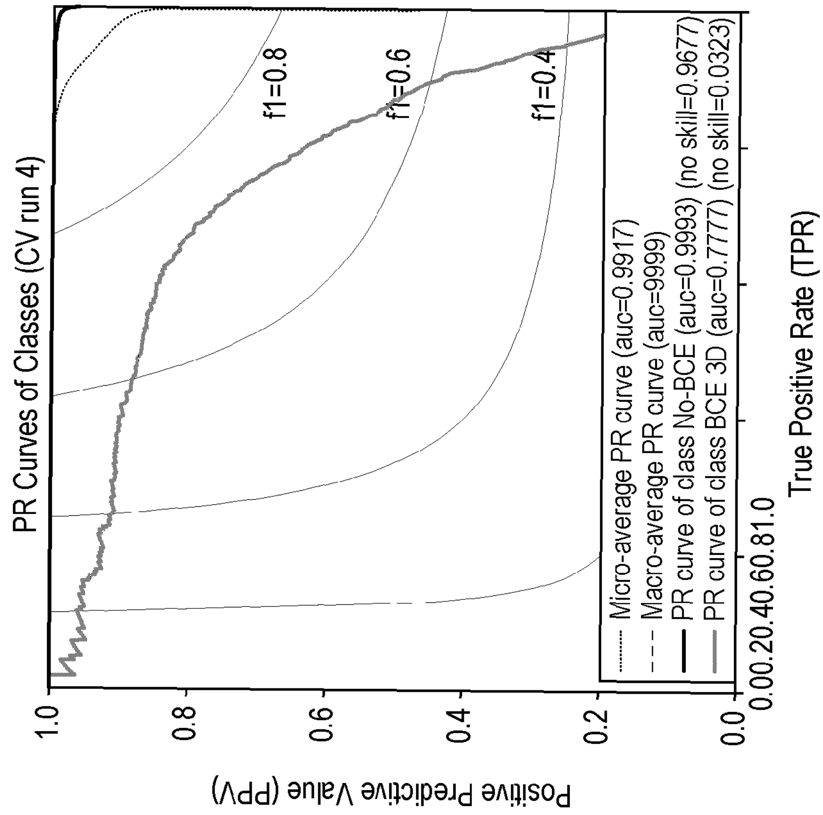


FIG. 22(d)

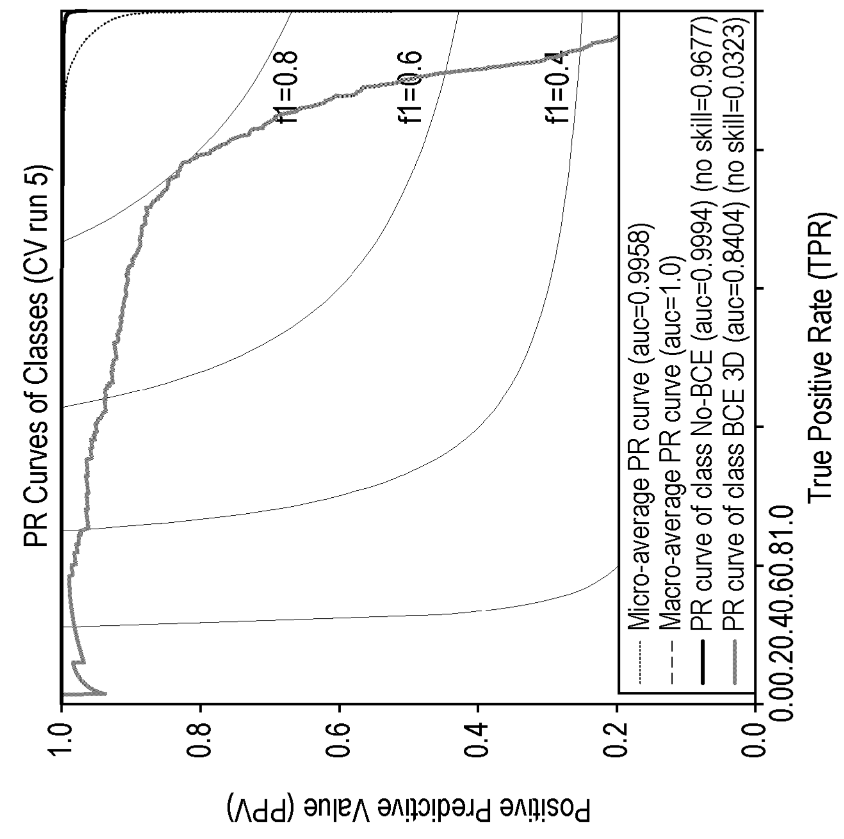


FIG. 22(e)

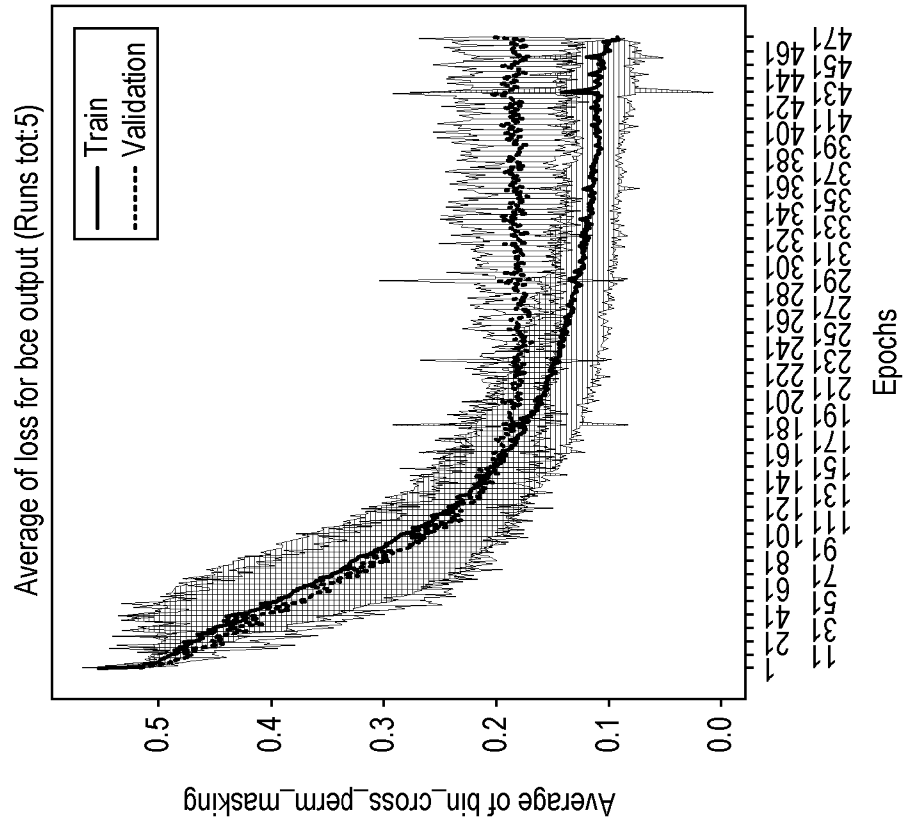


FIG. 22(f)

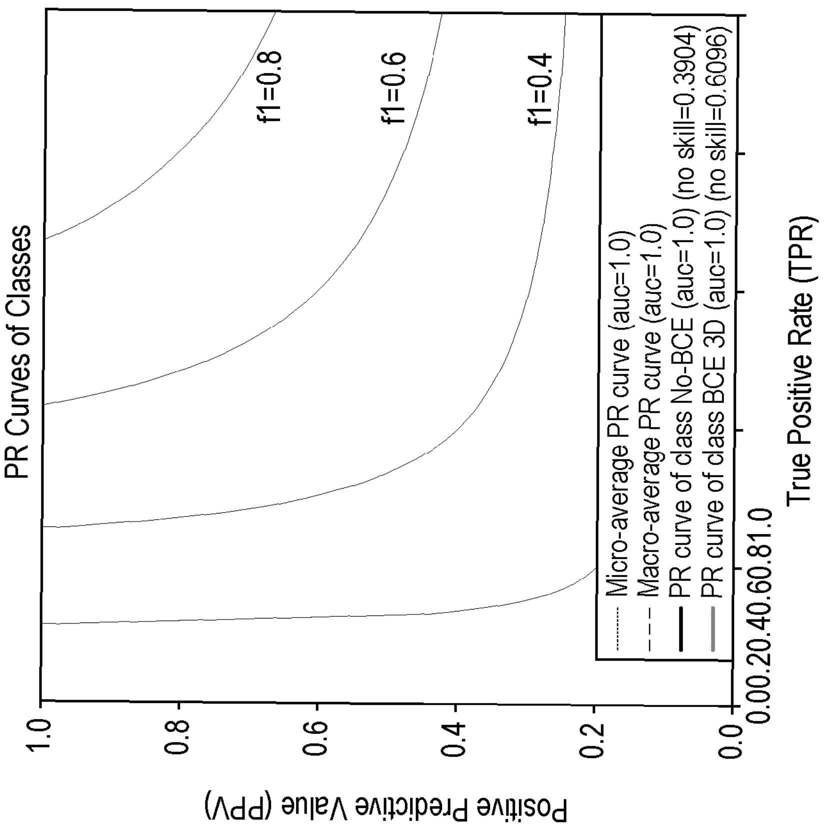


FIG. 23(a)

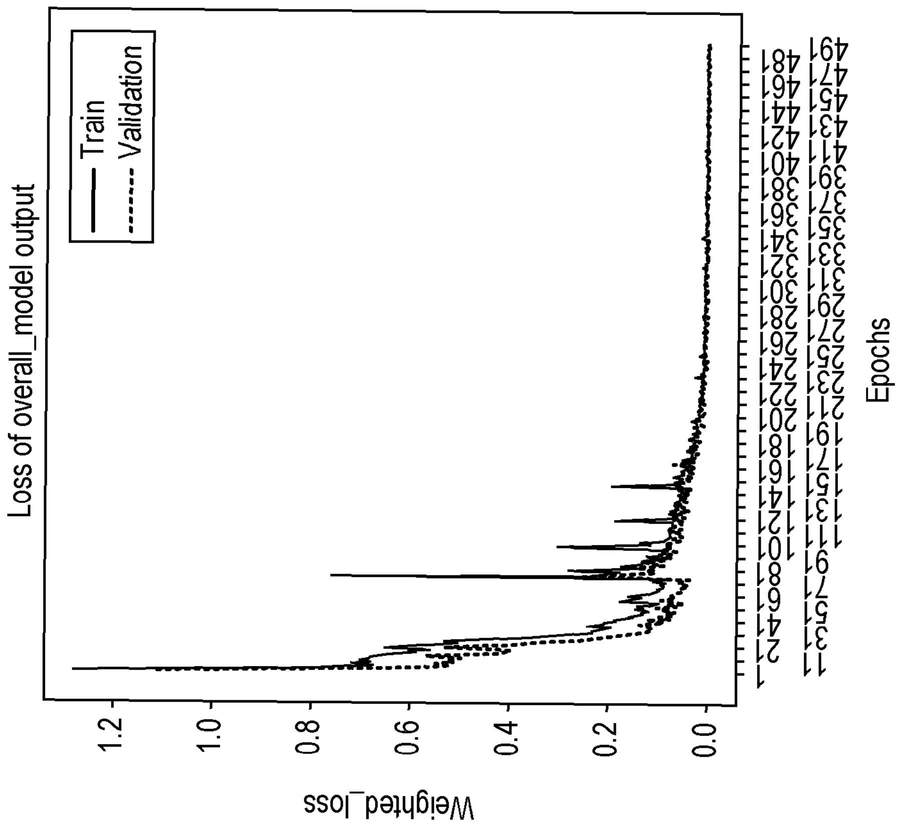


FIG. 23(b)

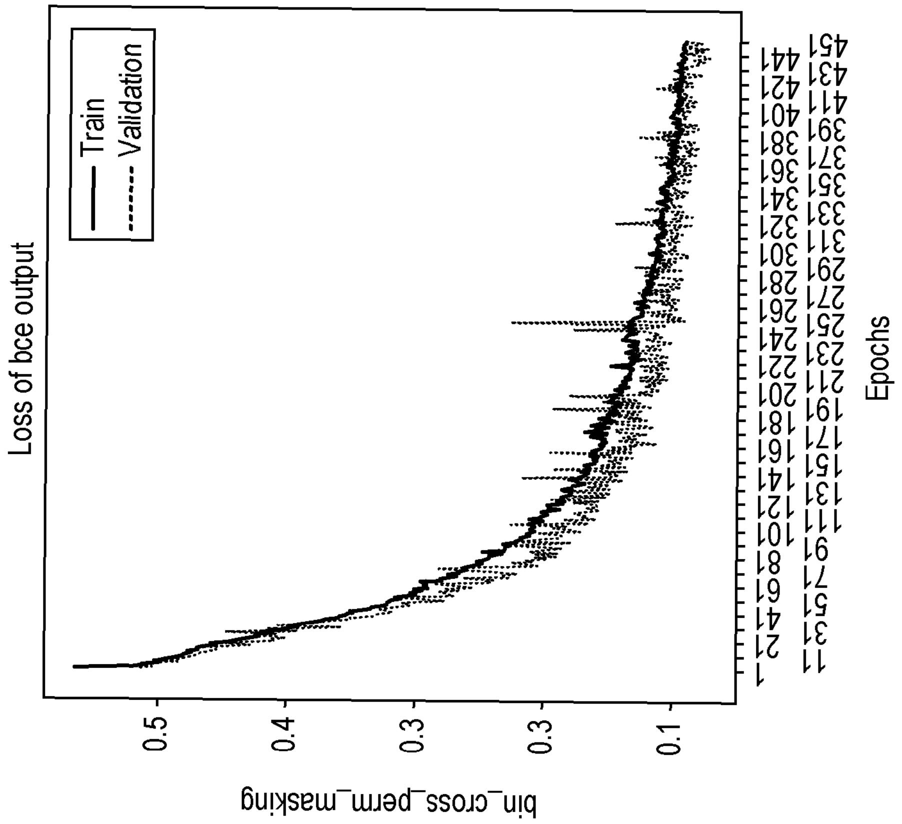


FIG. 24(b)

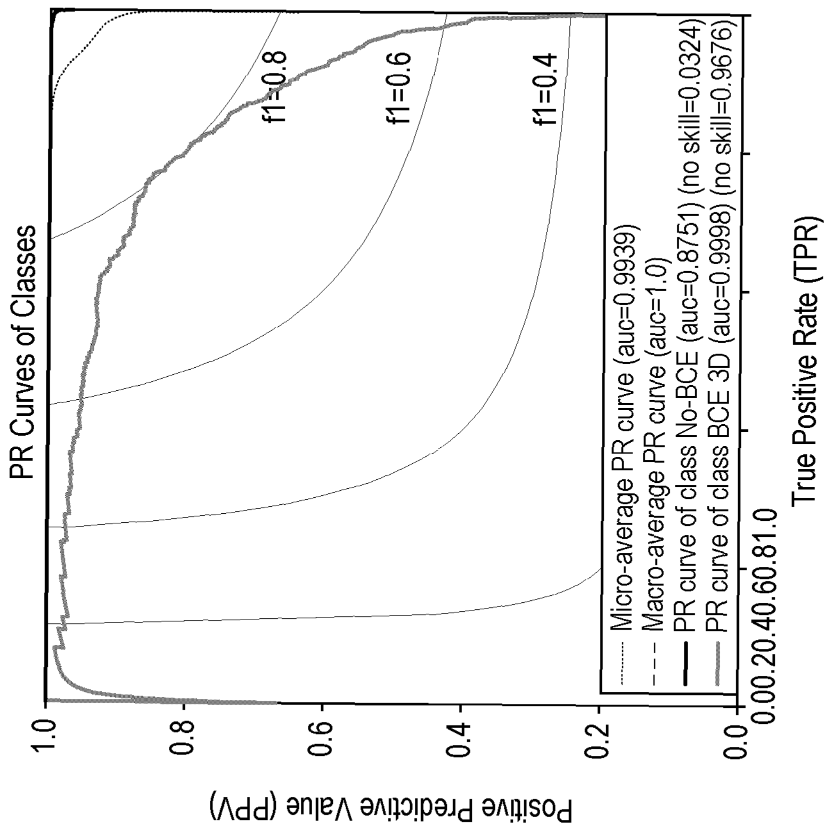


FIG. 24(a)

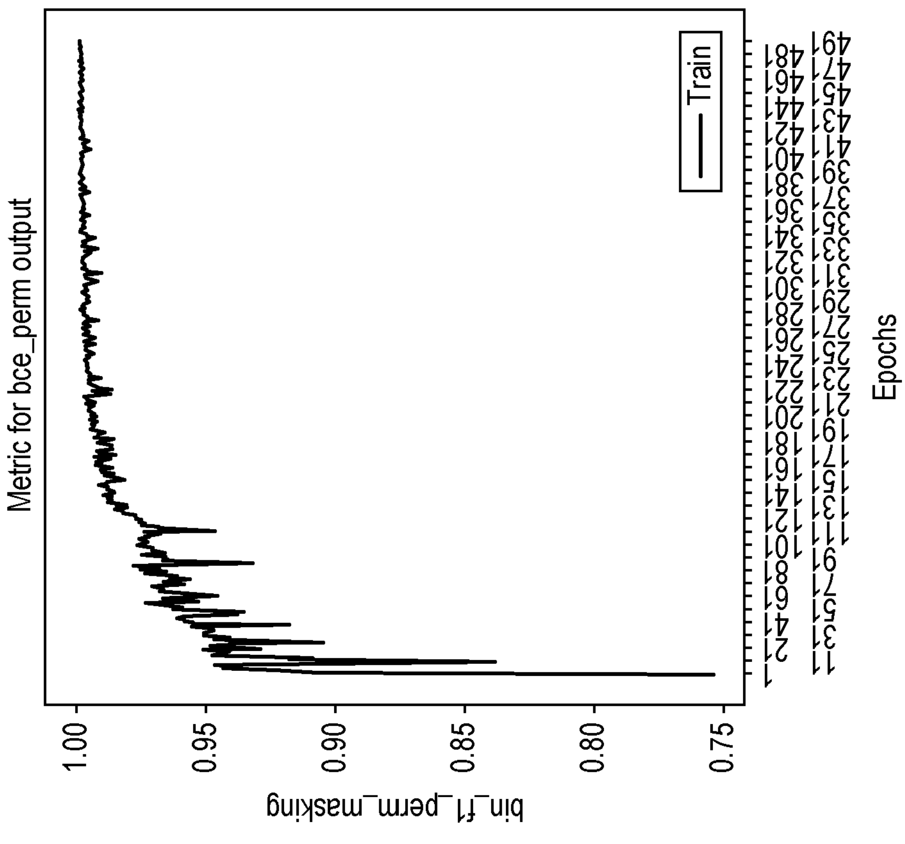


F1 curve for bce_perm for final estimates

FIG. 25(a)

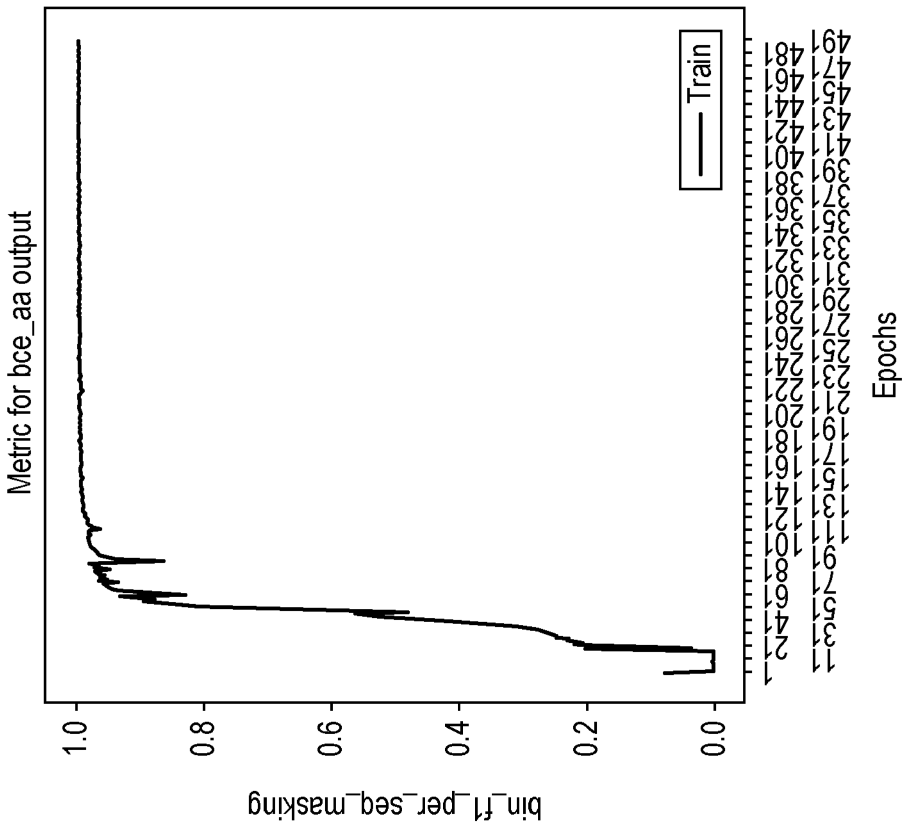


F1 curve for bce_aa for final estimates

FIG. 25(b)

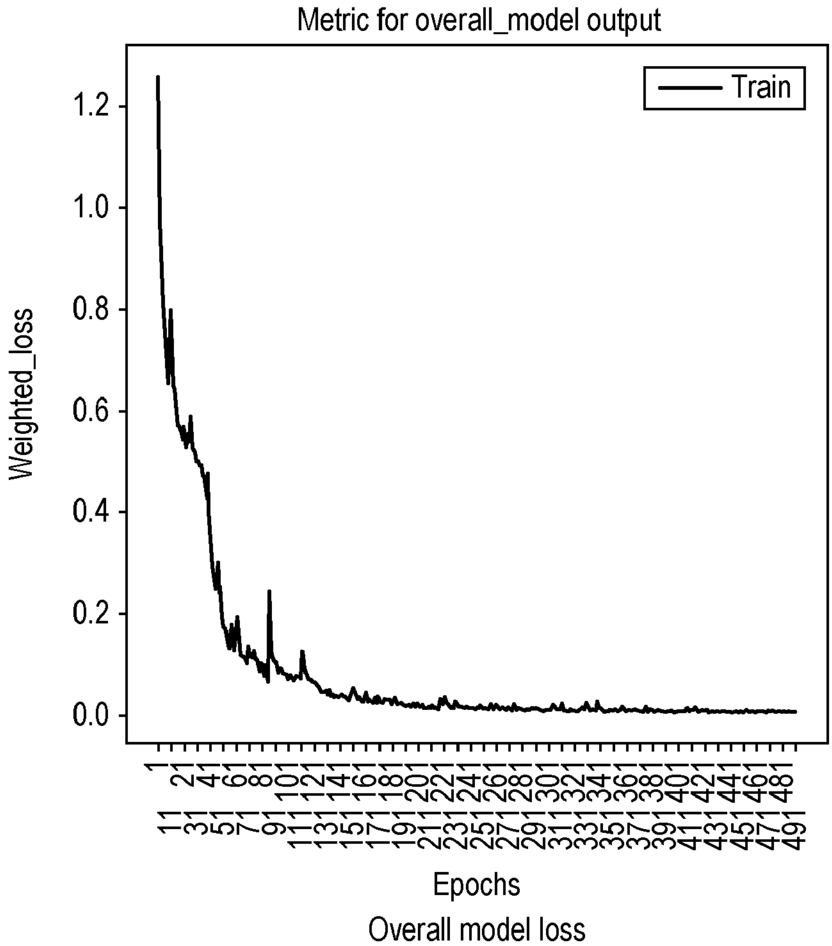
Metric for overall_model output
Train
Weighted_loss
1.2
1.0
0.8
0.6
0.4
0.2
0.0
1
11
21
31
41
51
61
71
81
91
101
111
121
131
141
151
161
171
181
191
201
211
221
231
241
251
261
271
281
291
301
311
321
331
341
351
361
371
381
391
401
411
421
431
441
451
461
471
481
491
Epochs
Overall model loss

*FIG. 25(c)*

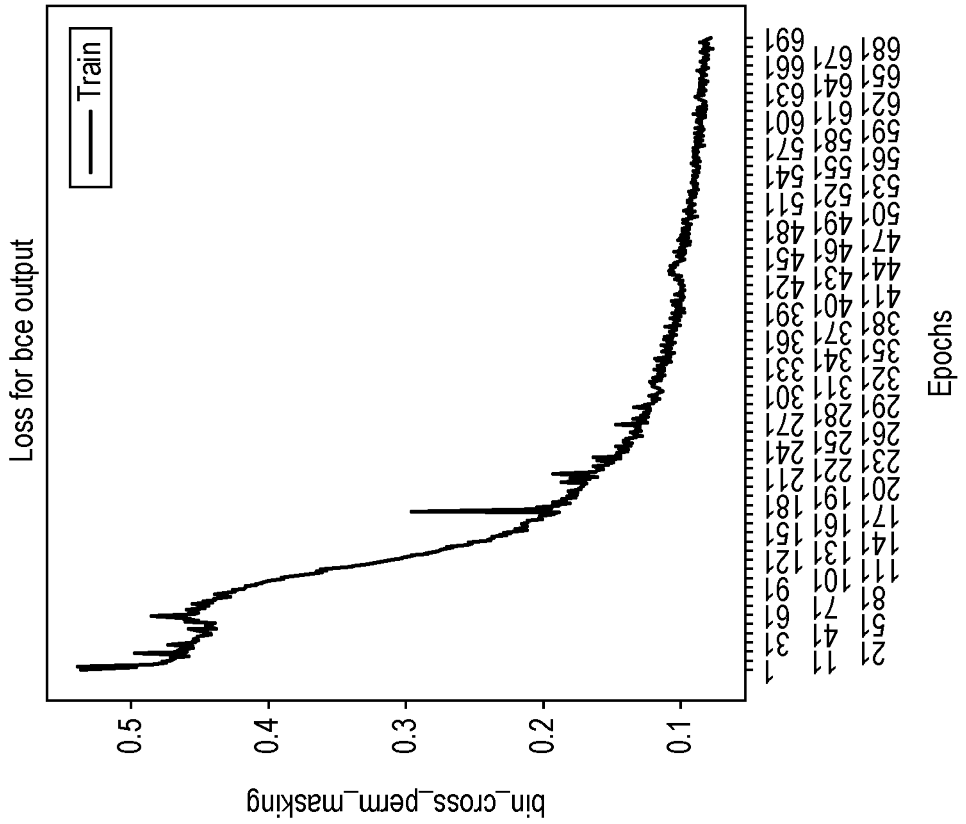


FIG. 26(b)

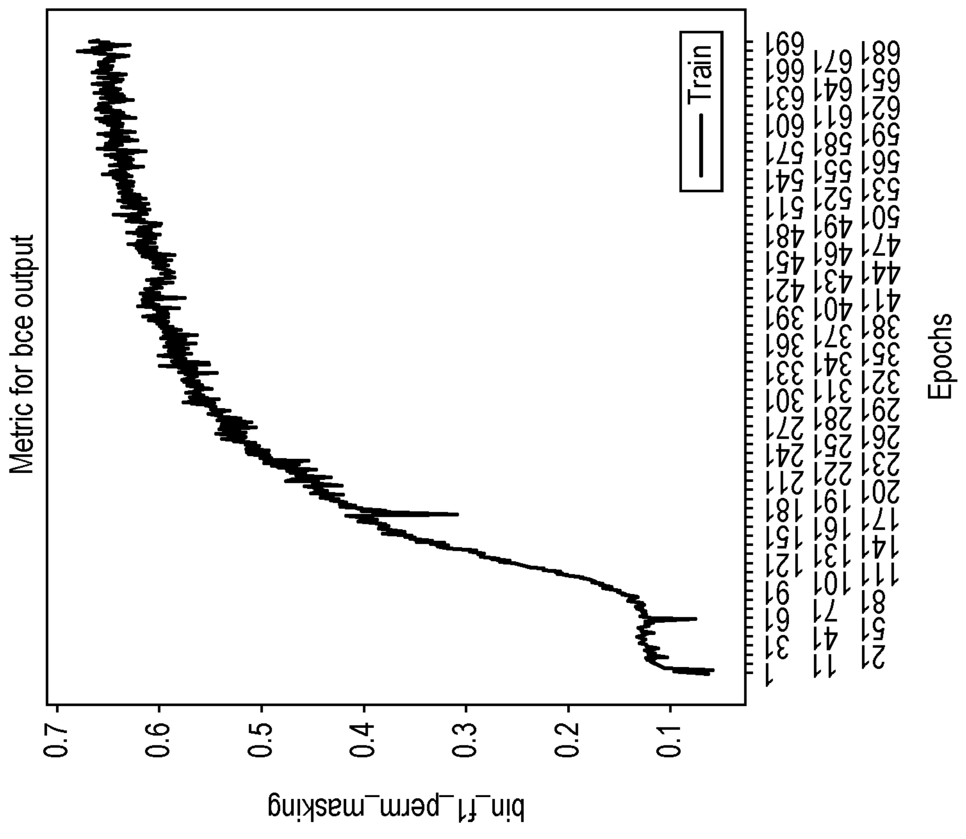


FIG. 26(a)

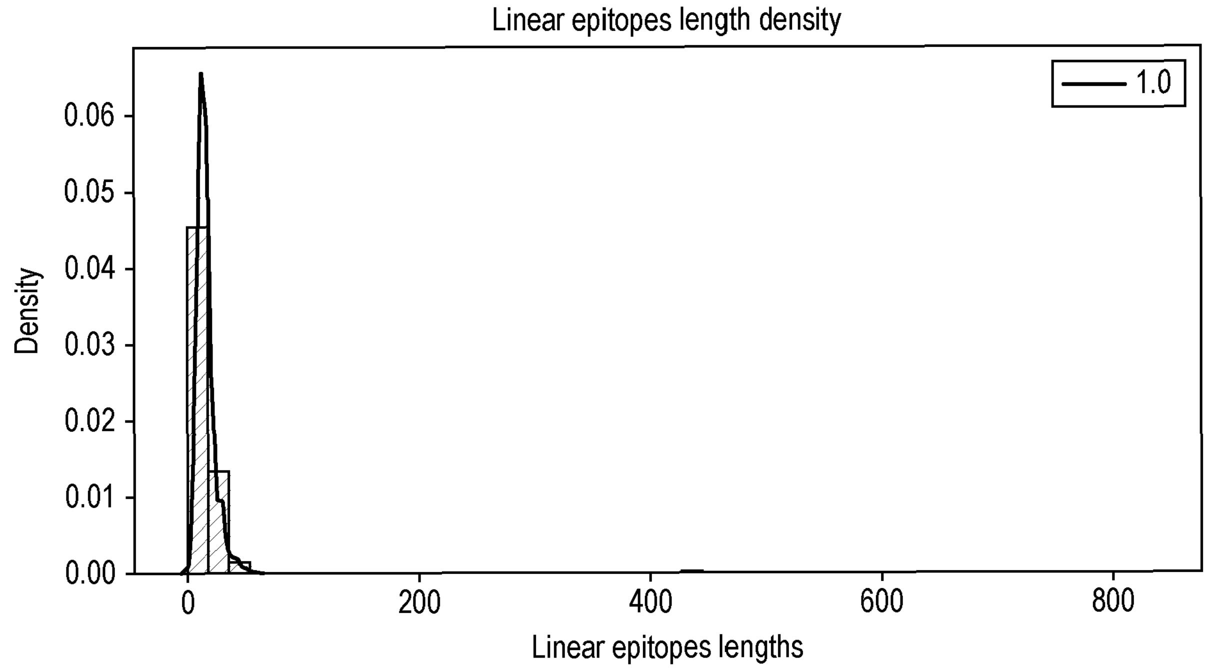


FIG. 27(a)

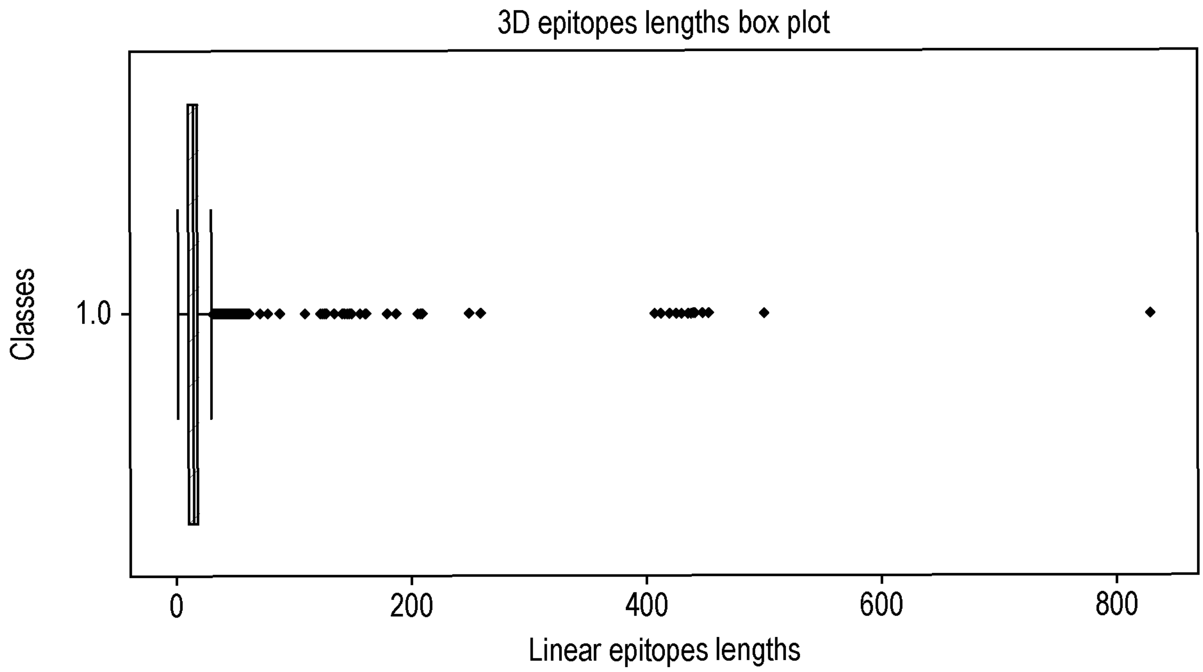


FIG. 27(b)

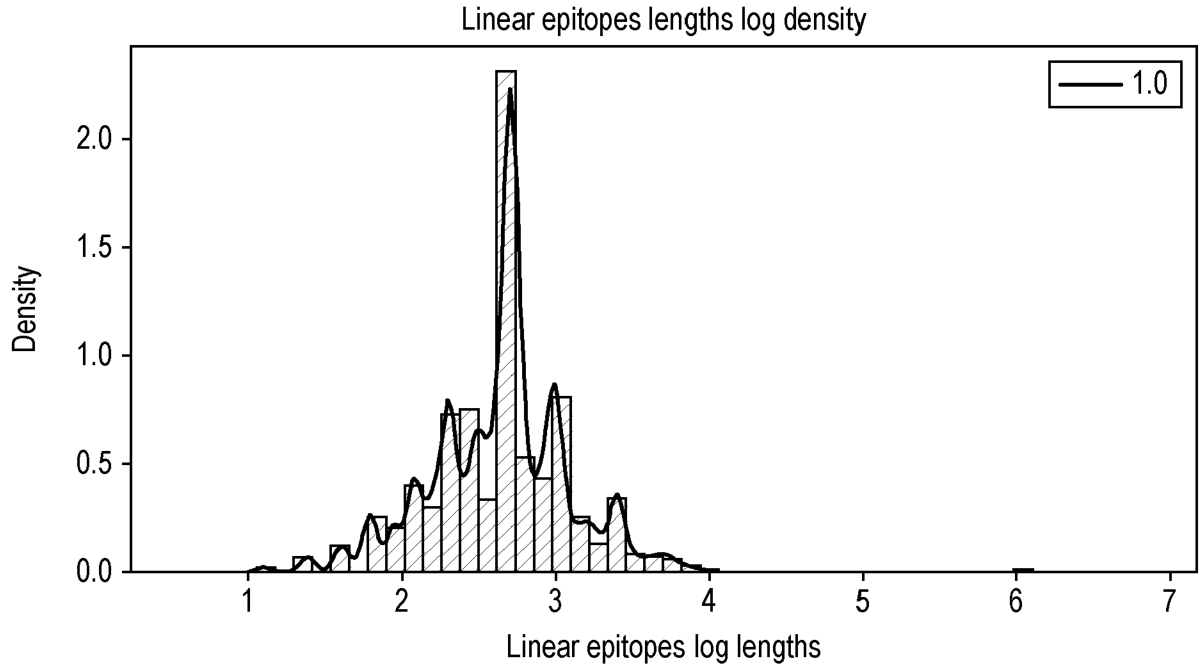


*FIG. 27(c)*

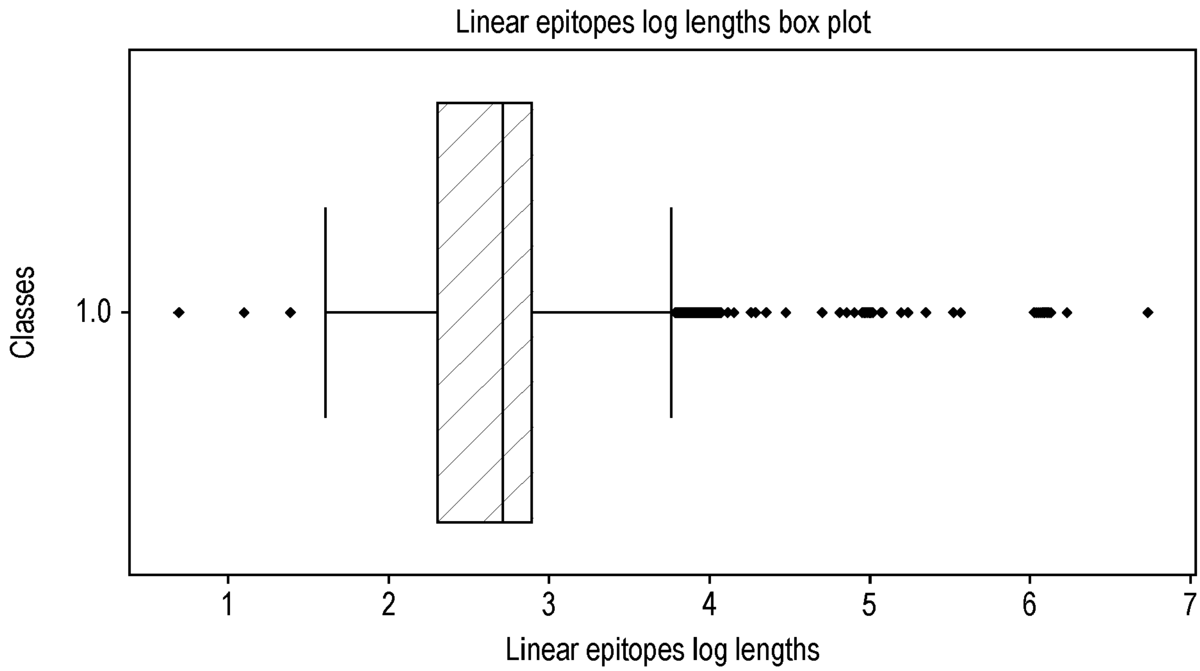


*FIG. 27(d)*

FIG. 28

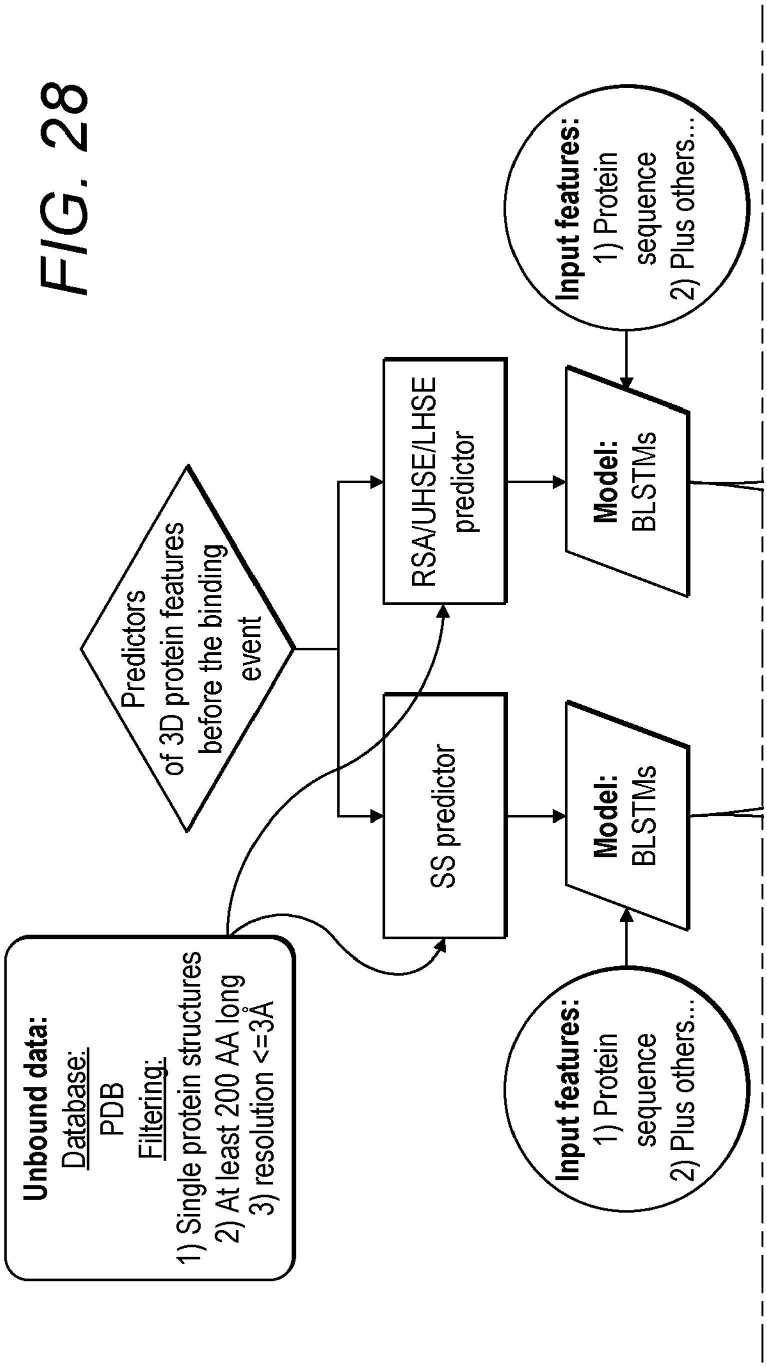
Unbound data:
Database:
PDB
Filtering:
1) Single protein structures
2) At least 200 AA long
3) resolution <=3Å
Predictors of 3D protein features before the binding event
SS predictor
RSA/UHSE/LHSE predictor
Model:
BLSTMs
Model:
BLSTMs
Input features:
1) Protein sequence
2) Plus others...
Input features:
1) Protein sequence
2) Plus others...

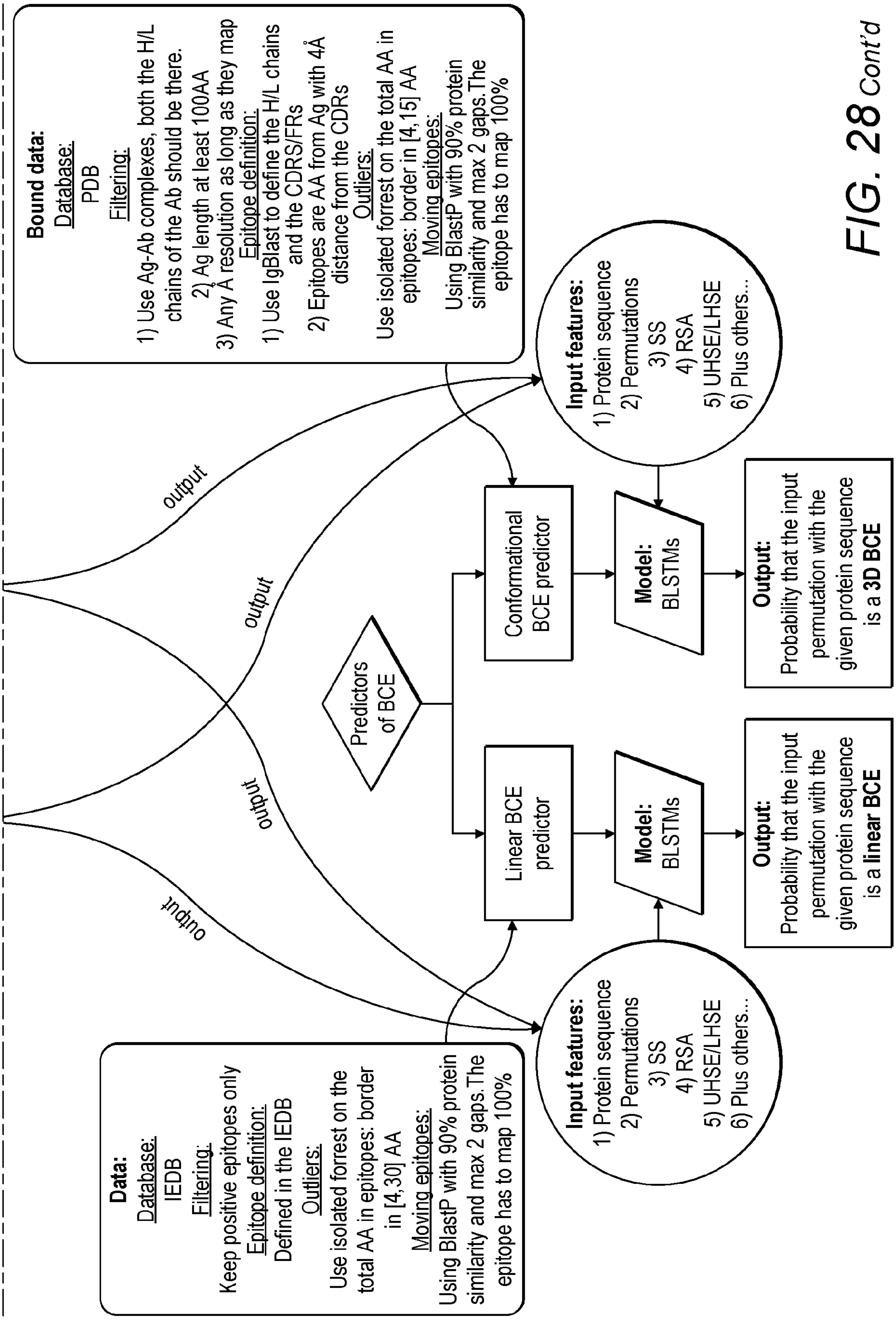
Bound data:
Database:
PDB
Filtering:
1) Use Ag-Ab complexes, both the H/L chains of the Ab should be there.
2) Ag length at least 100AA
3) Any Å resolution as long as they map
Epitope definition:
1) Use IgBlast to define the H/L chains and the CDRS/FRs
2) Epitopes are AA from Ag with 4Å distance from the CDRs
Outliers:
Use isolated forrest on the total AA in epitopes: border in [4,15] AA
Moving epitopes:
Using BlastP with 90% protein similarity and max 2 gaps.The epitope has to map 100%
Data:
Database:
IEDB
Filtering:
Keep positive epitopes only
Epitope definition:
Defined in the IEDB
Outliers:
Use isolated forrest on the total AA in epitopes: border in [4,30] AA
Moving epitopes:
Using BlastP with 90% protein similarity and max 2 gaps.The epitope has to map 100%
output
output
output
output
Predictors of BCE
Linear BCE predictor
Conformational BCE predictor
Input features:
1) Protein sequence
2) Permutations
3) SS
4) RSA
5) UHSE/LHSE
6) Plus others...
Input features:
1) Protein sequence
2) Permutations
3) SS
4) RSA
5) UHSE/LHSE
6) Plus others...
Model:
BLSTMs
Model:
BLSTMs
Output:
Probability that the input permutation with the given protein sequence is a linear BCE
Output:
Probability that the input permutation with the given protein sequence is a 3D BCE

*FIG. 28 Cont'd*

Europäisches Patentamt
European Patent Office
Office européen des brevets

# EUROPEAN SEARCH REPORT

Application Number
EP 23 38 6046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZENG YUANSONG ET AL: "Identifying B-cell epitopes using AlphaFold2 predicted structures and pretrained language model", BIOINFORMATICS (ONLINE), vol. 39, no. 4, 3 April 2023 (2023-04-03), XP093131201, GB, US, NL ISSN: 1367-4811, DOI: 10.1093/bioinformatics/btad187 Retrieved from the Internet: URL:https://academic.oup.com/bioinformatics/article-pdf/39/4/btad187/50087105/btad187.pdf> | 1-6,8,9, 11,14,15 | INV. G16B20/30 G16B40/20 |
| Y | * title, abstract, fig.1, items 2.1, 2.2.1, 2.2.4, 2.3.4, 3.4 * ----- | 7,10,12, 13 | |
| Y | BUKHARI SYED NISAR HUSSAIN ET AL: "Machine Learning Techniques for the Prediction of B-Cell and T-Cell Epitopes as Potential Vaccine Targets with a Specific Focus on SARS-CoV-2 Pathogen: A Review", PATHOGENS, vol. 11, no. 2, 24 January 2022 (2022-01-24), page 146, XP093131196, ISSN: 2076-0817, DOI: 10.3390/pathogens11020146 Retrieved from the Internet: URL:https://www.ncbi.nlm.nih.gov/pmc/articles/PMC8879824/pdf/pathogens-11-00146.pdf> * table 1 * ----- | 7 | TECHNICAL FIELDS SEARCHED (IPC) G16B |

-/--

The present search report has been drawn up for all claims

2

| Place of search | Date of completion of the search | Examiner |
|---|---|---|
| Munich | 15 February 2024 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 38 6046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZHANG JIAN ET AL: "Conformational B-Cell Epitopes Prediction from Sequences Using Cost-Sensitive Ensemble Classifiers and Spatial Clustering", BIOMED RESEARCH INTERNATIONAL, vol. 2014, 17 June 2014 (2014-06-17), pages 1-12, XP093131560, ISSN: 2314-6133, DOI: 10.1155/2014/689219 Retrieved from the Internet: URL:https://downloads.hindawi.com/journals/bmri/2014/689219.pdf> * p. 6, table 4 * ----- | 7,10 | |
| Y | SUN PINGPING ET AL: "Bioinformatics Resources and Tools for Conformational B-Cell Epitope Prediction", COMPUTATIONAL AND MATHEMATICAL METHODS IN MEDICINE, vol. 2013, 1 January 2013 (2013-01-01), pages 1-11, XP093131598, ISSN: 1748-670X, DOI: 10.1155/2013/943636 Retrieved from the Internet: URL:https://www.ncbi.nlm.nih.gov/pmc/articles/PMC3736542/pdf/CMMM2013-943636.pdf> * the whole document * ----- | 13 | TECHNICAL FIELDS SEARCHED (IPC) |
| Y | WO 2022/016125 A1 (GENENTECH INC [US]) 20 January 2022 (2022-01-20) * 185 * ----- | 12 | |

The present search report has been drawn up for all claims

2

| Place of search | Date of completion of the search | Examiner |
|---|---|---|
| Munich | 15 February 2024 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 38 6046

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2024

| Patent document cited in search report | Publication date | Patent family member(s) | Publication date |
|---|---|---|---|
| WO 2022016125 A1 | 20-01-2022 | AU 2021308081 A1 | 17-11-2022 |
| | | BR 112023000827 A2 | 07-02-2023 |
| | | CA 3180799 A1 | 20-01-2022 |
| | | CN 115997254 A | 21-04-2023 |
| | | EP 4182924 A1 | 24-05-2023 |
| | | IL 299801 A | 01-03-2023 |
| | | JP 2023534283 A | 08-08-2023 |
| | | KR 20230042048 A | 27-03-2023 |
| | | US 2022122690 A1 | 21-04-2022 |
| | | WO 2022016125 A1 | 20-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WOLFGANG KABSCH** ; **CHRISTIAN SANDER**. Dictionary of protein secondary structure: Pattern recognition of hydrogen-bonded and geometrical features. *Biopolymers*, December 1983, vol. 22 (12), 2577-2637
- **PETER JA COCK** ; **TIAGO ANTAO** ; **JEFFREY T CHANG** ; **BRAD A CHAPMAN** ; **CYMON J COX** ; **ANDREW DALKE** ; **IDDO FRIEDBERG** ; **THOMAS HAMELRYCK** ; **FRANK KAUFF** ; **BARTEK WILCZYNSKI et al.** Biopython: freely available python tools for computational molecular biology and bioinformatics. *Bioinformatics*, 2009, vol. 25 (11), 1422-1423
- **JIAN YE** ; **NING MA** ; **THOMAS L MADDEN** ; **JAMES M OSTELL.** Igblast: an immunoglobulin variable domain sequence analysis tool.. *Nucleic Acids Res*, July 2013, vol. 41, W34-40
- **G JOHNSON** ; **T T WU**. Kabat database and its applications: 30 years after the first variability plot.. *Nucleic acids research*, January 2000, vol. 28 (1), 214-218
- **F. PEDREGOSA** ; **G. VAROQUAUX** ; **A. GRAMFORT** ; **V. MICHEL** ; **B. THIRION** ; **O. GRISEL** ; **M. BLONDEL** ; **P. PRETTENHOFER** ; **R. WEISS** ; **V. DUBOURG**. Scikit-learn: Machine learning in Python.. *Journal of Machine Learning Research*, 2011, vol. 12, 2825-2830
- **T P HOPP** ; **K R WOODS.** Prediction of protein antigenic determinants from amino acid sequences.. *Proceedings of the National Academy of Sciences of the United States of America*, June 1981, vol. 78 (6), 3824-3828
- **R. GRANTHAM**. Amino acid difference formula to help explain protein evolution. *Science*, September 1974, vol. 185 (4154), 862
- **JOEL JANIN**. Surface and inside volumes in globular proteins.. *Nature*, February 1979, vol. 277, 491
- **H R GUY**. Amino acid side-chain partition energies and distribution of residues in soluble proteins. *Biophys J*, January 1985, vol. 47 (1), 61-70
- **J. K. MOHANA RAO** ; **PATRICK ARGOS**. A conformational preference parameter to predict helices in integral membrane proteins. *Biochimica et Biophysica Acta (BBA) - Protein Structure and Molecular Enzymology*, 1986, vol. 869 (2), 197-214
- **SANZO MIYAZAWA** ; **ROBERT L. JERNIGAN**. Estimation of effective interresidue contact energies from protein crystal structures: quasi-chemical approximation. *Macromolecules*, March 1985, vol. 18 (3), 534-552
- **GANG ZHAO** ; **ERWIN LONDON**. An amino acid ''transmembrane tendency'' scale that approaches the theoretical limit to accuracy for prediction of transmembrane helices: relationship to biological hydrophobicity. *Protein science : a publication of the Protein Society*, August 2006, vol. 15 (8), 1987-2001
- **CYRUS CHOTHIA**. The nature of the accessible and buried surfaces in proteins.. *Journal of Molecular Biology*, 1976, vol. 105 (1), 1-12
- **GD ROSE** ; **AR GESELOWITZ** ; **GJ LESSER** ; **RH LEE** ; **MH ZEHFUS**. Hydrophobicity of amino acid residues in globular proteins. *Science*, August 1985, vol. 229 (4716), 834
- **P Y CHOU** ; **G D FASMAN**. Prediction of the secondary structure of proteins from their amino acid sequence. *Adv Enzymol Relat Areas Mol Biol*, 1978, vol. 47, 45-148
- **SHNEIOR LIFSON** ; **CHRISTIAN SANDER**. Antiparallel and parallel -strands differ in amino acid residue preferences.. *Nature*, 1979, vol. 282 (5734), 109-111
- **M. COOPER** ; **ROBERT E. HAUSMAN**. The cell: a molecular approach. ASM Press, 2007, vol. 4
- **E A EMINI** ; **J V HUGHES** ; **D S PERLOW** ; **J BOGER**. Induction of hepatitis a virus-neutralizing antibody by a virus-specific synthetic peptide.. *Journal of virology*, September 1985, vol. 55 (3), 836-839
- **G. DELEAGE** ; **B. ROUX**. An algorithm for protein secondary structure prediction based on class prediction. *Protein Engineering, Design and Selection*, August 1987, vol. 1 (4), 289-294
- **P. MANAVALAN** ; **P. K. PONNUSWAMY**. Hydrophobic character of amino acid residues in globular proteins.. *Nature*, 1978, vol. 275 (5681), 673-674
- **MICHAEL LEVITT**. Conformational preferences of amino acids in globular proteins.. *Biochemistry*, October 1978, vol. 17 (20), 4277-4285
- **SERAFIN FRAGA**. Theoretical prediction of protein antigenic determinants from amino acid sequences. *Canadian Journal of Chemistry*, 1982, vol. 60 (20), 2606-2610

- **GJALT W. WELLING** ; **WICHER J. WEIJER** ; **RUURD VAN DER ZEE** ; **SYTSKE WELLING-WESTER**. Prediction of sequential antigenic regions in proteins. *FEBS Letters*, 1985, vol. 188 (2), 215-218
- **HENRY B. BULL** ; **KEITH BREESE**. Surface tension of amino acid solutions: A hydrophobicity scale of the amino acid residues. *Archives of Biochemistry and Biophysics*, 1974, vol. 161 (2), 665-670
- **C. A. BROWNE** ; **H. P. J. BENNETT** ; **S. SOLOMON**. The isolation of peptides by high-performance liquid chromatography using predicted elution positions. *Analytical Biochemistry*, 1982, vol. 124 (1), 201-208
- **R. WOLFENDEN** ; **L. ANDERSSON** ; **P. M. CULLIS** ; **C. C. B. SOUTHGATE**. Affinities of amino acid side chains for solvent water.. *Biochemistry*, February 1981, vol. 20 (4), 849-855
- **J L MEEK**. Prediction of peptide retention times in high-pressure liquid chromatography on the basis of amino acid composition.. *Proceedings of the National Academy of Sciences of the United States of America*, March 1980, vol. 77 (3), 1632-1636
- **R. BHASKARAN** ; **P. K. PONNUSWAMY**. Positional flexibilities of amino acid residues in globular proteins. *International Journal of Peptide and Protein Research*, 1988, vol. 32 (4), 241-255
- A model of evolutionary change in proteins. **SCHWARTZ R.M.** ; **DAYHOFF, M.O.** ; **B.C. ORCUTT**. Atlas of Protein Sequence and Structure. Natl. Biomed. Res. Found., 1978, vol. 5
- Lehninger principles of biochemistry. **DAVID L. (DAVID LEE) NELSON** ; **MICHAEL M. COX**. Principles of biochemistry. W.H.Freeman, 2017
- **K JWILSON** ; **A HONEGGER** ; **R P STOTZEL** ; **G J HUGHES.** The behaviour of peptides on reverse-phase supports during high-pressure liquid chromatography. The. *Biochemical journal*, October 1981, vol. 199 (1), 31-41
- **A. S. KOLASKAR** ; **PRASAD C. TONGAONKAR.** A semi-empirical method for prediction of antigenic determinants on protein antigens. *FEBS Letters*, 10 December 1990, vol. 276 (1-2), 172-174
- **PETER MCCALDON** ; **PATRICK ARGOS**. Oligopeptide biases in protein sequences and their use in predicting protein coding regions in nucleotide sequences. *Proteins: Structure, Function, and Bioinformatics*, 1988, vol. 4 (2), 99-122
- **J.M. ZIMMERMAN** ; **NAOMI ELIEZER** ; **R. SIMHA**. The characterization of amino acid sequences in proteins by statistical methods.. *Journal of Theoretical Biology*, 1968, vol. 21 (2), 170-201
- **DANIEL D. JONES**. Amino acid properties and side-chain orientation in proteins. *A cross correlation approach*, April 1975, vol. 50
- **D. EISENBERG** ; **E. SCHWARZ** ; **M. KOMAROMY** ; **R.WALL**. Analysis of membrane and surface protein sequences with the hydrophobic moment plot.. *Journal of Molecular Biology*, 1984, vol. 179 (1), 125-142
- **J. M. R. PARKER** ; **D. GUO** ; **R. S. HODGES**. New hydrophilicity scale derived from high-performance liquid chromatography peptide retention data: correlation of predicted surface residues with antigenicity and x-ray-derived accessible sites.. *Biochemistry*, September 1986, vol. 25 (19), 5425-5432
- **SHAUN D. BLACK** ; **DIANE R. MOULD**. Development of hydrophobicity parameters to analyze proteins which bear post- or cotranslational modifications. *Analytical Biochemistry*, 1991, vol. 193 (1), 72-82
- **J L. FAUCHERE** ; **V PLISKA**. *Hydrophobic parameters II of amino acid side-chains from the partitioning of N-acetyl-amino acid amides*, January 1983, vol. 18
- **ROBERT M. SWEET** ; **DAVID EISENBERG**. Correlation of sequence hydrophobicities measures similarity in three-dimensional protein structure.. *Journal of Molecular Biology*, 1983, vol. 171 (4), 479-488
- **JACK KYTE** ; **RUSSELL F. DOOLITTLE**. A simple method for displaying the hydropathic character of a protein.. *Journal of Molecular Biology*, 1982, vol. 157 (1), 105-132
- **CHARLES. TANFORD**. Contribution of hydrophobic interactions to the stability of the globular conformation of proteins.. *Journal of the American Chemical Society*, November 1962, vol. 84 (22), 4240-4247
- **DONALD J. ABRAHAM** ; **ALBERT J. LEO**. Extension of the fragment method to calculate amino acid zwitterion and side chain partition coefficients. *Proteins: Structure, Function, and Bioinformatics*, 1987, vol. 2 (2), 130-152
- **RICHARD COWAN** ; **R GWHITTAKER**. *Hydrophobicity indices for amino acid residues as determined by HPLC*, January 1990, vol. 3
- **MARK A. ROSEMAN**. Hydrophilicity of polar amino acid side-chains is markedly reduced by flanking peptide bonds.. *Journal of Molecular Biology*, 1988, vol. 200 (3), 513-522
- **A. A. ABODERIN**. An empirical hydrophobicity scale for alpha-amino-acids and some of its applications. *International Journal of Biochemistry*, 1971, vol. 2, 537-544